# EUROPEAN PATENT APPLICATION

(11) **EP 1 285 920 A1**
(43) Date of publication of application: **26.02.2003**
(21) Application number: 01118728.3
(22) Date of filing: 06.08.2001
(51) Int. Cl.: C07D 409/12, A61K 31/335, A61P 35/00

(54) **C10 Ester sustituted taxanes as antitumor agents**

(71) Applicant: Florida State University Research Foundation, Inc., Tallahassee, Florida 32306-2763 (US)
(72) Inventor: Holton, Robert A., Tallahassee, Florida 32312 (US)
(74) Representative: Maiwald, Walter, Dr. Dipl.-Chem.

(57) **Abstract**

Taxanes having an ester substituent at C(10), a hydroxy substituent at C(7), and a range of C(2), C(9), C(14), and side chain substituents.

## Description

### BACKGROUND OF THE INVENTION

The present invention is directed to novel taxanes, which have exceptional utility as antitumor agents.

The taxane family of terpenes, of which baccatin III and taxol are members, has been the subject of considerable interest in both the biological and chemical arts. Taxol itself is employed as a cancer chemotherapeutic agent and possesses a broad range of tumor-inhibiting activity. Taxol has a 2'R, 3'S configuration and the following structural formula: 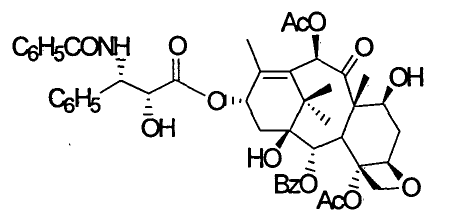 wherein Ac is acetyl.

Colin et al. reported in U.S. Patent 4,814,470 that certain taxol analogs have an activity significantly greater than that of taxol. One of these analogs, commonly referred to as docetaxel, has the following structural formula:

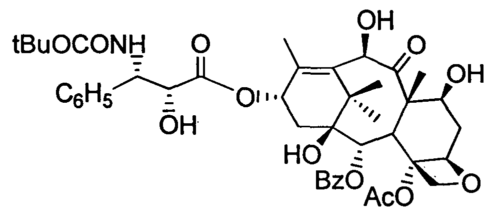

Although taxol and docetaxel are useful chemotherapeutic agents, there are limitations on their effectiveness, including limited efficacy against certain types of cancers and toxicity to subjects when administered at various doses. Accordingly, a need remains for additional chemotherapeutic agents with improved efficacy and less toxicity.

### SUMMARY OF THE INVENTION

Among the objects of the present invention, therefore, is the provision of taxanes, which compare favorably to taxol and docetaxel with respect to efficacy as anti-tumor agents and with respect to toxicity. In general, these taxanes possess an ester substituent other than formate, acetate and heterosubstituted acetate at C-10, a hydroxy substituent at C-7 and a range of C-3' substituents.

Briefly, therefore, the present invention is directed to the taxane composition, per se, to pharmaceutical compositions comprising the taxane and a pharmaceutically acceptable carrier, and to methods of administration.

Other objects and features of this invention will be in part apparent and in part pointed out hereinafter.

### DETAILED DESCRIPTION OF THE PREFERRED EMBODIMENTS

In one embodiment of the present invention, the taxanes of the present invention correspond to structure (1): 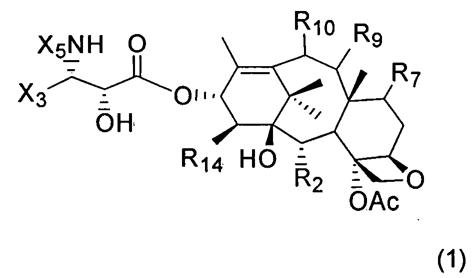 wherein
R₂ is acyloxy;
R₇ is hydroxy;
R₉ is keto, hydroxy, or acyloxy;
R₁₀ is R₁₀ₐCOO-;
R₁₀ₐ is hydrocarbyl, substituted hydrocarbyl, or heterocyclo wherein said hydrocarbyl or substituted hydrocarbyl contains carbon atoms in the alpha and beta positions relative to the carbon of which R₁₀ₐ is a substituent;
R₁₄ is hydrido or hydroxy;
X₃ is substituted or unsubstituted alkyl, alkenyl, alkynyl, phenyl or heterocyclo, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo;
Ac is acetyl; and
R₇, R₉, and R₁₀ independently have the alpha or beta stereochemical configuration.

In one embodiment, R₂ is an ester (R₂ₐC(O)O-), a carbamate (R₂aR_{2b}NC(O)O-), a carbonate (R₂ₐOC(O)O-), or a thiocarbamate (R₂ₐSC(O)O-) wherein R₂ₐ and R_{2b} are independently hydrogen, hydrocarbyl, substituted hydrocarbyl or heterocyclo. In a preferred embodiment, R₂ is an ester (R₂ₐC(O)O-), wherein R₂ₐ is aryl or heteroaromatic. In another preferred embodiment, R₂ is an ester (R₂ₐC(O)O-), wherein R₂ₐ is substituted or unsubstituted phenyl, furyl, thienyl, or pyridyl. In one particularly preferred embodiment, R₂ is benzoyloxy.

While R₉ is keto in one embodiment of the present invention, in other embodiments R₉ may have the alpha or beta stereochemical configuration, preferably the beta stereochemical configuration, and may be, for example, α- or β-hydroxy or α- or β-acyloxy. For example, when R₉ is acyloxy, it may be an ester (R₉ₐC(O)O-), a carbamate (R₉ₐR_{9b}NC(O)O-), a carbonate (R₉ₐOC(O)O-), or a thiocarbamate (R₉ₐSC(O)O-) wherein R₉ₐ and R_{9b} are independently hydrogen, hydrocarbyl, substituted hydrocarbyl or heterocyclo. If R₉ is an ester (R₉ₐC(O)O-), R₉ₐ is substituted or unsubstituted alkyl, substituted or unsubstituted alkenyl, substituted or unsubstituted aryl or substituted or unsubstituted heteroaromatic. Still more preferably, R₉ is an ester (R₉ₐC(O)O-), wherein R₉ₐ is substituted or unsubstituted phenyl, substituted or unsubstituted furyl, substituted or unsubstituted thienyl, or substituted or unsubstituted pyridyl. In one embodiment R₉ is (R₉ₐC(O)O-) wherein R₉ₐ is methyl, ethyl, propyl (straight, branched or cyclic), butyl (straight, branched or cyclic), pentyl, (straight, branched or cyclic), or hexyl (straight, branched or cyclic). In another embodiment R₉ is (R₉ₐC(O)O-) wherein R₉ₐ is substituted methyl, substituted ethyl, substituted propyl (straight, branched or cyclic), substituted butyl (straight, branched or cyclic), substituted pentyl, (straight, branched or cyclic), or substituted hexyl (straight, branched or cyclic) wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

In one embodiment, R₁₀ is R₁₀ₐCOO- wherein R₁₀ₐ is (i) substituted or unsubstituted C₂ to C₈ alkyl (straight, branched or cyclic), such as ethyl, propyl, butyl, pentyl, or hexyl; (ii) substituted or unsubstituted C₂ to C₈ alkenyl (straight, branched or cyclic), such as ethenyl, propenyl, butenyl, pentenyl or hexenyl; (iii) substituted or unsubstituted C₂ to C₈ alkynyl (straight or branched) such as ethynyl, propynyl, butynyl, pentynyl, or hexynyl; (iv) substituted or unsubstituted phenyl; or (v) substituted or unsubstituted heteroaromatic such as furyl, thienyl, or pyridyl. The substituents may be hydrocarbyl or any of the heteroatom containing substituents identified elsewhere herein for substituted hydrocarbyl. In a preferred embodiment, R₁₀ₐ is ethyl, straight, branched or cyclic propyl, straight, branched or cyclic butyl, straight, branched or cyclic pentyl, straight, branched or cyclic hexyl, straight or branched propenyl, isobutenyl, furyl or thienyl. In another embodiment, R₁₀ₐ is substituted ethyl, substituted propyl (straight, branched or cyclic), substituted propenyl (straight or branched), substituted isobutenyl, substituted furyl or substituted thienyl wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

Exemplary X₃ substituents include substituted or unsubstituted C₂ to C₈ alkyl, substituted or unsubstituted C₂ to C₈ alkenyl, substituted or unsubstituted C₂ to C₈ alkynyl, substituted or unsubstituted heteroaromatics containing 5 or 6 ring atoms, and substituted or unsubstituted phenyl. Exemplary preferred X₃ substituents include substituted or unsubstituted ethyl, propyl, butyl, cyclopropyl, cyclobutyl, cyclohexyl, isobutenyl, furyl, thienyl, and pyridyl.

Exemplary X₅ substituents include -COX₁₀, -COOX₁₀ or -CONHX₁₀ wherein X₁₀ is substituted or unsubstituted alkyl, alkenyl, phenyl or heteroaromatic. Exemplary preferred X₅ substituents include -COX₁₀, -COOX₁₀ or -CONHX₁₀ wherein X₁₀ is (i) substituted or unsubstituted C₁ to C₈ alkyl such as substituted or unsubstituted methyl, ethyl, propyl (straight, branched or cyclic), butyl (straight, branched or cyclic), pentyl (straight, branched or cyclic), or hexyl (straight, branched or cyclic); (ii) substituted or unsubstituted C₂ to C₈ alkenyl such as substituted or unsubstituted ethenyl, propenyl (straight, branched or cyclic), butenyl (straight, branched or cyclic), pentenyl (straight, branched or cyclic) or hexenyl (straight, branched or cyclic); (iii) substituted or unsubstituted C₂ to C₈ alkynyl such as substituted or unsubstituted ethynyl, propynyl (straight or branched), butynyl (straight or branched), pentynyl (straight or branched), or hexynyl (straight or branched); (iv) substituted or unsubstituted phenyl, or (v) substituted or unsubstituted heteroaromatic such as furyl, thienyl, or pyridyl, wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

In one embodiment of the present invention, the taxanes of the present invention correspond to structure (2): 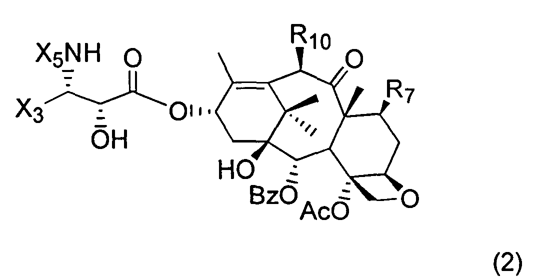 wherein
R₇ is hydroxy;
R₁₀ is R₁₀ₐCOO-;
X₃ is substituted or unsubstituted alkyl, alkenyl, alkynyl, or heterocyclo, wherein alkyl comprises at least two carbon atoms;
X₅ is -COX₁₀, -COOX₁₀, or -CONHX₁₀; and
X₁₀ is hydrocarbyl, substituted hydrocarbyl, or heterocyclo;
R₁₀ₐ is hydrocarbyl, substituted hydrocarbyl, or heterocyclo wherein said hydrocarbyl or substituted hydrocarbyl contains carbon atoms in the alpha and beta positions relative to the carbon of which R₁₀ₐ is a substituent;
Bz is benzoyl; and
Ac is acetyl.
For example, in this preferred embodiment in which the taxane corresponds to structure (2), R₁₀ₐ may be substituted or unsubstituted ethyl, propyl or butyl, more preferably substituted or unsubstituted ethyl or propyl, still more preferably substituted or unsubstituted ethyl, and still more preferably unsubstituted ethyl. While R₁₀ₐ is selected from among these, in one embodiment X₃ is selected from substituted or unsubstituted alkyl, alkenyl, phenyl or heterocyclo, more preferably substituted or unsubstituted alkenyl, phenyl or heterocyclo, still more preferably substituted or unsubstituted phenyl or heterocyclo, and still more preferably heterocyclo such as furyl, thienyl or pyridyl. While R₁₀ₐ and X₃ are selected from among these, in one embodiment X₅ is selected from -COX₁₀ wherein X₁₀ is phenyl, alkyl or heterocyclo, more preferably phenyl. Alternatively, while R₁₀ₐ and X₃ are selected from among these, in one embodiment X₅ is selected from -COX₁₀ wherein X₁₀ is phenyl, alkyl or heterocyclo, more preferably phenyl, or X₅ is -COOX₁₀ wherein X₁₀ is alkyl, preferably t-butyl. Among the more preferred embodiments, therefore, are taxanes corresponding to structure 2 in which (i) X₅ is -COOX₁₀ wherein X₁₀ is tert-butyl or X₅ is -COX₁₀ wherein X₁₀ is phenyl, (ii) X₃ is substituted or unsubstituted cycloalkyl, alkenyl, phenyl or heterocyclo, more preferably substituted or unsubstituted isobutenyl, phenyl, furyl, thienyl, or pyridyl, still more preferably unsubstituted isobutenyl, furyl, thienyl or pyridyl, and (iii) R₇ₐ is unsubstituted ethyl or propyl, more preferably ethyl.

Among the preferred embodiments in which the taxane corresponds to structure 1 or 2 wherein R₁₀ is R₁₀ₐCOO-, R₁₀ₐ may be substituted or unsubstituted ethyl or propyl, and more preferably unsubstituted ethyl or propyl. While R₁₀ₐ is selected from among these, in one embodiment X₃ is selected from substituted or unsubstituted cycloalkyl, phenyl or heterocyclo, more preferably substituted or unsubstituted cycloalkyl, phenyl, furyl, thienyl or pyridyl. While R₁₀ₐ and X₃ are selected from among these, in one embodiment X₅ is selected from-COOX₁₀ wherein X₁₀ is tert-butyl, tert-amyl, isobutyl, isopropyl or substituted or unsubstituted cycloalkyl. R₂, R₉ and R₁₄ are as defined in structures 1 and 2, and are preferably benzoyloxy, keto and hydrido, respectively. Among the more preferred embodiments, therefore, are taxanes corresponding to structure 2 in which (i) X₅ is -COOX₁₀ wherein X₁₀ is tert-butyl, tert-amyl, isobutyl, isopropyl, or unsubstituted cycloalkyl, and more preferably tert-butyl, (ii) X₃ is substituted or unsubstituted cycloalkyl, phenyl, furyl, thienyl, or pyridyl, and more preferably unsubstituted cycloalkyl, phenyl, furyl, thienyl or pyridyl, and (iii) R₁₀ₐ is unsubstituted ethyl or propyl. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Among the preferred embodiments, therefore, are taxanes corresponding to structure 1 or 2 wherein R₁₀ is R₁₀ₐCOO- wherein R₁₀ₐ is ethyl. In this embodiment, X₃ is preferably cycloalkyl, isobutenyl, or heterocyclo, more preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl. In one alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrido. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Also among the preferred embodiments are taxanes corresponding to structure 1 or 2 wherein R₁₀ is R₁₀ₐCOO- wherein R₁₀ₐ is propyl. In this embodiment, X₃ is preferably cycloalkyl, isobutenyl, phenyl, substituted phenyl such as p-nitrophenyl, or heterocyclo, more preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl. In one alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrido. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Taxanes having the general formula 1 may be obtained by treatment of a β-lactam with an alkoxide having the taxane tetracyclic nucleus and a C-13 metallic oxide substituent to form compounds having a β-amido ester substituent at C-13 (as described more fully in Holton U.S. Patent 5,466,834), followed by removal of the hydroxy protecting groups. The β-lactam has the following structural formula (3): 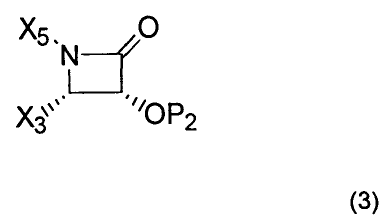 wherein P₂ is a hydroxy protecting group and X₃ and X₅ are as previously defined and the alkoxide has the structural formula (4): 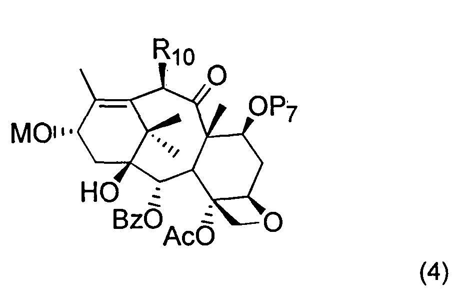 wherein M is a metal or ammonium, P₇ is a hydroxy protecting group and R₁₀ is as previously defined.

Alkoxide 4 may be prepared from 10-deacetylbaccatin III (or a derivative thereof) by selective protection of the C(7) hydroxyl group and then esterification of the C(10) hydroxyl group followed by treatment with a metallic amide. In one embodiment of the present invention, the C(7) hydroxyl group of 10-deacetylbaccatin III is selectively protected with a silyl group as described, for example, by Denis, et. al. (*J. Am. Chem. Soc.,* **1988**, 110, 5917). In general, the silylating agents may be used either alone or in combination with a catalytic amount of a base such as an alkali metal base.

Alternatively, the C(10) hydroxyl group of a taxane can be selectively acylated in the absence of a base, as described, for example in Holton et al., PCT Patent Application WO 99/09021. Acylating agents which may be used for the selective acylation of the C(10) hydroxyl group of a taxane include substituted or unsubstituted alkyl or aryl anhydrides. While the acylation of the C(10) hydroxy group of the taxane will proceed at an adequate rate for many acylating agents, it has been discovered that the reaction rate may be increased by including a Lewis acid in the reaction mixture. Preferred Lewis acids include zinc chloride, stannic chloride, cerium trichloride, cuprous chloride, lanthanum trichloride, dysprosium trichloride, and ytterbium trichloride. Zinc chloride or cerium trichloride is particularly preferred when the acylating agent is an anhydride.

Derivatives of 10-deacetylbaccatin III having alternative substituents at C(2), C(9) and C(14) and processes for their preparation are known in the art. Taxane derivatives having acyloxy substituents other than benzoyloxy at C(2) may be prepared, for example, as described in Holton et al., U.S. Patent No. 5,728,725 or Kingston et al., U.S. Patent No. 6,002,023. Taxanes having acyloxy or hydroxy substituents at C(9) in place of keto may be prepared, for example as described in Holton et al., U.S. Patent No. 6,011,056 or Gunawardana et al., U.S. Patent No. 5,352,806. Taxanes having a beta hydroxy substituent at C(14) may be prepared from naturally occurring 14-hydroxy-10-deacetylbaccatin III.

Processes for the preparation and resolution of the β-lactam starting material are generally well known. For example, the β-lactam may be prepared as described in Holton, U.S. Patent No. 5,430,160 and the resulting enantiomeric mixtures of β-lactams may be resolved by a stereoselective hydrolysis using a lipase or enzyme as described, for example, in Patel, U.S. Patent No. 5,879,929 Patel U.S. Patent No. 5,567,614 or a liver homogenate as described, for example, in PCT Patent Application No. 00/41204. In a preferred embodiment in which the β-lactam is furyl substituted at the C(4) position, the β-lactam can be prepared as illustrated in the following reaction scheme: 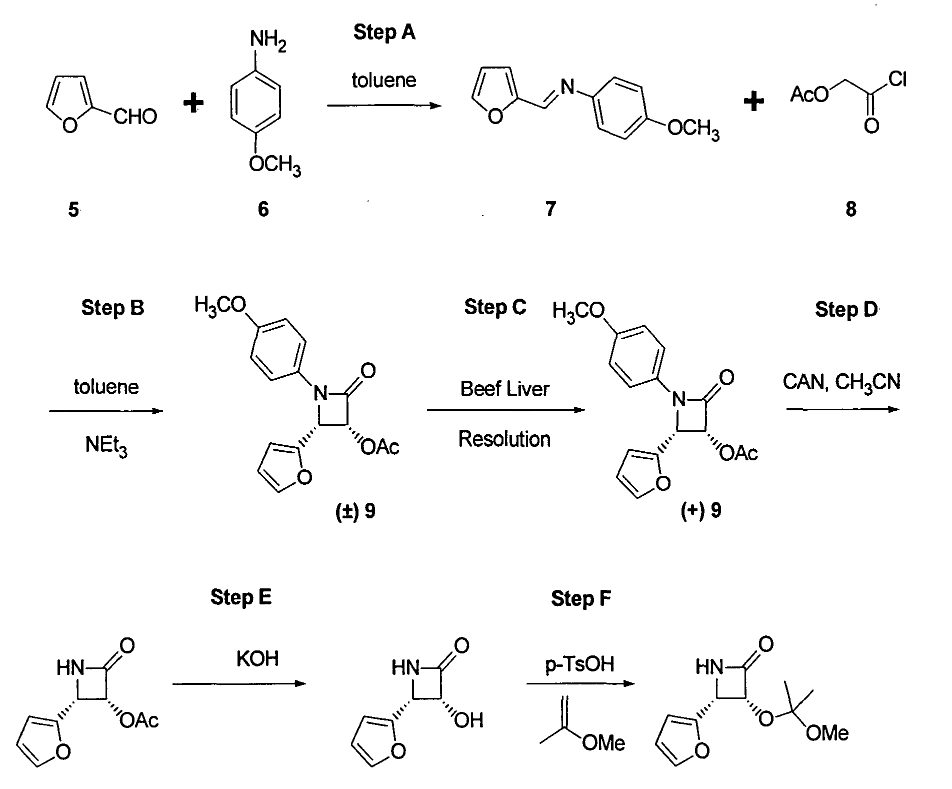 wherein Ac is acetyl, NEt₃ is triethylamine, CAN is ceric ammonium nitrate, and p-TsOH is p-toluenesulfonic acid. The beef liver resolution may be carried out, for example, by combining the enatiomeric β-lactam mixture with a beef liver suspension (prepared, for example, by adding 20 g of frozen beef liver to a blender and then adding a pH 8 buffer to make a total volume of 1 L).

Compounds of formula 1 of the instant invention are useful for inhibiting tumor growth in mammals including humans and are preferably administered in the form of a pharmaceutical composition comprising an effective antitumor amount of a compound of the instant invention in combination with at least one pharmaceutically or pharmacologically acceptable carrier. The carrier, also known in the art as an excipient, vehicle, auxiliary, adjuvant, or diluent, is any substance which is pharmaceutically inert, confers a suitable consistency or form to the composition, and does not diminish the therapeutic efficacy of the antitumor compounds. The carrier is "pharmaceutically or pharmacologically acceptable" if it does not produce an adverse, allergic or other untoward reaction when administered to a mammal or human, as appropriate.

The pharmaceutical compositions containing the antitumor compounds of the present invention may be formulated in any conventional manner. Proper formulation is dependent upon the route of administration chosen. The compositions of the invention can be formulated for any route of administration so long as the target tissue is available via that route. Suitable routes of administration include, but are not limited to, oral, parenteral (e.g., intravenous, intraarterial, subcutaneous, rectal, subcutaneous, intramuscular, intraorbital, intracapsular, intraspinal, intraperitoneal, or intrasternal), topical (nasal, transdermal, intraocular), intravesical, intrathecal, enteral, pulmonary, intralymphatic, intracavital, vaginal, transurethral, intradermal, aural, intramammary, buccal, orthotopic, intratracheal, intralesional, percutaneous, endoscopical, transmucosal, sublingual and intestinal administration.

Pharmaceutically acceptable carriers for use in the compositions of the present invention are well known to those of ordinary skill in the art and are selected based upon a number of factors: the particular antitumor compound used, and its concentration, stability and intended bioavailability; the disease, disorder or condition being treated with the composition; the subject, its age, size and general condition; and the route of administration. Suitable carriers are readily determined by one of ordinary skill in the art (see, for example, J. G. Nairn, in: Remington's Pharmaceutical Science (A. Gennaro, ed.), Mack Publishing Co., Easton, Pa., (1985), pp. 1492-1517, the contents of which are incorporated herein by reference).

The compositions are preferably formulated as tablets, dispersible powders, pills, capsules, gelcaps, caplets, gels, liposomes, granules, solutions, suspensions, emulsions, syrups, elixirs, troches, dragees, lozenges, or any other dosage form which can be administered orally. Techniques and compositions for making oral dosage forms useful in the present invention are described in the following references: 7 Modern Pharmaceutics, Chapters 9 and 10 (Banker & Rhodes, Editors, 1979); Lieberman et al., Pharmaceutical Dosage Forms: Tablets (1981); and Ansel, Introduction to Pharmaceutical Dosage Forms 2nd Edition (1976).

The compositions of the invention for oral administration comprise an effective antitumor amount of a compound of the invention in a pharmaceutically acceptable carrier. Suitable carriers for solid dosage forms include sugars, starches, and other conventional substances including lactose, talc, sucrose, gelatin, carboxymethylcellulose, agar, mannitol, sorbitol, calcium phosphate, calcium carbonate, sodium carbonate, kaolin, alginic acid, acacia, corn starch, potato starch, sodium saccharin, magnesium carbonate, tragacanth, microcrystalline cellulose, colloidal silicon dioxide, croscarmellose sodium, talc, magnesium stearate, and stearic acid. Further, such solid dosage forms may be uncoated or may be coated by known techniques; e.g., to delay disintegration and absorption.

The antitumor compounds of the present invention are also preferably formulated for parenteral administration, e.g., formulated for injection via intravenous, intraarterial, subcutaneous, rectal, subcutaneous, intramuscular, intraorbital, intracapsular, intraspinal, intraperitoneal, or intrasternal routes. The compositions of the invention for parenteral administration comprise an effective antitumor amount of the antitumor compound in a pharmaceutically acceptable carrier. Dosage forms suitable for parenteral administration include solutions, suspensions, dispersions, emulsions or any other dosage form which can be administered parenterally. Techniques and compositions for making parenteral dosage forms are known in the art.

Suitable carriers used in formulating liquid dosage forms for oral or parenteral administration include nonaqueous, pharmaceutically-acceptable polar solvents such as oils, alcohols, amides, esters, ethers, ketones, hydrocarbons and mixtures thereof, as well as water, saline solutions, dextrose solutions (e.g., DW5), electrolyte solutions, or any other aqueous, pharmaceutically acceptable liquid.

Suitable nonaqueous, pharmaceutically-acceptable polar solvents include, but are not limited to, alcohols (e.g., α-glycerol formal, β-glycerol formal, 1, 3-butyleneglycol, aliphatic or aromatic alcohols having 2-30 carbon atoms such as methanol, ethanol, propanol, isopropanol, butanol, t-butanol, hexanol, octanol, amylene hydrate, benzyl alcohol, glycerin (glycerol), glycol, hexylene glycol, tetrahydrofurfuryl alcohol, lauryl alcohol, cetyl alcohol, or stearyl alcohol, fatty acid esters of fatty alcohols such as polyalkylene glycols (e.g., polypropylene glycol, polyethylene glycol), sorbitan, sucrose and cholesterol); amides (e.g., dimethylacetamide (DMA), benzyl benzoate DMA, dimethylformamide, N-(β-hydroxyethyl)-lactamide, N, N-dimethylacetamide_amides, 2-pyrrolidinone, 1-methyl-2-pyrrolidinone, or polyvinylpyrrolidone); esters (e.g., 1-methyl-2-pyrrolidinone, 2-pyrrolidinone, acetate esters such as monoacetin, diacetin, and triacetin, aliphatic or aromatic esters such as ethyl caprylate or octanoate, alkyl oleate, benzyl benzoate, benzyl acetate, dimethylsulfoxide (DMSO), esters of glycerin such as mono, di, or tri-glyceryl citrates or tartrates, ethyl benzoate, ethyl acetate, ethyl carbonate, ethyl lactate, ethyl oleate, fatty acid esters of sorbitan, fatty acid derived PEG esters, glyceryl monostearate, glyceride esters such as mono, di, or tri-glycerides, fatty acid esters such as isopropyl myristrate, fatty acid derived PEG esters such as PEG-hydroxyoleate and PEG-hydroxystearate, N-methyl pyrrolidinone, pluronic 60, polyoxyethylene sorbitol oleic polyesters such as poly(ethoxylated)₃₀₋₆₀ sorbitol poly(oleate)_{2-4,} poly(oxyethylene)₁₅₋₂₀ monooleate, poly(oxyethylene)₁₅₋₂₀ mono 12-hydroxystearate, and poly(oxyethylene)₁₅₋₂₀ mono ricinoleate, polyoxyethylene sorbitan esters such as polyoxyethylene-sorbitan monooleate, polyoxyethylene-sorbitan monopalmitate, polyoxyethylene-sorbitan monolaurate, polyoxyethylene-sorbitan monostearate, and Polysorbate® 20, 40, 60 or 80 from ICI Americas, Wilmington, DE, polyvinylpyrrolidone, alkyleneoxy modified fatty acid esters such as polyoxyl 40 hydrogenated castor oil and polyoxyethylated castor oils (e.g., Cremophor® EL solution or Cremophor® RH 40 solution), saccharide fatty acid esters (i.e., the condensation product of a monosaccharide (e.g., pentoses such as ribose, ribulose, arabinose, xylose, lyxose and xylulose, hexoses such as glucose, fructose, galactose, mannose and sorbose, trioses, tetroses, heptoses, and octoses), disaccharide (e.g., sucrose, maltose, lactose and trehalose) or oligosaccharide or mixture thereof with a C₄-C₂₂ fatty acid(s)(e.g., saturated fatty acids such as caprylic acid, capric acid, lauric acid, myristic acid, palmitic acid and stearic acid, and unsaturated fatty acids such as palmitoleic acid, oleic acid, elaidic acid, erucic acid and linoleic acid)), or steroidal esters); alkyl, aryl, or cyclic ethers having 2-30 carbon atoms (e.g., diethyl ether, tetrahydrofuran, dimethyl isosorbide, diethylene glycol monoethyl ether); glycofurol (tetrahydrofurfuryl alcohol polyethylene glycol ether); ketones having 3-30 carbon atoms (e.g., acetone, methyl ethyl ketone, methyl isobutyl ketone); aliphatic, cycloaliphatic or aromatic hydrocarbons having 4-30 carbon atoms (e.g., benzene, cyclohexane, dichloromethane, dioxolanes, hexane, n-decane, n-dodecane, n-hexane, sulfolane, tetramethylenesulfon, tetramethylenesulfoxide, toluene, dimethylsulfoxide (DMSO), or tetramethylenesulfoxide); oils of mineral, vegetable, animal, essential or synthetic origin (e.g., mineral oils such as aliphatic or wax-based hydrocarbons, aromatic hydrocarbons, mixed aliphatic and aromatic based hydrocarbons, and refined paraffin oil, vegetable oils such as linseed, tung, safflower, soybean, castor, cottonseed, groundnut, rapeseed, coconut, palm, olive, corn, corn germ, sesame, persic and peanut oil and glycerides such as mono-, di- or triglycerides, animal oils such as fish, marine, sperm, cod-liver, haliver, squalene, squalane, and shark liver oil, oleic oils, and polyoxyethylated castor oil); alkyl or aryl: halides having 1-30 carbon atoms and optionally more than one halogen substituent; methylene chloride; monoethanolamine; petroleum benzin; trolamine; omega-3 polyunsaturated fatty acids (e.g., alpha-linolenic acid, eicosapentaenoic acid, docosapentaenoic acid, or docosahexaenoic acid); polyglycol ester of 12-hydroxystearic acid and polyethylene glycol (Solutol® HS-15, from BASF, Ludwigshafen, Germany); polyoxyethylene glycerol; sodium laurate; sodium oleate; or sorbitan monooleate.

Other pharmaceutically acceptable solvents for use in the invention are well known to those of ordinary skill in the art, and are identified in The Chemotherapy Source Book (Williams & Wilkens Publishing), The Handbook of Pharmaceutical Excipients, (American Pharmaceutical Association, Washington, D.C., and The Pharmaceutical Society of Great Britain, London, England, 1968), Modern Pharmaceutics, (G. Banker et al., eds., 3d ed.)(Marcel Dekker, Inc., New York, New York, 1995), The Pharmacological Basis of Therapeutics, (Goodman & Gilman, McGraw Hill Publishing), Pharmaceutical Dosage Forms, (H. Lieberman et al., eds., )(Marcel Dekker, Inc., New York, New York, 1980), Remington's Pharmaceutical Sciences (A. Gennaro, ed., 19th ed.)(Mack Publishing, Easton, PA, 1995), The United States Pharmacopeia 24, The National Formulary 19, (National Publishing, Philadelphia, PA, 2000), A.J. Spiegel et al., and Use of Nonaqueous Solvents in Parenteral Products, JOURNAL OF PHARMACEUTICAL SCIENCES, Vol. 52, No. 10, pp. 917-927 (1963).

Preferred solvents include those known to stabilize the antitumor compounds, such as oils rich in triglycerides, for example, safflower oil, soybean oil or mixtures thereof, and alkyleneoxy modified fatty acid esters such as polyoxyl 40 hydrogenated castor oil and polyoxyethylated castor oils (e.g., Cremophor® EL solution or Cremophor® RH 40 solution). Commercially available triglycerides include Intralipid® emulsified soybean oil (Kabi-Pharmacia Inc., Stockholm, Sweden), Nutralipid ® emulsion (McGaw, Irvine, California), Liposyn® II 20% emulsion (a 20% fat emulsion solution containing 100 mg safflower oil, 100 mg soybean oil, 12 mg egg phosphatides, and 25 mg glycerin per ml of solution; Abbott Laboratories, Chicago, Illinois), Liposyn® III 2% emulsion (a 2% fat emulsion solution containing 100 mg safflower oil, 100 mg soybean oil, 12 mg egg phosphatides, and 25 mg glycerin per ml of solution; Abbott Laboratories, Chicago, Illinois), natural or synthetic glycerol derivatives containing the docosahexaenoyl group at levels between 25% and 100% by weight based on the total fatty acid content (Dhasco® (from Martek Biosciences Corp., Columbia, MD), DHA Maguro® (from Daito Enterprises, Los Angeles, CA), Soyacal®, and Travemulsion®. Ethanol is a preferred solvent for use in dissolving the antitumor compound to form solutions, emulsions, and the like.

Additional minor components can be included in the compositions of the invention for a variety of purposes well known in the pharmaceutical industry. These components will for the most part impart properties which enhance retention of the antitumor compound at the site of administration, protect the stability of the composition, control the pH, facilitate processing of the antitumor compound into pharmaceutical formulations, and the like. Preferably, each of these components is individually present in less than about 15 weight % of the total composition, more preferably less than about 5 weight %, and most preferably less than about 0.5 weight % of the total composition. Some components, such as fillers or diluents, can constitute up to 90 wt.% of the total composition, as is well known in the formulation art. Such additives include cryoprotective agents for preventing reprecipitation of the taxane, surface active, wetting or emulsifying agents (e.g., lecithin, polysorbate-80, Tween® 80, pluronic 60, polyoxyethylene stearate ), preservatives (e.g., ethyl-p-hydroxybenzoate), microbial preservatives (e.g., benzyl alcohol, phenol, m-cresol, chlorobutanol, sorbic acid, thimerosal and paraben), agents for adjusting pH or buffering agents (e.g., acids, bases, sodium acetate, sorbitan monolaurate), agents for adjusting osmolarity (e.g., glycerin), thickeners (e.g., aluminum monostearate, stearic acid, cetyl alcohol, stearyl alcohol, guar gum, methyl cellulose, hydroxypropylcellulose, tristearin, cetyl wax esters, polyethylene glycol), colorants, dyes, flow aids, non-volatile silicones (e.g., cyclomethicone), clays (e.g., bentonites), adhesives, bulking agents, flavorings, sweeteners, adsorbents, fillers (e.g., sugars such as lactose, sucrose, mannitol, or sorbitol, cellulose, or calcium phosphate), diluents (e.g., water, saline, electrolyte solutions), binders (e.g., starches such as maize starch, wheat starch, rice starch, or potato starch, gelatin, gum tragacanth, methyl cellulose, hydroxypropyl methylcellulose, sodium carboxymethyl cellulose, polyvinylpyrrolidone, sugars, polymers, acacia), disintegrating agents (e.g., starches such as maize starch, wheat starch, rice starch, potato starch, or carboxymethyl starch, cross-linked polyvinyl pyrrolidone, agar, alginic acid or a salt thereof such as sodium alginate, croscarmellose sodium or crospovidone), lubricants (e.g., silica, talc, stearic acid or salts thereof such as magnesium stearate, or polyethylene glycol), coating agents (e.g., concentrated sugar solutions including gum arabic, talc, polyvinyl pyrrolidone, carbopol gel, polyethylene glycol, or titanium dioxide), and antioxidants (e.g., sodium metabisulfite, sodium bisulfite, sodium sulfite, dextrose, phenols, and thiophenols).

In a preferred embodiment, a pharmaceutical composition of the invention comprises at least one nonaqueous, pharmaceutically acceptable solvent and an antitumor compound having a solubility in ethanol of at least about 100, 200, 300, 400, 500, 600, 700 or 800 mg/ml. While not being bound to a particular theory, it is believed that the ethanol solubility of the antitumor compound may be directly related to its efficacy. The antitumor compound can also be capable of being crystallized from a solution. In other words, a crystalline antitumor compound can be dissolved in a solvent to form a solution and then recrystallized upon evaporation of the solvent without the formation of any amorphous antitumor compound. It is also preferred that the antitumor compound have an ID50 value (i.e, the drug concentration producing 50% inhibition of colony formation) of at least 4, 5, 6, 7, 8, 9, or 10 times less that of paclitaxel when measured according to the protocol set forth in the working examples.

Dosage form administration by these routes may be continuous or intermittent, depending, for example, upon the patient's physiological condition, whether the purpose of the administration is therapeutic or prophylactic, and other factors known to and assessable by a skilled practitioner.

Dosage and regimens for the administration of the pharmaceutical compositions of the invention can be readily determined by those with ordinary skill in treating cancer. It is understood that the dosage of the antitumor compounds will be dependent upon the age, sex, health, and weight of the recipient, kind of concurrent treatment, if any, frequency of treatment, and the nature of the effect desired. For any mode of administration, the actual amount of antitumor compound delivered, as well as the dosing schedule necessary to achieve the advantageous effects described herein, will also depend, in part, on such factors as the bioavailability of the antitumor compound, the disorder being treated, the desired therapeutic dose, and other factors that will be apparent to those of skill in the art. The dose administered to an animal, particularly a human, in the context of the present invention should be sufficient to effect the desired therapeutic response in the animal over a reasonable period of time. Preferably, an effective amount of the antitumor compound, whether administered orally or by another route, is any amount which would result in a desired therapeutic response when administered by that route. Preferably, the compositions for oral administration are prepared in such a way that a single dose in one or more oral preparations contains at least 20 mg of the antitumor compound per m² of patient body surface area, or at least 50, 100, 150, 200, 300, 400, or 500 mg of the antitumor compound per m² of patient body surface area, wherein the average body surface area for a human is 1.8 m². Preferably, a single dose of a composition for oral administration contains from about 20 to about 600 mg of the antitumor compound per m² of patient body surface area, more preferably from about 25 to about 400 mg/m^{2,} even more preferably, from about 40 to about 300 mg/m², and even more preferably from about 50 to about 200 mg/m². Preferably, the compositions for parenteral administration are prepared in such a way that a single dose contains at least 20 mg of the antitumor compound per m² of patient body surface area, or at least 40, 50, 100, 150, 200, 300, 400, or 500 mg of the antitumor compound per m² of patient body surface area. Preferably, a single dose in one or more parenteral preparations contains from about 20 to about 500 mg of the antitumor compound per m² of patient body surface area, more preferably from about 40 to about 400 mg/m² and even more preferably, from about 60 to about 350 mg/m². However, the dosage may vary depending on the dosing schedule which can be adjusted as necessary to achieve the desired therapeutic effect. It should be noted that the ranges of effective doses provided herein are not intended to limit the invention and represent preferred dose ranges. The most preferred dosage will be tailored to the individual subject, as is understood and determinable by one of ordinary skill in the art without undue experimentation.

The concentration of the antitumor compound in a liquid pharmaceutical composition is preferably between about 0.01 mg and about 10 mg per ml of the composition, more preferably between about 0.1 mg and about 7 mg per ml, even more preferably between about 0.5 mg and about 5 mg per ml, and most preferably between about 1.5 mg and about 4 mg per ml. Relatively low concentrations are generally preferred because the antitumor compound is most soluble in the solution at low concentrations. The concentration of the antitumor compound in a solid pharmaceutical composition for oral administration is preferably between about 5 weight % and about 50 weight %, based on the total weight of the composition, more preferably between about 8 weight % and about 40 weight %, and most preferably between about 10 weight % and about 30 weight %.

In one embodiment, solutions for oral administration are prepared by dissolving an antitumor compound in any pharmaceutically acceptable solvent capable of dissolving the compound (e.g., ethanol or methylene chloride) to form a solution. An appropriate volume of a carrier which is a solution, such as Cremophor® EL solution, is added to the solution while stirring to form a pharmaceutically acceptable solution for oral administration to a patient. If desired, such solutions can be formulated to contain a minimal amount of, or to be free of, ethanol, which is known in the art to cause adverse physiological effects when administered at certain concentrations in oral formulations.

In another embodiment, powders or tablets for oral administration are prepared by dissolving an antitumor compound in any pharmaceutically acceptable solvent capable of dissolving the compound (e.g.,ethanol or methylene chloride) to form a solution. The solvent can optionally be capable of evaporating when the solution is dried under vacuum. An additional carrier can be added to the solution prior to drying, such as Cremophor® EL solution. The resulting solution is dried under vacuum to form a glass. The glass is then mixed with a binder to form a powder. The powder can be mixed with fillers or other conventional tabletting agents and processed to form a tablet for oral administration to a patient. The powder can also be added to any liquid carrier as described above to form a solution, emulsion, suspension or the like for oral administration.

Emulsions for parenteral administration can be prepared by dissolving an antitumor compound in any pharmaceutically acceptable solvent capable of dissolving the compound (e.g., ethanol or methylene chloride) to form a solution. An appropriate volume of a carrier which is an emulsion, such as Liposyn® II or Liposyn® III emulsion, is added to the solution while stirring to form a pharmaceutically acceptable emulsion for parenteral administration to a patient. If desired, such emulsions can be formulated to contain a minimal amount of, or to be free of, ethanol or Cremophor® solution, which are known in the art to cause adverse physiological effects when administered at certain concentrations in parenteral formulations.

Solutions for parenteral administration can be prepared by dissolving an antitumor compound in any pharmaceutically acceptable solvent capable of dissolving the compound (e.g., ethanol or methylene chloride) to form a solution. An appropriate volume of a carrier which is a solution, such as Cremophor® solution, is added to the solution while stirring to form a pharmaceutically acceptable solution for parenteral administration to a patient. If desired, such solutions can be formulated to contain a minimal amount of, or to be free of, ethanol or Cremophor® solution, which are known in the art to cause adverse physiological effects when administered at certain concentrations in parenteral formulations.

If desired, the emulsions or solutions described above for oral or parenteral administration can be packaged in IV bags, vials or other conventional containers in concentrated form and diluted with any pharmaceutically acceptable liquid, such as saline, to form an acceptable taxane concentration prior to use as is known in the art.

### Definitions

The terms "hydrocarbon" and "hydrocarbyl" as used herein describe organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The "substituted hydrocarbyl" moieties described herein are hydrocarbyl moieties which are substituted with at least one atom other than carbon, including moieties in which a carbon chain atom is substituted with a hetero atom such as nitrogen, oxygen, silicon, phosphorous, boron, sulfur, or a halogen atom. These substituents include halogen, heterocyclo, alkoxy, alkenoxy, alkynoxy, aryloxy, hydroxy, protected hydroxy, keto, acyl, acyloxy, nitro, amino, amido, nitro, cyano, thiol, ketals, acetals, esters and ethers.

The term "heteroatom" shall mean atoms other than carbon and hydrogen.

The "heterosubstituted methyl" moieties described herein are methyl groups in which the carbon atom is covalently bonded to at least one heteroatom and optionally with hydrogen, the heteroatom being, for example, a nitrogen, oxygen, silicon, phosphorous, boron, sulfur, or halogen atom. The heteroatom may, in turn, be substituted with other atoms to form a heterocyclo, alkoxy, alkenoxy, alkynoxy, aryloxy, hydroxy, protected hydroxy, oxy, acyloxy, nitro, amino, amido, thiol, ketals, acetals, esters or ether moiety.

The "heterosubstituted acetate" moieties described herein are acetate groups in which the carbon of the methyl group is covalently bonded to at least one heteroatom and optionally with hydrogen, the heteroatom being, for example, a nitrogen, oxygen, silicon, phosphorous, boron, sulfur, or halogen atom. The heteroatom may, in turn, be substituted with other atoms to form a heterocyclo, alkoxy, alkenoxy, alkynoxy, aryloxy, hydroxy, protected hydroxy, oxy, acyloxy, nitro, amino, amido, thiol, ketals, acetals, esters or ether moiety.

Unless otherwise indicated, the alkyl groups described herein are preferably lower alkyl containing from one to eight carbon atoms in the principal chain and up to 20 carbon atoms. They may be straight or branched chain or cyclic and include methyl, ethyl, propyl, isopropyl, butyl, hexyl and the like.

Unless otherwise indicated, the alkenyl groups described herein are preferably lower alkenyl containing from two to eight carbon atoms in the principal chain and up to 20 carbon atoms. They may be straight or branched chain or cyclic and include ethenyl, propenyl, isopropenyl, butenyl, isobutenyl, hexenyl, and the like.

Unless otherwise indicated, the alkynyl groups described herein are preferably lower alkynyl containing from two to eight carbon atoms in the principal chain and up to 20 carbon atoms. They may be straight or branched chain and include ethynyl, propynyl, butynyl, isobutynyl, hexynyl, and the like.

The terms "aryl" or "ar" as used herein alone or as part of another group denote optionally substituted homocyclic aromatic groups, preferably monocyclic or bicyclic groups containing from 6 to 12 carbons in the ring portion, such as phenyl, biphenyl, naphthyl, substituted phenyl, substituted biphenyl or substituted naphthyl. Phenyl and substituted phenyl are the more preferred aryl.

The terms "halogen" or "halo" as used herein alone or as part of another group refer to chlorine, bromine, fluorine, and iodine.

The terms "heterocyclo" or "heterocyclic" as used herein alone or as part of another group denote optionally substituted, fully saturated or unsaturated, monocyclic or bicyclic, aromatic or nonaromatic groups having at least one heteroatom in at least one ring, and preferably 5 or 6 atoms in each ring. The heterocyclo group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heterocyclo include heteroaromatics such as furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "heteroaromatic" as used herein alone or as part of another group denote optionally substituted aromatic groups having at least one heteroatom in at least one ring, and preferably 5 or 6 atoms in each ring. The heteroaromatic group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heteroaromatics include furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "acyl," as used herein alone or as part of another group, denotes the moiety formed by removal of the hydroxyl group from the group --COOH of an organic carboxylic acid, e.g., RC(O)-, wherein R is R¹, R¹O-, R¹R²N-, or R¹S-, R¹ is hydrocarbyl, heterosubstituted hydrocarbyl, or heterocyclo and R² is hydrogen, hydrocarbyl or substituted hydrocarbyl.

The term "acyloxy," as used herein alone or as part of another group, denotes an acyl group as described above bonded through an oxygen linkage (--O--), e.g., RC(O)O- wherein R is as defined in connection with the term "acyl."

Unless otherwise indicated, the alkoxycarbonyloxy moieties described herein comprise lower hydrocarbon or substituted hydrocarbon or substituted hydrocarbon moieties.

Unless otherwise indicated, the carbamoyloxy moieties described herein are derivatives of carbamic acid in which one or both of the amine hydrogens is optionally replaced by a hydrocarbyl, substituted hydrocarbyl or heterocyclo moiety.

The terms "hydroxyl protecting group" and "hydroxy protecting group" as used herein denote a group capable of protecting a free hydroxyl group ("protected hydroxyl") which, subsequent to the reaction for which protection is employed, may be removed without disturbing the remainder of the molecule. A variety of protecting groups for the hydroxyl group and the synthesis thereof may be found in "Protective Groups in Organic Synthesis" by T. W. Greene, John Wiley and Sons, 1981, or Fieser & Fieser. Exemplary hydroxyl protecting groups include methoxymethyl, 1-ethoxyethyl, benzyloxymethyl, (.beta.-trimethylsilylethoxy)methyl, tetrahydropyranyl, 2,2,2-trichloroethoxycarbonyl, t-butyl(diphenyl)silyl, trialkylsilyl, trichloromethoxycarbonyl and 2,2,2-trichloroethoxymethyl.

As used herein, "Ac" means acetyl; "Bz" means benzoyl; "Et" means ethyl; "Me" means methyl; "Ph" means phenyl; "iPr" means isopropyl; "tBu" and "t-Bu" means tert-butyl; "R" means lower alkyl unless otherwise defined; "py" means pyridine or pyridyl; "TES" means triethylsilyl; "TMS" means trimethylsilyl; "LAH" means lithium aluminum hydride; "10-DAB" means 10-desacetylbaccatin III"; "amine protecting group" includes, but is not limited to, carbamates, for example, 2,2,2-trichloroethylcarbamate or tertbutylcarbamate; "protected hydroxy" means -OP wherein P is a hydroxy protecting group; "tBuOCO" and "BOC" mean tert-butoxycarbonyl; "tAmOCO" means tert-amyloxycarbonyl; "PhCO means phenylcarbonyl"; "2-FuCO" means 2-furylcarbonyl; "2-ThCO" means 2-thienylcarbonyl; "2-PyCO" means 2-pyridylcarbonyl; "3-PyCO" means 3-pyridylcarbonyl; "4-PyCO" means 4-pyridylcarbonyl; "C₄H₇CO" means butenylcarbonyl; "EtOCO" means ethoxycarbonyl; "ibueCO" means isobutenylcarbonyl; "iBuCO" means isobutylcarbonyl; "iBuOCO" means isobutoxycarbonyl; "iPrOCO" means isopropyloxycarbonyl; "nPrOCO" means n-propyloxycarbonyl; "nPrCO" means n-propylcarbonyl; "tC₃H₅CO" means *trans*-propenyl carbonyl"; "ibue" means isobutenyl; "THF" means tetrahydrofuran; "DMAP" means 4-dimethylamino pyridine; "LHMDS" means Lithium HexamethylDiSilazanide.

The following examples illustrate the invention.

### Example 1

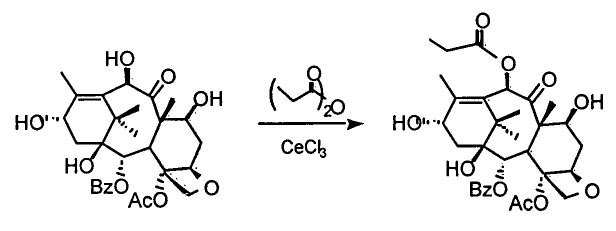

**10-Propionyl-10-deacetyl baccatin III.** To a mixture of 0.2 g (0.367 mmol) of 10-deacetyl baccatin III and 0.272 g (1.10 mmol) of CeCl₃ in 10 mL of THF at 25 °C was added 2.35 mL (18.36 mmol) of propionic anhydride. After 30 min the reaction mixture was diluted with 200 mL of EtOAc, then washed three times with 50 mL of saturated aqueous NaHCO₃ solution and brine. The organic extract was dried over Na₂SO₄ and concentrated *in vacuo.* The crude solid was purified by flash column chromatography on silica gel using 70% EtOAc/hexane as eluent to give 0.199 g (90%) of 10-propionyl-10-deacetyl baccatin III as a solid. 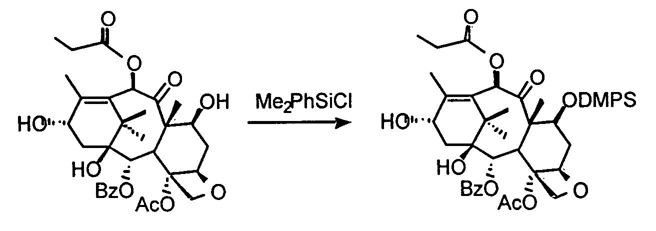 **7-Dimethylphenylsilyl-10-propionyl-10-deacetyl baccatin III.** To a solution of 0.200 g (0.333 mmol) of 10-propionyl-10-deacetyl baccatin III in 12 mL of THF at -10 °C under a nitrogen atmosphere was added dropwise 0.668 mL (4.00 mmol) of chlorodimethyl-phenylsilane and 2.48 mL (30.64 mmol) of pyridine. After 90 min the mixture was diluted with 100 mL of a 1:1 mixture of ethyl acetate and hexane. The mixture was washed with 20 mL of saturated aqueous sodium bicarbonate solution and the organic layer separated. The aqueous layer was extracted with 30 mL of a 1:1 mixture of ethyl acetate and hexane, and the combined organic extracts were washed with brine, dried over Na₂SO₄, and concentrated *in vacuo.* The crude solid was purified by flash column chromatography on silica gel using 50% EtOAc/hexane as eluent to give 0.242 g (99%) of 7-dimethylphenylsilyl-10-propionyl-10-deacetyl baccatin III as a solid. 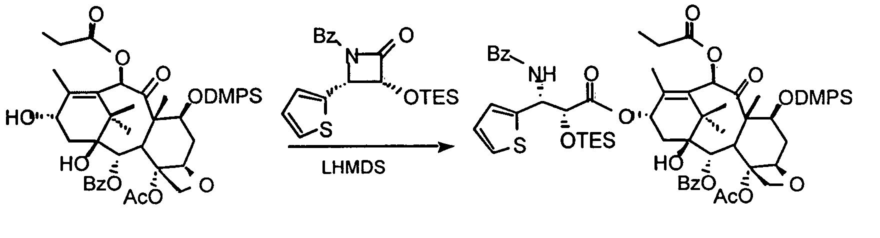 **7-Dimethylphenylsilyl-2'-O-triethylsilyl-3'-desphenyl-3'-(2-thienyl)-10-propionyl-10-deacetyl taxol.** To a solution of 0.400 g (0.544 mmol) of 7-dimethylphenylsilyl-10-propionyl-10-deacetyl baccatin III in 5.5 mL of THF at -45 °C under a nitrogen atmosphere was added 0.681 mL (0.681 mmol) of a 1M solution of LHMDS in THF. After 1 h, a solution of 0.317 g (0.818 mmol) of *cis*-N-benzoyl-3-triethylsilyloxy-4-(2-thienyl) azetidin-2-one in 3 mL of THF was added slowly. The mixture was warmed to 0 °C and after 3 h 10 mL of saturated aqueous sodium bicarbonate solution was added and the mixture was extracted three times with 50 mL of ethyl acetate. The combined organic extracts were washed with brine, dried over Na₂SO₄, and concentrated *in vacuo.* The crude product was purified by flash column chromatography on silica gel using 40% EtOAc/hexane as eluent to give 0.531 g (87%) of 7-dimethylphenylsilyl-2'-O-triethylsilyl-3'-desphenyl-3'-(2-thienyl)-10-propionyl-10-deacetyl taxol as a solid. 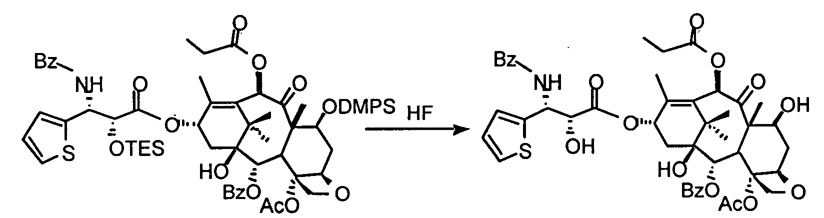 **3'-Desphenyl-3'-(2-thienyl)-10-propionyl-10-deacetyl taxol.** To a solution of 0.521 g (0.464 mmol) of 7-dimethylphenylsilyl-2'-O-triethylsilyl-3'-desphenyl-3'-(2-thienyl)-10-propionyl-10-deacetyl taxol in 2 mL of CH3CN and 2 mL of pyridine at 0 °C was added 0.5 mL of a solution of 30% HF in H₂O. After 3 h 20 mL of a saturated aqueous sodium bicarbonate solution was added and the mixture was extracted three times with 50 mL of ethyl acetate. The combined organic extracts were washed with brine, dried over Na₂SO₄, and concentrated *in vacuo.* The crude product was purified by flash column chromatography on silica gel using 70% EtOAc/hexane as eluent to give 0.405 g (100%) of 3'-desphenyl-3'-(2-thienyl)-10-propionyl-10-deacetyl taxol as a solid. m.p. 154-155 °C; [α]_{D}²⁵ = -45.0 (*c* 0.1 in CHCl3); Anal. Calcd. for C₄₆H₅₁NO₁₄S: C, 63.22; H, 5.88; Found: C, 62.94; H, 5.97.

| **3'-Desphenyl-3'-(2-thienyl)-10-propionyl-10-deacetyl taxol** ^{**1**}**H NMR data (CDCl**_{**3**}**)** | | | |
|---|---|---|---|
| **Proton** | **δ (ppm)** | **pattern** | **J (Hz)** |
| 2' | 4.78 | dd | H3'(2.1), 2'OH(4.1) |
| 2'OH | 3.51 | d | H2'(4.1) |
| 3' | 6.07 | dd | NH(8.6), H2'(2.1) |
| 5' | 7.04 | dd | (3.5), (5.0) |
| 1OH | 1.68 | s | |
| 2 | 5.69 | d | H3(7.0) |
| 3 | 3.85 | d | H2(7.0) |
| 4Ac | 2.42 | s | |
| 5 | 4.96 | app d | |
| 6α | 2.45-2.60 | app m | |
| 6β | 1.89 | ddd | H7(10.9), H5(2.5), H6α(14.5) |
| 7 | 4.42 | ddd | 7OH(4.2), H6α(6.8), H6β(10.8) |
| 7OH | 2.45-2.60 | app m | |
| 10 | 6.32 | s | |
| 13 | 6.27 | app t | H14α,β(9.0) |
| 14α | 2.40-2.43 | app m | |
| 14β | 2.34 | dd | H14α(15.5), H13(9.0) |
| Me 16 | 1.16 | s | |
| Me 17 | 1.25 | app m | |
| Me18 | 1.84 | s | |
| Me19 | 1.70 | s | |
| 20α | 4.31 | d | H20β(8.5) |
| 20β | 4.22 | d | H20α(8.5) |
| o-benzoate | 8.14-8.16 | m | |
| o-benzamide | 7.72-7.73 | m | |
| NH | 6.88 | d | H3'(8.6) |
| CH3CH2 | 1.24 | t | CH3CH2(7.0) |
| CH3CH2 | 2.45-2.60 | app m | |

### Example 2

The procedures described in Example 1 were repeated, but other suitably protected β-lactams were substituted for the β-lactam of Example 1 to prepare the series of compounds having structural formula (13) and the combinations of substituents identified in the following table

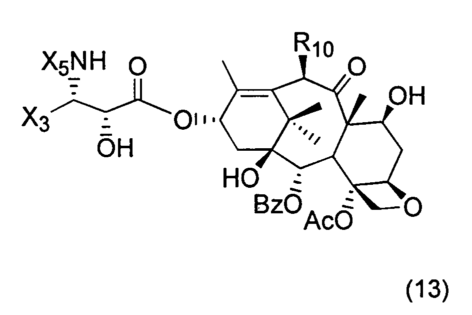

| **Compound** | **X**_{**5**} | **X**_{**3**} | **R**_{**10**} |
|---|---|---|---|
| 0499 | tBuOCO- | Isobutenyl | EtCOO- |
| 0503 | tBuOCO- | 2-pyridyl | EtCOO- |
| 0517 | tBuOCO- | 3-pyridyl | EtCOO- |
| 0521 | tBuOCO- | 4-pyridyl | EtCOO- |
| 0536 | tBuOCO- | 2-furyl | EtCOO- |
| 0549 | tBuOCO- | 3-furyl | EtCOO- |
| 0550 | tBuOCO- | 2-thienyl | EtCOO- |
| 0562 | tBuOCO- | 3-thienyl | EtCOO- |
| 0578 | tBuOCO- | Cyclopropyl | EtCOO- |
| 0583 | tBuOCO- | Isopropyl | EtCOO- |
| 0596 | tBuOCO- | Cyclobutyl | EtCOO- |
| 0602 | tBuOCO- | p-nitrophenyl | EtCOO- |
| 0611 | tBuOCO- | Phenyl | EtCOO- |
| 0625 | PhCO- | Isobutenyl | EtCOO- |
| 0634 | PhCO- | 2-pyridyl | EtCOO- |
| 0647 | PhCO- | 3-pyridyl | EtCOO- |
| 0659 | PhCO- | 4-pyridyl | EtCOO- |
| 0663 | PhCO- | 2-furyl | EtCOO- |
| 0670 | PhCO- | 3-furyl | EtCOO- |
| 0687 | PhCO- | 2-thienyl | EtCOO- |
| 0691 | PhCO- | 3-thienyl | EtCOO- |
| 0706 | PhCO- | Cyclopropyl | EtCOO- |
| 0719 | PhCO- | Isopropyl | EtCOO- |
| 0720 | PhCO- | Cyclobutyl | EtCOO- |
| 0732 | PhCO- | p-nitrophenyl | EtCOO- |
| 0748 | PhCO- | Phenyl | EtCOO- |
| 0838 | tBuOCO- | Isobutenyl | CproCOO- |
| 0843 | tBuOCO- | 2-furyl | CproCOO- |
| 0854 | tBuOCO- | 2-thienyl | CproCOO- |
| 0860 | tBuOCO- | Cyclopropyl | CproCOO- |
| 0879 | tBuOCO- | p-nitrophenyl | CproCOO- |
| 0882 | tBuOCO- | Phenyl | CproCOO- |
| 0890 | PhCO- | Isobutenyl | CproCOO- |
| 0908 | PhCO- | 2-furyl | CproCOO- |
| 0919 | PhCO- | 2-thienyl | CproCOO- |
| 0923 | PhCO- | Cyclopropyl | CproCOO- |
| 0937 | PhCO- | Phenyl | CproCOO- |
| 0947 | tBuOCO- | Isobutenyl | PrCOO- |
| 0951 | tBuOCO- | 2-pyridyl | PrCOO- |
| 0966 | tBuOCO- | 3-pyridyl | PrCOO- |
| 0978 | tBuOCO- | 4-pyridyl | PrCOO- |
| 0983 | tBuOCO- | 2-furyl | PrCOO- |
| 0999 | tBuOCO- | 3-furyl | PrCOO- |
| 1003 | tBuOCO- | 2-thienyl | PrCOO- |
| 1011 | tBuOCO- | 3-thienyl | PrCOO- |
| 1020 | tBuOCO- | Cyclopropyl | PrCOO- |
| 1031 | tBuOCO- | Isopropyl | PrCOO- |
| 1044 | tBuOCO- | Cyclobutyl | PrCOO- |
| 1060 | tBuOCO- | Phenyl | PrCOO- |
| 1879 | tBuOCO- | Isobutenyl | 2-ThCOO- |
| 1883 | tBuOCO- | 2-pyridyl | 2-ThCOO- |
| 1892 | tBuOCO- | 2-furyl | 2-ThCOO- |
| 1900 | tBuOCO- | 2-thienyl | 2-ThCOO- |
| 1911 | tBuOCO- | p-nitrophenyl | 2-ThCOO- |
| 1923 | tBuOCO- | 3-furyl | 2-ThCOO- |
| 1939 | tBuOCO- | 3-thienyl | 2-ThCOO- |
| 1948 | tBuOCO- | 3-pyridyl | 2-ThCOO- |
| 1954 | tBuOCO- | 4-pyridyl | 2-ThCOO- |
| 1964 | tBuOCO- | Isopropyl | 2-ThCOO- |
| 1970 | tBuOCO- | Cyclobutyl | 2-ThCOO- |
| 1988 | tBuOCO- | Phenyl | 2-ThCOO- |
| 2101 | tBuOCO- | Isobutenyl | 2-FuCOO- |
| 2111 | tBuOCO- | 2-pyridyl | 2-FuCOO- |
| 2124 | tBuOCO- | 3-pyridyl | 2-FuCOO- |
| 2132 | tBuOCO- | 4-pyridyl | 2-FuCOO- |
| 2142 | tBuOCO- | 2-furyl | 2-FuCOO- |
| 2159 | tBuOCO- | 3-furyl | 2-FuCOO- |
| 2164 | tBuOCO- | 2-thienyl | 2-FuCOO- |
| 2173 | tBuOCO- | 3-thienyl | 2-FuCOO- |
| 2181 | tBuOCO- | Isopropyl | 2-FuCOO- |
| 2199 | tBuOCO- | Cyclobutyl | 2-FuCOO- |
| 2202 | tBuOCO- | p-nitrophenyl | 2-FuCOO- |
| 2212 | tBuOCO- | Phenyl | 2-FuCOO- |
| 2226 | tBuOCO- | Isobutenyl | IPrCOO- |
| 2238 | tBuOCO- | 2-pyridyl | IPrCOO- |
| 2242 | tBuOCO- | 3-pyridyl | IPrCOO- |
| 2255 | tBuOCO- | 4-pyridyl | IPrCOO- |
| 2269 | tBuOCO- | 2-furyl | IPrCOO- |
| 2273 | tBuOCO- | 3-furyl | IPrCOO- |
| 2287 | tBuOCO- | 2-thienyl | IPrCOO- |
| 2291 | tBuOCO- | 3-thienyl | IPrCOO- |
| 2306 | tBuOCO- | Isopropyl | IPrCOO- |
| 2319 | tBuOCO- | Cyclobutyl | IPrCOO- |
| 2320 | tBuOCO- | p-nitrophenyl | IprCOO- |
| 2332 | tBuOCO- | Isobutenyl | tC₃H₅COO- |
| 2348 | tBuOCO- | 2-pyridyl | tC₃H₅COO- |
| 2353 | tBuOCO- | 3-pyridyl | tC₃H₅COO- |
| 2366 | tBuOCO- | 4-pyridyl | tC₃H₅COO- |
| 2379 | tBuOCO- | 2-furyl | tC₃H₅COO- |
| 2380 | tBuOCO- | 3-furyl | tC₃H₅COO- |
| 2392 | tBuOCO- | 2-thienyl | tC₃H₅COO- |
| 2408 | tBuOCO- | 3-thienyl | tC₃H₅COO- |
| 2413 | tBuOCO- | Isopropyl | tC₃H₅COO- |
| 2424 | tBuOCO- | Cyclobutyl | tC₃H₅COO- |
| 2439 | tBuOCO- | p-nitrophenyl | tC₃H₅COO- |
| 2442 | tBuOCO- | Phenyl | tC₃H₅COO- |
| 2455 | tBuOCO- | Isobutenyl | IbueCOO- |
| 2464 | tBuOCO- | 2-pyridyl | IbueCOO- |
| 2472 | tBuOCO- | 4-pyridyl | IbueCOO- |
| 2488 | tBuOCO- | 2-furyl | IbueCOO- |
| 2499 | tBuOCO- | 3-furyl | IbueCOO- |
| 2503 | tBuOCO- | 2-thienyl | IbueCOO- |
| 2511 | tBuOCO- | 3-thienyl | IbueCOO- |
| 2520 | tBuOCO- | Phenyl | IbueCOO- |
| 2781 | tBuOCO- | 3-furyl | CproCOO- |
| 2794 | tBuOCO- | 3-thienyl | CproCOO- |
| 2802 | tBuOCO- | 2-pyridyl | CproCOO- |
| 2813 | tBuOCO- | 4-pyridyl | CproCOO- |
| 2826 | PhCO- | 3-furyl | CproCOO- |
| 2838 | PhCO- | 3-thienyl | CproCOO- |
| 2844 | PhCO- | 2-pyridyl | CproCOO- |
| 2855 | PhCO- | 4-pyridyl | CproCOO- |
| 2869 | PhCO- | p-nitrophenyl | CproCOO- |
| 3053 | 2-FuCO- | 2-thienyl | EtCOO- |
| 3071 | iPrOCO- | 2-thienyl | CproCOO- |
| 3096 | EtOCO- | 2-thienyl | PrCOO- |
| 3102 | iBuOCO- | 2-furyl | CproCOO- |
| 3110 | iBuOCO- | 2-furyl | PrCOO- |
| 3129 | iBuOCO- | 2-thienyl | CproCOO- |
| 3132 | nPrCO- | 2-thienyl | CproCOO- |
| 3148 | nPrCO- | 2-thienyl | PrCOO- |
| 3163 | iBuOCO- | 2-thienyl | EtCOO- |
| 3204 | PhCO- | 2-furyl | PrCOO- |
| 3219 | nPrCO- | 2-furyl | EtCOO- |
| 3222 | nPrCO- | 2-furyl | PrCOO- |
| 3258 | PhCO- | 2-thienyl | PrCOO- |
| 3265 | iBuOCO- | 2-thienyl | PrCOO- |
| 3297 | 2-FuCO- | 2-thienyl | CproCOO- |
| 3314 | nPrCO- | 2-thienyl | PrCOO- |
| 3352 | 2-FuCO- | 2-thienyl | PrCOO- |
| 3361 | iPrOCO- | 2-thienyl | EtCOO- |
| 3370 | EtOCO- | 2-thienyl | EtCOO- |
| 3408 | 2-ThCO- | 2-thienyl | PrCOO- |
| 3417 | iPrOCO- | 2-furyl | PrCOO- |
| 3425 | 2-ThCO- | 2-thienyl | EtCOO- |
| 3453 | 2-ThCO- | 2-thienyl | CproCOO- |
| 3482 | PhCO- | Cyclopropyl | PrCOO- |
| 3494 | tC₃H₅CO- | 2-thienyl | EtCOO- |
| 3513 | tC₃H₅CO- | 2-thienyl | CproCOO- |
| 3522 | iPrOCO- | 2-furyl | EtCOO- |
| 3535 | EtOCO- | 2-furyl | EtCOO- |
| 3543 | C₄H₇CO- | 2-thienyl | CproCOO- |
| 3588 | C₄H₇CO- | 2-thienyl | EtCOO- |
| 3595 | tC₃H₅CO- | 2-thienyl | PrCOO- |
| 3603 | C₄H₇CO- | 2-thienyl | PrCOO- |
| 3644 | 2-ThCO- | 2-furyl | EtCOO- |
| 3656 | 2-ThCO- | 2-furyl | PrCOO- |
| 3663 | 2-ThCO- | 2-furyl | CproCOO- |
| 3677 | EtOCO- | 2-furyl | CproCOO- |
| 3686 | 2-FuCO- | 2-furyl | PrCOO- |
| 3693 | EtOCO- | 2-furyl | PrCOO- |
| 3800 | C₄H₇CO- | 2-furyl | PrCOO- |
| 3818 | 2-FuCO- | 2-furyl | EtCOO- |
| 3853 | iPrOCO- | 2-furyl | CproCOO- |
| 3866 | 2-FuCO- | 2-furyl | CproCOO- |
| 3909 | iPrOCO- | 2-thienyl | PrCOO- |
| 3938 | C₄H₇CO- | 2-furyl | CproCOO- |
| 3945 | C₄H₇CO- | 2-furyl | EtCOO- |
| 3957 | iBuOCO- | 2-furyl | PrCOO- |
| 3971 | tC₃H₅CO- | 2-furyl | CproCOO- |
| 3982 | tC₃H₅CO- | 2-furyl | EtCOO- |
| 3994 | tC₃H₅CO- | 2-furyl | PrCOO- |
| 4051 | EtOCO- | 2-thienyl | CproCOO- |
| 4062 | nPrCO- | 2-furyl | CproCOO- |
| 4112 | 3-PyCO- | 2-thienyl | CproCOO- |
| 4121 | 3-PyCO- | 2-thienyl | EtCOO- |
| 4190 | 3-PyCO- | 2-thienyl | PrCOO- |
| 4207 | 4-PyCO- | 2-thienyl | EtCOO- |
| 4329 | ibueCO- | 2-thienyl | CproCOO- |
| 4335 | ibueCO- | 2-thienyl | EtCOO- |
| 4344 | ibueCO- | 2-thienyl | PrCOO- |
| 4665 | iBuOCO- | 3-furyl | CproCOO- |
| 4704 | iBuOCO- | 3-furyl | PrCOO- |
| 4711 | iBuOCO- | 3-thienyl | EtCOO- |
| 4720 | iBuOCO- | Isobutenyl | CproCOO- |
| 4799 | iBuOCO- | Cyclopropyl | EtCOO- |
| 4808 | iBuOCO- | Cyclopropyl | NPrCOO- |
| 4834 | iBuOCO- | 3-thienyl | NPrCOO- |
| 4888 | tC₃H₅CO- | 3-furyl | EtCOO- |
| 4919 | tC₃H₅CO- | 3-furyl | NPrCOO- |
| 4944 | tC₃H₅CO- | 3-furyl | CproCOO- |
| 5011 | iBuOCO- | 3-thienyl | CproCOO- |
| 5040 | tC₃H₅CO- | 3-thienyl | CproCOO- |
| 5065 | iBuOCO- | Isobutenyl | EtCOO- |
| 5144 | iBuOCO- | Isobutenyl | NprCOO- |
| 5232 | iBuOCO- | Cyclopropyl | CproCOO- |
| 5495 | tBuOCO- | 3-furyl | EtCOO- |
| 6522 | tAmOCO- | 2-furyl | EtCOO- |

### Example 3

Following the processes described in Example 1 and elsewhere herein, the following specific taxanes having structural formula 14 may be prepared, wherein R₁₀ is as previously defined, including wherein R₁₀ is R₁₀ₐCOO- and R₁₀ₐ is (i) substituted or unsubstituted C₂ to C₈ alkyl such as ethyl, or straight, branched or cyclic propyl, butyl, pentyl, or hexyl; (ii) substituted or unsubstituted C₂ to C₈ alkenyl such as ethenyl or straight, branched or cyclic propenyl, butenyl, pentenyl or hexenyl; (iii) substituted or unsubstituted C₂ to C₈ alkynyl such as ethynyl or straight or branched propynyl, butynyl, pentynyl, or hexynyl; (iv) substituted or unsubstituted phenyl, or (v) substituted or unsubstituted heteroaromatic such as furyl, thienyl, or pyridyl. The substituents may be those identified elsewhere herein for substituted hydrocarbyl. In one embodiment, R₁₀ may be R₁₀ₐCOO- wherein R₁₀ₐ is ethyl, straight, branched or cyclic propyl, straight or branched propenyl, isobutenyl, furyl or thienyl.

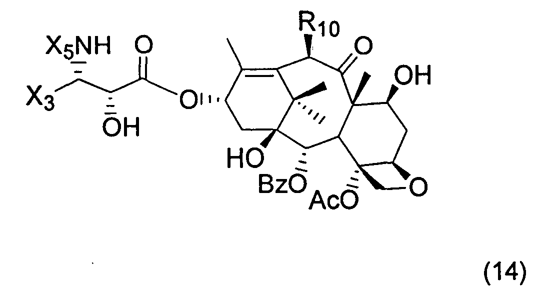

| **X**_{**5**} | **X**_{**3**} | **R**_{**10**} |
|---|---|---|
| tBuOCO- | 2-furyl | RₐCOO- |
| tBuOCO- | 3-furyl | RₐCOO- |
| tBuOCO- | 2-thienyl | RₐCOO- |
| tBuOCO- | 3-thienyl | RₐCOO- |
| tBuOCO- | 2-pyridyl | RₐCOO- |
| tBuOCO- | 3-pyridyl | RₐCOO- |
| tBuOCO- | 4-pyridyl | RₐCOO- |
| tBuOCO- | Isobutenyl | RₐCOO- |
| tBuOCO- | Isopropyl | RₐCOO- |
| tBuOCO- | Cyclopropyl | RₐCOO- |
| tBuOCO- | Cyclobutyl | RₐCOO- |
| tBuOCO- | Cyclopentyl | RₐCOO- |
| tBuOCO- | Phenyl | RₐCOO- |
| benzoyl | 2-furyl | RₐCOO- |
| benzoyl | 3-furyl | RₐCOO- |
| benzoyl | 2-thienyl | RₐCOO- |
| benzoyl | 3-thienyl | RₐCOO- |
| benzoyl | 2-pyridyl | RₐCOO- |
| benzoyl | 3-pyridyl | RₐCOO- |
| benzoyl | 4-pyridyl | RₐCOO- |
| benzoyl | Isobutenyl | RₐCOO- |
| benzoyl | Isopropyl | RₐCOO- |
| benzoyl | Cyclopropyl | RₐCOO- |
| benzoyl | Cyclobutyl | RₐCOO- |
| benzoyl | Cyclopentyl | RₐCOO- |
| benzoyl | Phenyl | RₐCOO- |
| 2-FuCO- | 2-furyl | RₐCOO- |
| 2-FuCO- | 3-furyl | RₐCOO- |
| 2-FuCO- | 2-thienyl | RₐCOO- |
| 2-FuCO- | 3-thienyl | RₐCOO- |
| 2-FuCO- | 2-pyridyl | RₐCOO- |
| 2-FuCO- | 3-pyridyl | RₐCOO- |
| 2-FuCO- | 4-pyridyl | RₐCOO- |
| 2-FuCO- | Isobutenyl | RₐCOO- |
| 2-FuCO- | Isopropyl | RₐCOO- |
| 2-FuCO- | Cyclopropyl | RₐCOO- |
| 2-FuCO- | Cyclobutyl | RₐCOO- |
| 2-FuCO- | Cyclopentyl | RₐCOO- |
| 2-FuCO- | Phenyl | RₐCOO- |
| 2-ThCO- | 2-furyl | RₐCOO- |
| 2-ThCO- | 3-furyl | RₐCOO- |
| 2-ThCO- | 2-thienyl | RₐCOO- |
| 2-ThCO- | 3-thienyl | RₐCOO- |
| 2-ThCO- | 2-pyridyl | RₐCOO- |
| 2-ThCO- | 3-pyridyl | RₐCOO- |
| 2-ThCO- | 4-pyridyl | RₐCOO- |
| 2-ThCO- | Isobutenyl | RₐCOO- |
| 2-ThCO- | Isopropyl | RₐCOO- |
| 2-ThCO- | Cyclopropyl | RₐCOO- |
| 2-ThCO- | Cyclobutyl | RₐCOO- |
| 2-ThCO- | Cyclopentyl | RₐCOO- |
| 2-ThCO- | Phenyl | RₐCOO- |
| 2-PyCO- | 2-furyl | RₐCOO- |
| 2-PyCO- | 3-furyl | RₐCOO- |
| 2-PyCO- | 2-thienyl | RₐCOO- |
| 2-PyCO- | 3-thienyl | RₐCOO- |
| 2-PyCO- | 2-pyridyl | RₐCOO- |
| 2-PyCO- | 3-pyridyl | RₐCOO- |
| 2-PyCO- | 4-pyridyl | RₐCOO- |
| 2-PyCO- | Isobutenyl | RₐCOO- |
| 2-PyCO- | Isopropyl | RₐCOO- |
| 2-PyCO- | Cyclopropyl | RₐCOO- |
| 2-PyCO- | Cyclobutyl | RₐCOO- |
| 2-PyCO- | Cyclopentyl | RₐCOO- |
| 2-PyCO- | Phenyl | RₐCOO- |
| 3-PyCO- | 2-furyl | RₐCOO- |
| 3-PyCO- | 3-furyl | RₐCOO- |
| 3-PyCO- | 2-thienyl | RₐCOO- |
| 3-PyCO- | 3-thienyl | RₐCOO- |
| 3-PyCO- | 2-pyridyl | RₐCOO- |
| 3-PyCO- | 3-pyridyl | RₐCOO- |
| 3-PyCO- | 4-pyridyl | RₐCOO- |
| 3-PyCO- | Isobutenyl | RₐCOO- |
| 3-PyCO- | Isopropyl | RₐCOO- |
| 3-PyCO- | Cyclopropy | RₐCOO- |
| 3-PyCO- | Cyclobutyl | RₐCOO- |
| 3-PyCO- | Cyclopentyl | RₐCOO- |
| 3-PyCO- | Phenyl | RₐCOO- |
| 4-PyCO- | 2-furyl | RₐCOO- |
| 4-PyCO- | 3-furyl | RₐCOO- |
| 4-PyCO- | 2-thienyl | RₐCOO- |
| 4-PyCO- | 3-thienyl | RₐCOO- |
| 4-PyCO- | 2-pyridyl | RₐCOO- |
| 4-PyCO- | 3-pyridyl | RₐCOO- |
| 4-PyCO- | 4-pyridyl | RₐCOO- |
| 4-PyCO- | Isobutenyl | RₐCOO- |
| 4-PyCO- | Isopropyl | RₐCOO- |
| 4-PyCO- | Cyclopropyl | RₐCOO- |
| 4-PyCO- | Cyclobutyl | RₐCOO- |
| 4-PyCO- | Cyclopentyl | RₐCOO- |
| 4-PyCO- | Phenyl | RₐCOO- |
| C₄H₇CO- | 2-furyl | RₐCOO- |
| C₄H₇CO- | 3-furyl | RₐCOO- |
| C₄H₇CO- | 2-thienyl | RₐCOO- |
| C₄H₇CO- | 3-thienyl | RₐCOO- |
| C₄H₇CO- | 2-pyridyl | RₐCOO- |
| C₄H₇CO- | 3-pyridyl | RₐCOO- |
| C₄H₇CO- | 4-pyridyl | RₐCOO- |
| C₄H₇CO- | Isobutenyl | RₐCOO- |
| C₄H₇CO- | Isopropyl | RₐCOO- |
| C₄H₇CO- | Cyclopropyl | RₐCOO- |
| C₄H₇CO- | Cyclobutyl | RₐCOO- |
| C₄H₇CO- | Cyclopentyl | RₐCOO- |
| C₄H₇CO- | Phenyl | RₐCOO- |
| EtOCO- | 2-furyl | RₐCOO- |
| EtOCO- | 3-furyl | RₐCOO- |
| EtOCO- | 2-thienyl | RₐCOO- |
| EtOCO- | 3-thienyl | RₐCOO- |
| EtOCO- | 2-pyridyl | RₐCOO- |
| EtOCO- | 3-pyridyl | RₐCOO- |
| EtOCO- | 4-pyridyl | RₐCOO- |
| EtOCO- | Isobutenyl | RₐCOO- |
| EtOCO- | Isopropyl | RₐCOO- |
| EtOCO- | Cyclopropyl | RₐCOO- |
| EtOCO- | Cyclobutyl | RₐCOO- |
| EtOCO- | Cyclopentyl | RₐCOO- |
| EtOCO- | Phenyl | RₐCOO- |
| ibueCO- | 2-furyl | RₐCOO- |
| ibueCO- | 3-furyl | RₐCOO- |
| ibueCO- | 2-thienyl | RₐCOO- |
| ibueCO- | 3-thienyl | RₐCOO- |
| ibueCO- | 2-pyridyl | RₐCOO- |
| ibueCO- | 3-pyridyl | RₐCOO- |
| ibueCO- | 4-pyridyl | RₐCOO- |
| ibueCO- | Isobutenyl | RₐCOO- |
| ibueCO- | Isopropyl | RₐCOO- |
| ibueCO- | Cyclopropyl | RₐCOO- |
| ibueCO- | Cyclobutyl | RₐCOO- |
| ibueCO- | Cyclopentyl | RₐCOO- |
| ibueCO- | Phenyl | RₐCOO- |
| iBuCO- | 2-furyl | RₐCOO- |
| iBuCO- | 3-furyl | RₐCOO- |
| iBuCO- | 2-thienyl | RₐCOO- |
| iBuCO- | 3-thienyl | RₐCOO- |
| iBuCO- | 2-pyridyl | RₐCOO- |
| iBuCO- | 3-pyridyl | RₐCOO- |
| iBuCO- | 4-pyridyl | RₐCOO- |
| iBuCO- | Isobutenyl | RₐCOO- |
| iBuCO- | Isopropyl | RₐCOO- |
| iBuCO- | Cyclopropyl | RₐCOO- |
| iBuCO- | Cyclobutyl | RₐCOO- |
| iBuCO- | Cyclopentyl | RₐCOO- |
| iBuCO- | Phenyl | RₐCOO- |
| iBuOCO- | 2-furyl | RₐCOO- |
| iBuOCO- | 3-furyl | RₐCOO- |
| iBuOCO- | 2-thienyl | RₐCOO- |
| iBuOCO- | 3-thienyl | RₐCOO- |
| iBuOCO- | 2-pyridyl | RₐCOO- |
| iBuOCO- | 3-pyridyl | RₐCOO- |
| iBuOCO- | 4-pyridyl | RₐCOO- |
| iBuOCO- | Isobutenyl | RₐCOO- |
| iBuOCO- | Isopropyl | RₐCOO- |
| iBuOCO- | Cyclopropy | RₐCOO- |
| iBuOCO- | Cyclobutyl | RₐCOO- |
| iBuOCO- | Cyclopentyl | RₐCOO- |
| iBuCO- | Phenyl | RₐCOO- |
| iPrOCO- | 2-furyl | RₐCOO- |
| iPrOCO- | 3-furyl | RₐCOO- |
| iPrOCO- | 2-thienyl | RₐCOO- |
| iPrOCO- | 3-thienyl | RₐCOO- |
| iPrOCO- | 2-pyridyl | RₐCOO- |
| iPrOCO- | 3-pyridyl | RₐCOO- |
| iPrOCO- | 4-pyridyl | RₐCOO- |
| iPrOCO- | Isobutenyl | RₐCOO- |
| iPrOCO- | Isopropyl | RₐCOO- |
| iPrOCO- | Cyclopropyl | RₐCOO- |
| iPrOCO- | Cyclobutyl | RₐCOO- |
| iPrOCO- | Cyclopentyl | RₐCOO- |
| iPrOCO- | Phenyl | RₐCOO- |
| nPrOCO- | 2-furyl | RₐCOO- |
| nPrOCO- | 3-furyl | RₐCOO- |
| nPrOCO- | 2-thienyl | RₐCOO- |
| nPrOCO- | 3-thienyl | RₐCOO- |
| nPrOCO- | 2-pyridyl | RₐCOO- |
| nPrOCO- | 3-pyridyl | RₐCOO- |
| nPrOCO- | 4-pyridyl | RₐCOO- |
| nPrOCO- | Isobutenyl | RₐCOO- |
| nPrOCO- | Isopropyl | RₐCOO- |
| nPrOCO- | Cyclopropyl | RₐCOO- |
| nPrOCO- | Cyclobutyl | RₐCOO- |
| nPrOCO- | Cyclopentyl | RₐCOO- |
| nPrOCO- | Phenyl | RₐCOO- |
| nPrCO- | 2-furyl | RₐCOO- |
| nPrCO- | 3-furyl | RₐCOO- |
| nPrCO- | 2-thienyl | RₐCOO- |
| nPrCO- | 3-thienyl | RₐCOO- |
| nPrCO- | 2-pyridyl | RₐCOO- |
| nPrCO- | 3-pyridyl | RₐCOO- |
| nPrCO- | 4-pyridyl | RₐCOO- |
| nPrCO- | Isobutenyl | RₐCOO- |
| nPrCO- | Isopropyl | RₐCOO- |
| nPrCO- | Cyclopropyl | RₐCOO- |
| nPrCO- | Cyclobutyl | RₐCOO- |
| nPrCO- | Cyclopentyl | RₐCOO- |
| NPrCO- | Phenyl | RₐCOO- |
| tBuOCO- | Cyclopentyl | EtCOO- |
| Benzoyl | Cyclopentyl | EtCOO- |
| 2-FuCO- | 3-furyl | EtCOO- |
| 2-FuCO- | 3-thienyl | EtCOO- |
| 2-FuCO- | 2-pyridyl | EtCOO- |
| 2-FuCO- | 3-pyridyl | EtCOO- |
| 2-FuCO- | 4-pyridyl | EtCOO- |
| 2-FuCO- | Isobutenyl | EtCOO- |
| 2-FuCO- | Isopropyl | EtCOO- |
| 2-FuCO- | Cyclopropyl | EtCOO- |
| 2-FuCO- | Cyclobutyl | EtCOO- |
| 2-FuCO- | Cyclopentyl | EtCOO- |
| 2-FuCO- | Phenyl | EtCOO- |
| 2-ThCO- | 3-furyl | EtCOO- |
| 2-ThCO- | 3-thienyl | EtCOO- |
| 2-ThCO- | 2-pyridyl | EtCOO- |
| 2-ThCO- | 3-pyridyl | EtCOO- |
| 2-ThCO- | 4-pyridyl | EtCOO- |
| 2-ThCO- | Isobutenyl | EtCOO- |
| 2-ThCO- | Isopropyl | EtCOO- |
| 2-ThCO- | Cyclopropyl | EtCOO- |
| 2-ThCO- | Cyclobutyl | EtCOO- |
| 2-ThCO- | Cyclopentyl | EtCOO- |
| 2-ThCO- | Phenyl | EtCOO- |
| 2-PyCO- | 2-furyl | EtCOO- |
| 2-PyCO- | 3-furyl | EtCOO- |
| 2-PyCO- | 2-thienyl | EtCOO- |
| 2-PyCO- | 3-thienyl | EtCOO- |
| 2-PyCO- | 2-pyridyl | EtCOO- |
| 2-PyCO- | 3-pyridyl | EtCOO- |
| 2-PyCO- | 4-pyridyl | EtCOO- |
| 2-PyCO- | Isobutenyl | EtCOO- |
| 2-PyCO- | Isopropyl | EtCOO- |
| 2-PyCO- | Cyclopropyl | EtCOO- |
| 2-PyCO- | Cyclobutyl | EtCOO- |
| 2-PyCO- | Cyclopentyl | EtCOO- |
| 2-PyCO- | Phenyl | EtCOO- |
| 3-PyCO- | 2-furyl | EtCOO- |
| 3-PyCO- | 3-furyl | EtCOO- |
| 3-PyCO- | 3-thienyl | EtCOO- |
| 3-PyCO- | 2-pyridyl | EtCOO- |
| 3-PyCO- | 3-pyridyl | EtCOO- |
| 3-PyCO- | 4-pyridyl | EtCOO- |
| 3-PyCO- | Isobutenyl | EtCOO- |
| 3-PyCO- | Isopropyl | EtCOO- |
| 3-PyCO- | Cyclopropyl | EtCOO- |
| 3-PyCO- | Cyclobutyl | EtCOO- |
| 3-PyCO- | Cyclopentyl | EtCOO- |
| 3-PyCO- | Phenyl | EtCOO- |
| 4-PyCO- | 2-furyl | EtCOO- |
| 4-PyCO- | 3-furyl | EtCOO- |
| 4-PyCO- | 3-thienyl | EtCOO- |
| 4-PyCO- | 2-pyridyl | EtCOO- |
| 4-PyCO- | 3-pyridyl | EtCOO- |
| 4-PyCO- | 4-pyridyl | EtCOO- |
| 4-PyCO- | Isobutenyl | EtCOO- |
| 4-PyCO- | Isopropyl | EtCOO- |
| 4-PyCO- | Cyclopropyl | EtCOO- |
| 4-PyCO- | Cyclobutyl | EtCOO- |
| 4-PyCO- | Cyclopentyl | EtCOO- |
| 4-PyCO- | Phenyl | EtCOO- |
| C₄H₇CO- | 3-furyl | EtCOO- |
| C₄H₇CO- | 3-thienyl | EtCOO- |
| C₄H₇CO- | 2-pyridyl | EtCOO- |
| C₄H₇CO- | 3-pyridyl | EtCOO- |
| C₄H₇CO- | 4-pyridyl | EtCOO- |
| C₄H₇CO- | Isobutenyl | EtCOO- |
| C₄H₇CO- | Isopropyl | EtCOO- |
| C₄H₇CO- | Cyclopropyl | EtCOO- |
| C₄H₇CO- | Cyclobutyl | EtCOO- |
| C₄H₇CO- | Cyclopentyl | EtCOO- |
| C₄H₇CO- | Phenyl | EtCOO- |
| EtOCO- | 3-furyl | EtCOO- |
| EtOCO- | 3-thienyl | EtCOO- |
| EtOCO- | 2-pyridyl | EtCOO- |
| EtOCO- | 3-pyridyl | EtCOO- |
| EtOCO- | 4-pyridyl | EtCOO- |
| EtOCO- | Isobutenyl | EtCOO- |
| EtOCO- | Isopropyl | EtCOO- |
| EtOCO- | Cyclopropyl | EtCOO- |
| EtOCO- | Cyclobutyl | EtCOO- |
| EtOCO- | Cyclopentyl | EtCOO- |
| EtOCO- | Phenyl | EtCOO- |
| ibueCO- | 2-furyl | EtCOO- |
| ibueCO- | 3-furyl | EtCOO- |
| ibueCO- | 3-thienyl | EtCOO- |
| ibueCO- | 2-pyridyl | EtCOO- |
| ibueCO- | 3-pyridyl | EtCOO- |
| ibueCO- | 4-pyridyl | EtCOO- |
| ibueCO- | Isobutenyl | EtCOO- |
| ibueCO- | Isopropyl | EtCOO- |
| ibueCO- | Cyclopropyl | EtCOO- |
| ibueCO- | Cyclobutyl | EtCOO- |
| ibueCO- | Cyclopentyl | EtCOO- |
| ibueCO- | Phenyl | EtCOO- |
| iBuCO- | 2-furyl | EtCOO- |
| iBuCO- | 3-furyl | EtCOO- |
| iBuCO- | 2-thienyl | EtCOO- |
| iBuCO- | 3-thienyl | EtCOO- |
| iBuCO- | 2-pyridyl | EtCOO- |
| iBuCO- | 3-pyridyl | EtCOO- |
| iBuCO- | 4-pyridyl | EtCOO- |
| iBuCO- | Isobutenyl | EtCOO- |
| iBuCO- | Isopropyl | EtCOO- |
| iBuCO- | Cyclopropyl | EtCOO- |
| iBuCO- | Cyclobutyl | EtCOO- |
| iBuCO- | Cyclopentyl | EtCOO- |
| iBuCO- | Phenyl | EtCOO- |
| iBuOCO- | 2-furyl | EtCOO- |
| iBuOCO- | 2-pyridyl | EtCOO- |
| iBuOCO- | 3-pyridyl | EtCOO- |
| iBuOCO- | 4-pyridyl | EtCOO- |
| iBuOCO- | Isopropyl | EtCOO- |
| iBuOCO- | Cyclobutyl | EtCOO- |
| iBuOCO- | Cyclopentyl | EtCOO- |
| iBuOCO- | Phenyl | EtCOO- |
| iPrOCO- | 3-furyl | EtCOO- |
| iPrOCO- | 3-thienyl | EtCOO- |
| iPrOCO- | 2-pyridyl | EtCOO- |
| iPrOCO- | 3-pyridyl | EtCOO- |
| iPrOCO- | 4-pyridyl | EtCOO- |
| iPrOCO- | Isobutenyl | EtCOO- |
| iPrOCO- | Isopropyl | EtCOO- |
| iPrOCO- | Cyclopropyl | EtCOO- |
| iPrOCO- | Cyclobutyl | EtCOO- |
| iPrOCO- | Cyclopentyl | EtCOO- |
| iPrOCO- | Phenyl | EtCOO- |
| nPrOCO- | 2-furyl | EtCOO- |
| nPrOCO- | 3-furyl | EtCOO- |
| nPrOCO- | 2-thienyl | EtCOO- |
| nPrOCO- | 3-thienyl | EtCOO- |
| nPrOCO- | 2-pyridyl | EtCOO- |
| nPrOCO- | 3-pyridyl | EtCOO- |
| nPrOCO- | 4-pyridyl | EtCOO- |
| nPrOCO- | Isobutenyl | EtCOO- |
| nPrOCO- | Isopropyl | EtCOO- |
| nPrOCO- | Cyclopropyl | EtCOO- |
| nPrOCO- | Cyclobutyl | EtCOO- |
| nPrOCO- | Cyclopentyl | EtCOO- |
| nPrOCO- | Phenyl | EtCOO- |
| nPrCO- | 3-furyl | EtCOO- |
| nPrCO- | 3-thienyl | EtCOO- |
| nPrCO- | 2-pyridyl | EtCOO- |
| nPrCO- | 3-pyridyl | EtCOO- |
| nPrCO- | 4-pyridyl | EtCOO- |
| nPrCO- | Isobutenyl | EtCOO- |
| nPrCO- | Isopropyl | EtCOO- |
| nPrCO- | Cyclopropyl | EtCOO- |
| nPrCO- | Cyclobutyl | EtCOO- |
| nPrCO- | Cyclopentyl | EtCOO- |
| nPrOCO- | Phenyl | EtCOO- |

### Example 4

Following the processes described in Example 1 and elsewhere herein, the following specific taxanes having structural formula 15 may be prepared, wherein R₇ is hydroxy and R₁₀ in each of the series (that is, each of series "A" through "K") is as previously defined, including wherein R₁₀ is R₁₀ₐCOO- and R₁₀ₐ is (i) substituted or unsubstituted, preferably unsubstituted, C₂ to C₈ alkyl (straight, branched or cyclic), such as ethyl, propyl, butyl, pentyl, or hexyl; (ii) substituted or unsubstituted, preferably unsubstituted, C₂ to C₈ alkenyl (straight, branched or cyclic), such as ethenyl, propenyl, butenyl, pentenyl or hexenyl; (iii) substituted or unsubstituted, preferably unsubstituted, C₂ to C₈ alkynyl (straight or branched) such as ethynyl, propynyl, butynyl, pentynyl, or hexynyl; (iv) substituted or unsubstituted, preferably unsubstituted, phenyl; or (v) substituted or unsubstituted, preferably unsubstituted, heteroaromatic such as furyl, thienyl, or pyridyl.

In the "A" series of compounds, X₁₀ is as otherwise as defined herein. Preferably, heterocyclo is substituted or unsubstituted furyl, thienyl, or pyridyl, X₁₀ is substituted or unsubstituted furyl, thienyl, pyridyl, phenyl, or lower alkyl (e.g., tert-butyl), and R₇ and R₁₀ each have the beta stereochemical configuration.

In the "B" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇ and R₁₀ each have the beta stereochemical configuration.

In the "C" series of compounds, X₁₀ and R₉ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₉ₐ is preferably substituted or unsubstituted furyl, thienyl, pyridyl, phenyl, or lower alkyl, and R₇, R₉ and R₁₀ each have the beta stereochemical configuration.

In the "D" and "E" series of compounds, X₁₀ is as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), and R₇, R₉ (series D only) and R₁₀ each have the beta stereochemical configuration.

In the "F" series of compounds, X₁₀, R₂ₐ and R₉ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

In the "G" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

In the "H" series of compounds, X₁₀ is as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇ and R₁₀ each have the beta stereochemical configuration.

In the "I" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇ and R₁₀ each have the beta stereochemical configuration.

In the "J" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

In the "K" series of compounds, X₁₀, R₂ₐ and R₉ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

Any substituents of each X₃, X₅, R₂, R₉, R₁₀ may be hydrocarbyl or any of the heteroatom containing substituents selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

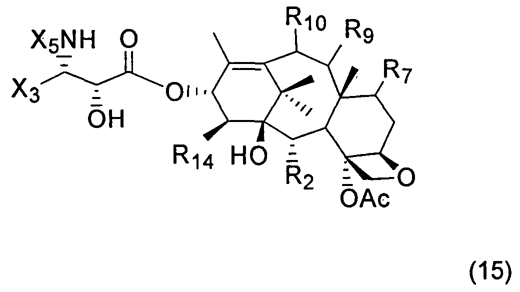

| **Series** | **X**_{**5**} | **X**_{**3**} | **R**_{**10**} | **R**_{**2**} | **R**_{**9**} | **R**_{**14**} |
|---|---|---|---|---|---|---|
| A1 | -COOX₁₀ | Heterocyclo | R₁₀ₐCOO- | C₆H₅COO- | O | H |
| A2 | -COX₁₀ | Heterocyclo | R₁₀ₐCOO- | C₆H₅COO- | O | H |
| A3 | -CONHX₁₀ | Heterocyclo | R₁₀ₐCOO- | C₆H₅COO- | O | H |
| A4 | -COOX₁₀ | Optionally substituted C₂ to C₈ alkyl | R₁₀ₐCOO- | C₆H₅COO- | O | H |
| A5 | -COX₁₀ | Optionally substituted C₂ to C₈ alkyl | R₁₀ₐCOO- | C₆H₅COO- | O | H |
| A6 | -CONHX₁₀ | Optionally substituted C₂ to C₈ alkyl | R₁₀ₐCOO- | C₆H₅COO- | O | H |
| A7 | -COOX₁₀ | Optionally substituted C₂ to C₈ alkenyl | R₁₀ₐCOO- | C₆H₅COO- | O | H |
| A8 | -COX₁₀ | Optionally substituted C₂ to C₈ alkenyl | R₁₀ₐCOO- | C₆H₅COO- | O | H |
| A9 | -CONHX₁₀ | Optionally substituted C₂ to C₈ alkenyl | R₁₀ₐCOO- | C₆H₅COO- | O | H |
| A10 | -COOX₁₀ | Optionally substituted C₂ to C₈ alkynyl | R₁₀ₐCOO- | C₆H₅COO- | O | H |
| A11 | -COX₁₀ | Optionally substituted C₂ to C₈ alkynyl | R₁₀ₐCOO- | C₆H₅COO- | O | H |
| A12 | -CONHX₁₀ | Optionally substituted C₂ to C₈ alkynyl | R₁₀ₐCOO- | C₆H₅COO- | O | H |
| B1 | -COOX₁₀ | Heterocyclo | R₁₀ₐCOO- | R₂ₐCOO- | O | H |
| B2 | -COX₁₀ | Heterocyclo | R₁₀ₐCOO- | R₂ₐCOO- | O | H |
| B3 | -CONHX₁₀ | Heterocyclo | R₁₀ₐCOO- | R₂ₐCOO- | O | H |
| B4 | -COOX₁₀ | Optionally substituted C₂ to C₈ alkyl | R₁₀ₐCOO- | R₂ₐCOO- | O | H |
| B5 | -COX₁₀ | Optionally substituted C₂ to C₈ alkyl | R₁₀ₐCOO- | R₂ₐCOO- | O | H |
| B6 | -CONHX₁₀ | Optionally substituted C₂ to C₈ alkyl | R₁₀ₐCOO- | R₂ₐCOO- | O | H |
| B7 | -COOX₁₀ | Optionally substituted C₂ to C₈ alkenyl | R₁₀ₐCOO- | R₂ₐCOO- | O | H |
| B8 | -COX₁₀ | Optionally substituted C₂ to C₈ alkenyl | R₁₀ₐCOO- | R₂ₐCOO- | O | H |
| B9 | -CONHX₁₀ | Optionally substituted C₂ to C₈ alkenyl | R₁₀ₐCOO- | R₂ₐCOO- | O | H |
| B10 | -COOX₁₀ | Optionally substituted C₂ to C₈ alkynyl | R₁₀ₐCOO- | R₂ₐCOO- | O | H |
| B11 | -COX₁₀ | Optionally substituted C₂ to C₈ alkynyl | R₁₀ₐCOO- | R₂ₐCOO- | O | H |
| B12 | -CONHX₁₀ | Optionally substituted C₂ to C₈ alkynyl | R₁₀ₐCOO- | R₂ₐCOO- | O | H |
| C1 | -COOX₁₀ | Heterocyclo | R₁₀ₐCOO- | C₆H₅COO- | R₉ₐCOO- | H |
| C2 | -COX₁₀ | Heterocyclo | R₁₀ₐCOO- | C₆H₅COO- | R₉ₐCOO- | H |
| C3 | -CONHX₁₀ | Heterocyclo | R₁₀ₐCOO- | C₆H₅COO- | R₉ₐCOO- | H |
| C4 | -COOX₁₀ | Optionally substituted C₂ to C₈ alkyl | R₁₀ₐCOO- | C₆H₅COO- | R₉ₐCOO- | H |
| C5 | -COX₁₀ | Optionally substituted C₂ to C₈ alkyl | R₁₀ₐCOO- | C₆H₅COO- | R₉ₐCOO- | H |
| C6 | -CONHX₁₀ | Optionally substituted C₂ to C₈ alkyl | R₁₀ₐCOO- | C₆H₅COO- | R₉ₐCOO- | H |
| C7 | -COOX₁₀ | Optionally substituted C₂ to C₈ alkenyl | R₁₀ₐCOO- | C₆H₅COO- | R₉ₐCOO- | H |
| C8 | -COX₁₀ | Optionally substituted C₂ to C₈ alkenyl | R₁₀ₐCOO- | C₆H₅COO- | R₉ₐCOO- | H |
| C9 | -CONHX₁₀ | Optionally substituted C₂ to C₈ alkenyl | R₁₀ₐCOO- | C₆H₅COO- | R₉ₐCOO- | H |
| C10 | -COOX₁₀ | Optionally substituted C₂ to C₈ alkynyl | R₁₀ₐCOO- | C₆H₅COO- | R₉ₐCOO- | H |
| C11 | -COX₁₀ | Optionally substituted C₂ to C₈ alkynyl | R₁₀ₐCOO- | C₆H₅COO- | R₉ₐCOO- | H |
| C12 | -CONHX₁₀ | Optionally substituted C₂ to C₈ alkynyl | R₁₀ₐCOO- | C₆H₅COO- | R₉ₐCOO- | H |
| D1 | -COOX₁₀ | Heterocyclo | R₁₀ₐCOO- | C₆H₅COO- | OH | H |
| D2 | -COX₁₀ | Heterocyclo | R₁₀ₐCOO- | C₆H₅COO- | OH | H |
| D3 | -CONHX₁₀ | Heterocyclo | R₁₀ₐCOO- | C₆H₅COO- | OH | H |
| D4 | -COOX₁₀ | Optionally substituted C₂ to C₈ alkyl | R₁₀ₐCOO- | C₆H₅COO- | OH | H |
| D5 | -COX₁₀ | Optionally substituted C₂ to C₈ alkyl | R₁₀ₐCOO- | C₆H₅COO- | OH | H |
| D6 | -CONHX₁₀ | Optionally substituted C₂ to C₈ alkyl | R₁₀ₐCOO- | C₆H₅COO- | OH | H |
| D7 | -COOX₁₀ | Optionally substituted C₂ to C₈ alkenyl | R₁₀ₐCOO- | C₆H₅COO- | OH | H |
| D8 | -COX₁₀ | Optionally substituted C₂ to C₈ alkenyl | R₁₀ₐCOO- | C₆H₅COO- | OH | H |
| D9 | -CONHX₁₀ | Optionally substituted C₂ to C₈ alkenyl | R₁₀ₐCOO- | C₆H₅COO- | OH | H |
| D10 | -COOX₁₀ | Optionally substituted C₂ to C₈ alkynyl | R₁₀ₐCOO- | C₆H₅COO- | OH | H |
| D11 | -COX₁₀ | Optionally substituted C₂ to C₈ alkynyl | R₁₀ₐCOO- | C₆H₅COO- | OH | H |
| D12 | -CONHX₁₀ | Optionally substituted C₂ to C₈ alkynyl | R₁₀ₐCOO- | C₆H₅COO- | OH | H |
| E1 | -COOX₁₀ | Heterocyclo | R₁₀ₐCOO- | C₆H₅COO- | O | OH |
| E2 | -COX₁₀ | Heterocyclo | R₁₀ₐCOO- | C₆H₅COO- | O | OH |
| E3 | -CONHX₁₀ | Heterocyclo | R₁₀ₐCOO- | C₆H₅COO- | O | OH |
| E4 | -COOX₁₀ | Optionally substituted C₂ to C₈ alkyl | R₁₀ₐCOO- | C₆H₅COO- | O | OH |
| E5 | -COX₁₀ | Optionally substituted C₂ to C₈ alkyl | R₁₀ₐCOO- | C₆H₅COO- | O | OH |
| E6 | -CONHX₁₀ | Optionally substituted C₂ to C₈ alkyl | R₁₀ₐCOO- | C₆H₅COO- | O | OH |
| E7 | -COOX₁₀ | Optionally substituted C₂ to C₈ alkenyl | R₁₀ₐCOO- | C₆H₅COO- | O | OH |
| E8 | -COX₁₀ | Optionally substituted C₂ to C₈ alkenyl | R₁₀ₐCOO- | C₆H₅COO- | O | OH |
| E9 | -CONHX₁₀ | Optionally substituted C₂ to C₈ alkenyl | R₁₀ₐCOO- | C₆H₅COO- | O | OH |
| E10 | -COOX₁₀ | Optionally substituted C₂ to C₈ alkynyl | R₁₀ₐCOO- | C₆H₅COO- | O | OH |
| E11 | -COX₁₀ | Optionally substituted C₂ to C₈ alkynyl | R₁₀ₐCOO- | C₆H₅COO- | O | OH |
| E12 | -CONHX₁₀ | Optionally substituted C₂ to C₈ alkynyl | R₁₀ₐCOO- | C₆H₅COO- | O | OH |
| F1 | -COOX₁₀ | Heterocyclo | R₁₀ₐCOO- | R₂ₐCOO- | R₉ₐCOO- | H |
| F2 | -COX₁₀ | Heterocyclo | R₁₀ₐCOO- | R₂ₐCOO- | R₉ₐCOO- | H |
| F3 | -CONHX₁₀ | Heterocyclo | R₁₀ₐCOO- | R₂ₐCOO- | R₉ₐCOO- | H |
| F4 | -COOX₁₀ | Optionally substituted C₂ to C₈ alkyl | R₁₀ₐCOO- | R₂ₐCOO- | R₉ₐCOO- | H |
| F5 | -COX₁₀ | Optionally substituted C₂ to C₈ alkyl | R₁₀ₐCOO- | R₂ₐCOO- | R₉ₐCOO- | H |
| F6 | -CONHX₁₀ | Optionally substituted C₂ to C₈ alkyl | R₁₀ₐCOO | R₂ₐCOO- | R₉ₐCOO- | H |
| F7 | -COOX₁₀ | Optionally substituted C₂ to C₈ alkenyl | R₁₀ₐCOO- | R₂ₐCOO- | R₉ₐCOO- | H |
| F8 | -COX₁₀ | Optionally substituted C₂ to C₈ alkenyl | R₁₀ₐCOO- | R₂ₐCOO- | R₉ₐCOO- | H |
| F9 | -CONHX₁₀ | Optionally substituted C₂ to C₈ alkenyl | R₁₀ₐCOO- | R₂ₐCOO- | R₉ₐCOO- | H |
| F10 | -COOX₁₀ | Optionally substituted C₂ to C₈ alkynyl | R₁₀ₐCOO- | R₂ₐCOO- | R₉ₐCOO- | H |
| F11 | -COX₁₀ | Optionally substituted C₂ to C₈ alkynyl | R₁₀ₐCOO- | R₂ₐCOO- | R₉ₐCOO- | H |
| F12 | -CONHX₁₀ | Optionally substituted C₂ to C₈ alkynyl | R₁₀ₐCOO- | R₂ₐCOO- | R₉ₐCOO- | H |
| G1 | -COOX₁₀ | Heterocyclo | R₁₀ₐCOO- | R₂ₐCOO- | OH | H |
| G2 | -COX₁₀ | Heterocyclo | R₁₀ₐCOO- | R₂ₐCOO- | OH | H |
| G3 | -CONHX₁₀ | Heterocyclo | R₁₀ₐCOO- | R₂ₐCOO- | OH | H |
| G4 | -COOX₁₀ | Optionally substituted C₂ to C₈ alkyl | R₁₀ₐCOO- | R₂ₐCOO- | OH | H |
| G5 | -COX₁₀ | Optionally substituted C₂ to C₈ alkyl | R₁₀ₐCOO- | R₂ₐCOO- | OH | H |
| G6 | -CONHX₁₀ | Optionally substituted C₂ to C₈ alkyl | R₁₀ₐCOO- | R₂ₐCOO- | OH | H |
| G7 | -COOX₁₀ | Optionally substituted C₂ to C₈ alkenyl | R₁₀ₐCOO- | R₂ₐCOO- | OH | H |
| G8 | -COX₁₀ | Optionally substituted C₂ to C₈ alkenyl | R₁₀ₐCOO- | R₂ₐCOO- | OH | H |
| G9 | -CONHX₁₀ | Optionally substituted C₂ to C₈ alkenyl | R₁₀ₐCOO- | R₂ₐCOO- | OH | H |
| G10 | -COOX₁₀ | Optionally substituted C₂ to C₈ alkynyl | R₁₀ₐCOO- | R₂ₐCOO- | OH | H |
| G11 | -COX₁₀ | Optionally substituted C₂ to C₈ alkynyl | R₁₀ₐCOO- | R₂ₐCOO- | OH | H |
| G12 | -CONHX₁₀ | Optionally substituted C₂ to C₈ alkynyl | R₁₀ₐCOO- | R₂ₐCOO- | OH | H |
| H1 | -COOX₁₀ | Heterocyclo | R₁₀ₐCOO- | C₆H₅COO- | OH | OH |
| H2 | -COX₁₀ | Heterocyclo | R₁₀ₐCOO- | C₆H₅COO- | OH | OH |
| H3 | -CONHX₁₀ | Heterocyclo | R₁₀ₐCOO- | C₆H₅COO- | OH | OH |
| H4 | -COOX₁₀ | Optionally substituted C₂ to C₈ alkyl | R₁₀ₐCOO- | C₆H₅COO- | OH | OH |
| H5 | -COX₁₀ | Optionally substituted C₂ to C₈ alkyl | R₁₀ₐCOO- | C₆H₅COO- | OH | OH |
| H6 | -CONHX₁₀ | Optionally substituted C₂ to C₈ alkyl | R₁₀ₐCOO- | C₆H₅COO- | OH | OH |
| H7 | -COOX₁₀ | Optionally substituted C₂ to C₈ alkenyl | R₁₀ₐCOO- | C₆H₅COO- | OH | OH |
| H8 | -COX₁₀ | Optionally substituted C₂ to C₈ alkenyl | R₁₀ₐCOO- | C₆H₅COO- | OH | OH |
| H9 | -CONHX₁₀ | Optionally substituted C₂ to C₈ alkenyl | R₁₀ₐCOO- | C₆H₅COO- | OH | OH |
| H10 | -COOX₁₀ | Optionally substituted C₂ to C₈ alkynyl | R₁₀ₐCOO- | C₆H₅COO- | OH | OH |
| H11 | -COX₁₀ | Optionally substituted C₂ to C₈ alkynyl | R₁₀ₐCOO- | C₆H₅COO- | OH | OH |
| H12 | -CONHX₁₀ | Optionally substituted C₂ to C₈ alkynyl | R₁₀ₐCOO- | C₆H₅COO- | OH | OH |
| I1 | -COOX₁₀ | Heterocyclo | R₁₀ₐCOO- | R₂ₐCOO- | O | OH |
| I2 | -COX₁₀ | Heterocyclo | R₁₀ₐCOO- | R₂ₐCOO- | O | OH |
| I3 | -CONHX₁₀ | Heterocyclo | R₁₀ₐCOO- | R₂ₐCOO- | O | OH |
| I4 | -COOX₁₀ | Optionally substituted C₂ to C₈ alkyl | R₁₀ₐCOO- | R₂ₐCOO- | O | OH |
| I5 | -COX₁₀ | Optionally substituted C₂ to C₈ alkyl | R₁₀ₐCOO- | R₂ₐCOO- | O | OH |
| I6 | -CONHX₁₀ | Optionally substituted C₂ to C₈ alkyl | R₁₀ₐCOO- | R₂ₐCOO- | O | OH |
| I7 | -COOX₁₀ | Optionally substituted C₂ to C₈ alkenyl | R₁₀ₐCOO- | R₂ₐCOO- | O | OH |
| I8 | -COX₁₀ | Optionally substituted C₂ to C₈ alkenyl | R₁₀ₐCOO- | R₂ₐCOO- | O | OH |
| I9 | -CONHX₁₀ | Optionally substituted C₂ to C₈ alkenyl | R₁₀ₐCOO- | R₂ₐCOO- | O | OH |
| I10 | -COOX₁₀ | Optionally substituted C₂ to C₈ alkynyl | R₁₀ₐCOO- | R₂ₐCOO- | O | OH |
| I11 | -COX₁₀ | Optionally substituted C₂ to C₈ alkynyl | R₁₀ₐCOO- | R₂ₐCOO- | O | OH |
| I12 | -CONHX₁₀ | Optionally substituted C₂ to C₈ alkynyl | R₁₀ₐCOO- | R₂ₐCOO- | O | OH |
| J1 | -COOX₁₀ | Heterocyclo | R₁₀ₐCOO- | R₂ₐCOO- | OH | OH |
| J2 | -COX₁₀ | Heterocyclo | R₁₀ₐCOO- | R₂ₐCOO- | OH | OH |
| J3 | -CONHX₁₀ | Heterocyclo | R₁₀ₐCOO- | R₂ₐCOO- | OH | OH |
| J4 | -COOX₁₀ | Optionally substituted C₂ to C₈ alkyl | R₁₀ₐCOO- | R₂ₐCOO- | OH | OH |
| J5 | -COX₁₀ | Optionally substituted C₂ to C₈ alkyl | R₁₀ₐCOO- | R₂ₐCOO- | OH | OH |
| J6 | -CONHX₁₀ | Optionally substituted C₂ to C₈ alkyl | R₁₀ₐCOO- | R₂ₐCOO- | OH | OH |
| J7 | -COOX₁₀ | Optionally substituted C₂ to C₈ alkenyl | R₁₀ₐCOO- | R₂ₐCOO- | OH | OH |
| J8 | -COX₁₀ | Optionally substituted C₂ to C₈ alkenyl | R₁₀ₐCOO- | R₂ₐCOO- | OH | OH |
| J9 | -CONHX₁₀ | Optionally substituted C₂ to C₈ alkenyl | R₁₀ₐCOO- | R₂ₐCOO- | OH | OH |
| J10 | -COOX₁₀ | Optionally substituted C₂ to C₈ alkynyl | R₁₀ₐCOO- | R₂ₐCOO- | OH | OH |
| J11 | -COX₁₀ | Optionally substituted C₂ to C₈ alkynyl | R₁₀ₐCOO- | R₂ₐCOO- | OH | OH |
| J12 | -CONHX₁₀ | Optionally substituted C₂ to C₈ alkynyl | R₁₀ₐCOO- | R₂ₐCOO- | OH | OH |
| K1 | -COOX₁₀ | Heterocyclo | R₁₀ₐCOO- | R₂ₐCOO- | R₉ₐCOO- | OH |
| K2 | -COX₁₀ | Heterocyclo | R₁₀ₐCOO- | R₂ₐCOO- | R₉ₐCOO- | OH |
| K3 | -CONHX₁₀ | Heterocyclo | R₁₀ₐCOO- | R₂ₐCOO- | R₉ₐCOO- | OH |
| K4 | -COOX₁₀ | Optionally substituted C₂ to C₈ alkyl | R₁₀ₐCOO- | R₂ₐCOO- | R₉ₐCOO- | OH |
| K5 | -COX₁₀ | Optionally substituted C₂ to C₈ alkyl | R₁₀ₐCOO- | R₂ₐCOO- | R₉ₐCOO- | OH |
| K6 | -CONHX₁₀ | Optionally substituted C₂ to C₈ alkyl | R₁₀ₐCOO- | R₂ₐCOO- | R₉ₐCOO- | OH |
| K7 | -COOX₁₀ | Optionally substituted C₂ to C₈ alkenyl | R₁₀ₐCOO- | R₂ₐCOO- | R₉ₐCOO- | OH |
| K8 | -COX₁₀ | Optionally substituted C₂ to C₈ alkenyl | R₁₀ₐCOO- | R₂ₐCOO- | R₉ₐCOO- | OH |
| K9 | -CONHX₁₀ | Optionally substituted C₂ to C₈ alkenyl | R₁₀ₐCOO- | R₂ₐCOO- | R₉ₐCOO- | OH |
| K10 | -COOX₁₀ | Optionally substituted C₂ to C₈ alkynyl | R₁₀ₐCOO- | R₂ₐCOO- | R₉ₐCOO- | OH |
| K11 | -COX₁₀ | Optionally substituted C₂ to C₈ alkynyl | R₁₀ₐCOO- | R₂ₐCOO- | R₉ₐCOO- | OH |
| K12 | -CONHX₁₀ | Optionally substituted C₂ to C₈ alkynyl | R₁₀ₐCOO- | R₂ₐCOO- | R₉ₐCOO- | OH |

### Example 5

### In Vitro cytotoxicity measured by the cell colony formation assay

Four hundred cells (HCT116) were plated in 60 mm Petri dishes containing 2.7 mL of medium (modified McCoy's 5a medium containing 10% fetal bovine serum and 100 units/mL penicillin and 100 mg/mL streptomycin). The cells were incubated in a CO2 incubator at 37 °C for 5 h for attachment to the bottom of Petri dishes. The compounds identified in Example 2 were made up fresh in medium at ten times the final concentration, and then 0.3 mL of this stock solution was added to the 2.7 mL of medium in the dish. The cells were then incubated with drugs for 72 h at 37 °C. At the end of incubation the drug-containing media were decanted, the dishes were rinsed with 4 mL of Hank's Balance Salt Solution (HBSS), 5 mL of fresh medium was added, and the dishes were returned to the incubator for colony formation. The cell colonies were counted using a colony counter after incubation for 7 days. Cell survival was calculated and the values of ID50 (the drug concentration producing 50% inhibition of colony formation) were determined for each tested compound.

| **Compound** | **IN VITRO ID 50 (nm) HCT116** |
|---|---|
| taxol | 2.1 |
| docetaxel | 0.6 |
| 0499 | <1 |
| 0503 | <1 |
| 0517 | <10 |
| 0521 | <1 |
| 0536 | <1 |
| 0549 | <10 |
| 0550 | <10 |
| 0562 | <1 |
| 0578 | <1 |
| 0583 | <10 |
| 0596 | <10 |
| 0602 | <1 |
| 0611 | <10 |
| 0625 | <1 |
| 0634 | <10 |
| 0647 | 12.0 |
| 0659 | <1 |
| 0663 | <1 |
| 0670 | <1 |
| 0687 | <1 |
| 0691 | <1 |
| 0706 | <1 |
| 0719 | <10 |
| 0720 | <10 |
| 0732 | <10 |
| 0748 | <10 |
| 0838 | <1 |
| 0843 | <1 |
| 0854 | <1 |
| 0860 | <1 |
| 0879 | <1 |
| 0882 | <1 |
| 0890 | <1 |
| 0908 | <1 |
| 0919 | <1 |
| 0923 | <1 |
| 0937 | <10 |
| 0947 | <1 |
| 0951 | <1 |
| 0966 | <10 |
| 0978 | <1 |
| 0983 | <1 |
| 0999 | <1 |
| 1003 | <1 |
| 1011 | <1 |
| 1020 | <1 |
| 1031 | <10 |
| 1044 | <1 |
| 1060 | <1 |
| 1879 | <10 |
| 1883 | <10 |
| 1892 | <1 |
| 1900 | <1 |
| 1911 | <10 |
| 1923 | <1 |
| 1939 | <1 |
| 1948 | <10 |
| 1954 | <1 |
| 1964 | <10 |
| 1970 | <10 |
| 1988 | <10 |
| 2101 | <1 |
| 2111 | <1 |
| 2124 | <10 |
| 2132 | <1 |
| 2142 | <1 |
| 2159 | <1 |
| 2164 | <1 |
| 2173 | <1 |
| 2181 | <10 |
| 2199 | <10 |
| 2202 | <1 |
| 2212 | <10 |
| 2226 | <1 |
| 2238 | <1 |
| 2242 | <10 |
| 2255 | <1 |
| 2269 | <1 |
| 2273 | <1 |
| 2287 | <1 |
| 2291 | <1 |
| 2306 | <10 |
| 2319 | <10 |
| 2320 | <1 |
| 2332 | <1 |
| 2348 | <1 |
| 2353 | <10 |
| 2366 | <1 |
| 2379 | <1 |
| 2380 | <1 |
| 2392 | <1 |
| 2408 | <1 |
| 2413 | <10 |
| 2424 | <10 |
| 2439 | <10 |
| 2442 | <1 |
| 2455 | <10 |
| 2464 | <1 |
| 2472 | <1 |
| 2488 | <1 |
| 2499 | <1 |
| 2503 | <1 |
| 2511 | <1 |
| 2520 | <10 |
| 2781 | <1 |
| 2794 | <1 |
| 2802 | <1 |
| 2813 | <1 |
| 2826 | <1 |
| 2838 | <1 |
| 2844 | <10 |
| 2855 | <1 |
| 2869 | <10 |
| 3053 | <1 |
| 3071 | <1 |
| 3096 | <1 |
| 3102 | <1 |
| 3110 | <1 |
| 3129 | <10 |
| 3132 | <1 |
| 3148 | <1 |
| 3163 | <1 |
| 3204 | <1 |
| 3219 | <1 |
| 3222 | <1 |
| 3258 | <1 |
| 3265 | <10 |
| 3297 | <1 |
| 3314 | <1 |
| 3352 | <1 |
| 3361 | <1 |
| 3370 | <1 |
| 3408 | <1 |
| 3417 | <1 |
| 3425 | <1 |
| 3453 | <1 |
| 3482 | <1 |
| 3494 | <1 |
| 3513 | <1 |
| 3522 | <1 |
| 3535 | <1 |
| 3543 | <10 |
| 3588 | <10 |
| 3595 | <1 |
| 3603 | <10 |
| 3644 | <1 |
| 3656 | <1 |
| 3663 | <1 |
| 3677 | <1 |
| 3686 | <1 |
| 3693 | <1 |
| 3800 | <1 |
| 3818 | <1 |
| 3853 | <1 |
| 3866 | <1 |
| 3909 | <1 |
| 3938 | <10 |
| 3945 | <1 |
| 3957 | <10 |
| 3971 | <1 |
| 3982 | <1 |
| 3994 | <1 |
| 4051 | <1 |
| 4062 | <1 |
| 4112 | <10 |
| 4121 | <10 |
| 4190 | <10 |
| 4207 | <10 |
| 4329 | <1 |
| 4335 | <1 |
| 4344 | <1 |
| 4665 | <10 |
| 4704 | <10 |
| 4711 | <10 |
| 4720 | <10 |
| 4799 | <1 |
| 4808 | <10 |
| 4834 | <10 |
| 4888 | <1 |
| 4919 | <1 |
| 4944 | <1 |
| 5011 | <10 |
| 5040 | <1 |
| 5065 | <10 |
| 5144 | <10 |
| 5232 | <10 |
| 5495 | <1 |
| 6522 | <1 |

### Example 6

### Preparation of Solutions for Oral Administration

Solution 1: Antitumor compound 0499 was dissolved in ethanol to form a solution containing 106 mg of the compound per ml of solution. An equal volume of Cremophor® EL solution was added to the solution while stirring to form a solution containing 53 mg of compound 0499 per ml of solution. This solution was diluted using 9 parts by weight of saline to form a pharmaceutically acceptable solution for administration to a patient.

Solution 2: Antitumor compound 0550 was dissolved in ethanol to form a solution containing 140 mg of the compound per ml of solution. An equal volume of Cremophor® EL solution was added to the solution while stirring to form a solution containing 70 mg of compound 0550 per ml of solution. This solution was diluted using 9 parts by weight of saline to form a pharmaceutically acceptable solution for administration to a patient.

Solution 3: Antitumor compound 0611 was dissolved in ethanol to form a solution containing 150 mg of the compound per ml of solution. An equal volume of Cremophor® EL solution was added to the solution while stirring to form a solution containing 75 mg of compound 0611 per ml of solution. This solution was diluted using 9 parts by weight of saline to form a pharmaceutically acceptable solution for administration to a patient.

Solution 4: Antitumor compound 0748 was dissolved in ethanol to form a solution containing 266 mg of the compound per ml of solution. An equal volume of Cremophor® EL solution was added to the solution while stirring to form a solution containing 133 mg of compound 0748 per ml of solution. This solution was diluted using 9 parts by weight of saline to form a pharmaceutically acceptable solution for administration to a patient.

### Example 7

### Preparation of a Suspension for Oral Administration

An oral composition of an antitumor compound of the invention can be prepared by suspending 25 mg of the compound as a fine powder in one ml of carrier containing 1 % carboxymethylcellulose (CMC) in deionized water.

### Example 8

### Preparation of a Tablet for Oral Administration

An antitumor compound of the invention (100 mg) can be dissolved in methylene chloride (2 ml) and Cremophor® EL solution (100mg) can be added. The methylene chloride can be evaporated under vacuum to form a glass. Microcrystalline cellulose (600 mg) can be added to the glass and mixed to form a powder which can be processed to form a tablet.

### Example 9

### Preparation of Emulsions for Parenteral Administration

Emulsion 1: An antitumor compound of the invention can be dissolved in 100% ethanol to form a solution containing 40 mg of the compound per ml of the solution. The solution can be diluted with 19 parts by weight of Liposyn® II (20%) with stirring to form an emulsion containing 2 mg of the compound per ml for parenteral administration.

Emulsion 2: An antitumor compound of the invention can be dissolved in 100% ethanol to form a solution containing 40 mg of the compound per ml of the solution. The solution can be diluted with 19 parts by weight of Liposyn® III (2%) with stirring to form an emulsion containing 2 mg of the compound per ml for parenteral administration.

Emulsion 3: An antitumor compound of the invention can be dissolved in 100% ethanol to form a solution containing 40 mg of the compound per ml of the solution. The solution can be diluted with 9 parts by weight of Liposyn® III (2%) with stirring to form an emulsion containing 4 mg of the compound per ml for parenteral administration.

### Example 10

### Preparation of Solutions Containing the Compound for Parenteral Administration

Solution 1: An antitumor compound of the invention can be dissolved in 100% ethanol to form a solution containing 140 mg of the compound per ml. The solution can be diluted with an equal volume of Cremophor® EL solution with stirring and then diluted with 9 parts by weight of normal saline to form a solution containing 7 mg of the compound per ml of solution for parenteral administration.

Solution 2: An antitumor compound of the invention can be dissolved in 100% ethanol to form a solution containing 140 mg of the compound per ml of the solution. The solution can be diluted with an equal volume of Cremophor® EL solution with stirring and then diluted with 4 parts by weight of normal saline to form a solution containing 11.7 mg of the compound per ml of solution for parenteral administration.

Solution 3: An antitumor compound of the invention can be dissolved in 100% ethanol to form a solution containing 140 mg of the compound per ml of the solution. The solution can be diluted with an equal volume of Cremophor® EL solution with stirring and then diluted with 2.33 parts by weight of normal saline to form a solution containing 16.2 mg of the compound per ml of solution for parenteral administration.

### Example 11

### In Vivo Evaluation of Antitumor Compounds Against Human Lung Carcinoma Xenografts

Antitumor compounds were formulated for an i.v. dosing regime prepared in the following vehicle: 10% ethanol, 10% Cremophor and 80% isotonic saline. The compounds were formulated in the manner as described for Solution 2 of Example 10. Taxol (paclitaxel, Bristol Meyers Squibb), used as a control, was obtained as the marketed pharmaceutical drug.

The two lung tumor xenografts used in the study were the SK-MES carcinoma, which is very sensitive to Taxol, and the NCI-H1299 (H1299) carcinoma, which is more resistant to Taxol compared to the SK-MES neoplasm.

Female NCr-nude mice bearing either SK-MES or H1299 neoplasms (implanted subcutaneously in the flank with 1mm³ human lung carcinoma fragments) were pair-matched into four groups of six mice each on Day 1, with the mean tumor size for the groups ranging from 241-244 mg. Treatment groups consisted of: treatment with the vehicle (Group 1); treatment with Taxol (Group 2); and treatment with antitumor compound 0503 (Group 3).

Taxol and the antitumor compound 0503 were administered intravenously at the maximum tolerated dose (MTD) appropriate for each compound, with half the dose being given one hour apart on the qd x 1 schedule. The MTDs for antitumor compound 0503 was calculated from earlier single dose data for mice receiving i.v. administration of this compound. Taxol itself was given as a split i.v. dose of 36 mg/kg, with the two 18 mg/kg doses separated by one hour. Vehicle control animals were dosed twice intravenously with half the total volume being given separated by one hour. The study was terminated on Day 60.

The results are summarized in Table 1 and include mean days of survival (MDS), number of toxic deaths, number of survivors, number of complete or partial responses, and number with stable disease. The mean days of survival are the number of days after which a neoplasm reached a size of 1.5 g and the animal was euthanized. A complete response is obtained if the tumor is not palpable at the end of the study. A partial response is obtained if the tumor shrinks to a size smaller than its size on Day 1 of the study. "Stable disease" occurs when the treatment limits the growth of the neoplasm to a small size that does not reach 1.5 g in size by the termination of the study.

A tumor scoring system was designed to provide a more quantitative ranking of efficacy (therapeutic potential) among the antitumor agents evaluated against the different human solid tumor xenografts. Each mouse in a study was given a score of from 1 to 10 at the end of the study. The scores can be summarized as follows:

| Score | Description |
|---|---|
| <1 | Serious toxicity |
| 2-3 | Tumor reached the cut-off size but significant tumor growth |
| | delay was apparent |
| 4-6 | Tumor growth was significantly inhibited (Stable Disease) |
| 7-9 | Partial tumor shrinkage (Partial Response) |
| 10 | Complete Response (Perfect score) |

The individual score for each mouse was averaged into a mean score for each treatment group. This tumor scoring system provided a better comparison of the different antitumor compounds by quantifying the treatment results (complete response versus partial response).

**Table 1**

| Group | MDS (n) | Toxic Deaths | Survivors | Complete Response | Partial Response | Stable Disease | Mean Score |
|---|---|---|---|---|---|---|---|
| 1 | 12.2 ± 1.3 (6) | 0 | 0 | 0 | 0 | 0 | 1 ± 0 |
| 2 | 16 ± 2.0 (6) | 0 | 0 | 0 | 0 | 0 | 1.3 ± 0.3 |
| 3 | 46.0 ± 0(2) | 0 | 4 | 3 | 0 | 1 | 6.5 ± 1.6 |

An MDS value of 12.2 days was calculated for the vehicle-treated control group. Taxol (36 mg/kg; Group 2) only produced a modest 30% survival extension (MDS = 16.0 days) compared to vehicle controls, with no recorded tumor regressions. In contrast, antitumor compound 0503 was highly active. Compound 0503 (72.1 mg/kg; Group 3) produced four and three complete responses with significant survival extensions recorded in the remaining treated animals compared to the vehicle or Taxol control groups. Antitumor compound 0503 was well tolerated.

In the H1299 experiment, mice were pair-matched into groups of six animals each on Day 1, with the mean tumor size for the groups ranging from 229-233 mg. The treatment protocol for the H1299 test was identical to the SK-MES test protocol. An MDS value of 24.5 days was calculated for the vehicle-treated control group. Taxol (36 mg/kg; Group 2) was not active in the H1299 model, causing only a 10% survival extension (MDS = 27.0 days) in five animals compared to vehicle-treated animals (not statistically significant). One partial response was observed following Taxol treatment.

**Table 2**

| Group | MDS (n) | Toxic Deaths | Survivors | Complete Response | Partial Response | Stable Disease | Mean Score |
|---|---|---|---|---|---|---|---|
| 1 | 24.5 ± 3.3 (6) | 0 | 0 | 0 | 0 | 0 | 1.2 ± 0.2 |
| 2 | 27.0 ± 4.6 (5) | 0 | 1 | 0 | 0 | 0 | 2.7 ± 1.3 |
| 3 | -- | 1 | 5 | 2 | 0 | 3 | 5.5 ± 1.6 |

Antitumor compound 0503 demonstrated significant efficacy against the Taxol-refractory H1299 neoplasm. Compound 0503 (72.1 mg/kg; Group 3) produced two complete responses in the H1299 test. Other mice treated with this compound experienced significantly increased survival times compared to vehicle-treated or Taxol-treated animals.

One mouse died in the H1299 test from toxicity in the groups treated by antitumor compound 0503. Since the doses and schedule were the same as in the SK-MES experiment where no toxic deaths were observed, the side effects experienced by the nude mice most likely were not due to systemic drug toxicity. One explanation based on the extreme reaction of the H1299 tumors to antitumor compound 0503, including necrosis and hemorrhaging of the carcinomas, is that the compound caused a destruction of the tumor architecture and stroma resulting in a release of toxic substance from the neoplasm to the host mice.

Antitumor compound 0503 was effective when administered as a single bolus against upstaged (250 mg) human lung carcinoma xenografts.

### Example 12

### In Vivo Evaluation of Antitumor Compounds Against the HCT116 Human Colon Carcinoma Xenograft

Antitumor compounds were formulated for an i.v. dosing regime and were prepared in the following vehicle: 5% ethanol, 5% Cremophor and 90% isotonic saline. The compounds were formulated in the manner as described for Solution 1 of Example 10. Taxol (paclitaxel, Bristol Meyers Squibb) was obtained as the marketed pharmaceutical drug. The dosing volume was 0.3 ml per 20 g mouse.

The HCT116 carcinoma is representative of human colon tumors and is generally insensitive to chemotherapy drugs, thus, such neoplasms are not treated clinically with Taxol.

Female NCr-nude mice bearing HCT116 neoplasms (implanted subcutaneously in the flank with 1 mm³ human carcinoma fragments) were pair-matched into groups of five mice each on Day 1, with the mean tumor size for groups ranging from 254-260 mg, except the mean tumor size for Group 3 (second Taxol control) was 104.8 mg on Day 1. Treatment groups consisted of: treatment with the vehicle (Group 1); i.v. treatment with Taxol in split doses on Day 1 (Group 2); intraperitoneal (i.p.) treatment with Taxol in non-split, qd x 5 doses over Days 1-5 (Group 3); and treatment with antitumor compounds 0503, 0536, 0663, 0670, 0687, 0706, 0908, 0947, 0951, 0983, 0999, 1003 and 1011 (Groups 4-16, respectively). Treatment groups are summarized in Table 3.

The antitumor compounds were given intravenously in a single maximum tolerated dose (MTD) appropriate for each compound. The Taxol controls were administered to two separate groups: Group 2 was given in a split dose, qd x 1 schedule on Day 1 and Group 3 was given in a non-split schedule over five days (qd x 5). The MTDs for the antitumor compounds were calculated from earlier single dose data for mice receiving i.v. administration of these compounds. All vehicle control animals in the split dosing regimes were dosed twice intravenously with half of the total volume being given separated by one hour. The study was terminated on Day 60.

The results are summarized in Table 3 and include mean days of survival (MDS), number of toxic deaths, number of survivors, number of complete or partial responses, and number with stable disease. The mean days of survival is the number of days after which a neoplasm reached a size of 2.0 g and the animal was euthanized. A complete response is obtained if the tumor is not palpable at the end of the study. A partial response is obtained if the tumor shrinks to a size smaller than its size on Day 1 of the study. "Stable disease" occurs when the treatment limits the growth of the neoplasm to a small size that does not reach 2.0 g in size by the termination of the study.

**Table 3**

| Group | Antitumor Compound | MDS (n) | Toxic Deaths | Survivors | Complete Response | Partial Response | Stable Disease | Mean Score |
|---|---|---|---|---|---|---|---|---|
| 1 | Vehicle control | 22.2 ± 2.0 (5) | 0 | 0 | 0 | 0 | 0 | 1 ± 0 |
| 2 | Taxol (36 mg/kg) | 27.5 ± 1.7 (4) | 0 | 1 | 0 | 0 | 1 | 2.2 ± 0.5 |
| 3 | Taxol (18 mg/kg) | 40.8 ± 3.6 (4) | 0 | 1 | 0 | 1 | 0 | 3.6 ± 1.4 |
| 4 | No. 0503 (72.6 mg/kg) | -- | 0 | 5 | 1 | 4 | 0 | 9.2 ± 0.2 |
| 5 | No. 0536 (49 mg/kg) | - | 0 | 5 | 1 | 4 | 0 | 9.2 ± 0.2 |
| 6 | No. 0663 (159 mg/kg) | -- | 1 | 4 | 2 | 2 | 0 | 7.6 ± 1.9 |
| 7 | No. 0670 (123 mg/kg) | 29.8 ± 0.7 (2) | 0 | 3 | 2 | 1 | 0 | 6.6 ± 1.9 |
| 8 | No. 0687 (159 mg/kg) | -- | 0 | 5 | 4 | 1 | 0 | 9.6 ± 0.4 |
| 9 | No. 0706 (108 mg/kg) | -- | 0 | 5 | 1 | 4 | 0 | 9.2 ± 0.2 |
| 10 | No. 0908 (159 mg/kg) | -- | 0 | 5 | 1 | 4 | 0 | 9.2 ± 0.2 |
| 11 | No. 0947 (76.9 mg/kg) | -- | 0 | 5 | 1 | 4 | 0 | 9.2 ± 0.2 |
| 12 | No. 0951 (72.6 mg/kg) | -- | 0 | 5 | 3 | 2 | 0 | 9.6 ± 0.2 |
| 13 | No. 0983 (72.6 mg/kg) | -- | 2 | 3 | 2 | 1 | 0 | 5.8 ± 2.4 |
| 14 | No. 0999 (49 mg/kg) | -- | 0 | 5 | 1 | 4 | 0 | 9.2 ± 0.2 |
| 15 | No. 1003 (49 mg/kg) | -- | 0 | 5 | 2 | 3 | 0 | 9.4 ± 0.2 |
| 16 | No. 1011 (108 mg/kg) | -- | 0 | 5 | 3 | 2 | 0 | 9.6 ± 0.2 |

All HCT116 carcinomas in the five mice in the vehicle control group (Group 1) grew progressively until the 2.0 g cut-off size was reached. An MDS value of 22.2 days was calculated for Group 1. Taxol given i.v. at a single dose of 36 mg/kg (Group 2) produced an MDS of 27.5 days in four mice and caused one case of stable disease (Table 1). This 24% increase in survival compared to animals in control Group 1 is statistically significant at p = 0.048 (unpaired *t* test). Taxol administered i.p. at a dose of 18 mg/kg on a daily x 5 schedule (Group 3) resulted in an MDS value of 40.8 days for four mice, with one animal experiencing a partial response at the end of the study (12 mg tumor on Day 60). The 84% survival extension compared to Group 1 control mice was significant (p = 0.001; unpaired *t* test). In general, the antitumor compounds were highly active in the HCT116 test (Table 3). Every mouse experienced a durable complete response or a partial response at the end of the study (Day 60). No tumor in any treated animal reached the 2.0 g endpoint, except for compound 0670.

Antitumor compound 0670 produced a mix of cases of tumor shrinkage and tumors reaching the 2.0 g cut-off size. Two complete responses and one partial response were documented for compound 0670 treated mice (Group 7); an MDS value of 29.8 days was calculated for the remaining two animals in this group (significant versus Group 1 at p = 0.038; unpaired *t* test).

On Day 60, the small tumors in the animals experiencing durable, partial HCT116 shrinkage (partial responses) were excised and weighed. A comparison of the actual weights of these small neoplasms with the weight estimated from caliper measurement translated into mg tumor weight demonstrated agreement between the two sets of HCT116 weights on Day 60. All mice designated partial responses in Table 3 clearly experienced substantial tumor shrinkage from the Day 1 carcinoma starting size, as evidenced by the actual HCT116 weights on Day 60.

Eleven of thirteen antitumor compounds were well tolerated in the study. For these eleven compounds, no toxic deaths were reported, and group mean weight losses were generally in the 8%-12% range on Day 6. This is well within acceptable limits for side effects for cancer chemotherapy drugs set by the NCI. As noted in Table 3, antitumor compound 0983 caused two toxic deaths and produced group mean weight losses of over 20% on Days 6-10. Taxol was well-tolerated on both schedules, causing only modest weight loss and no deaths.

All thirteen antitumor compounds submitted for evaluation against the HCT116 human colon cancer xenograft demonstrated significant activity against this colon tumor. Every animal treated experienced a durable partial or complete shrinkage of its neoplasm.

The chemotherapy drug Taxol was included in the study as the positive drug control. Taxol itself on a qd x 1 schedule extended survival by a modest 24% over control mice, and produced no complete responses or partial responses. Taxol given on its optimal qd x 5 schedule caused a 84% survival extension compared to control animals, and produced one partial response. Therefore the efficacy achieved with Taxol administration was far less than that achieved with the other antitumor compounds.

### Example 13

### In Vivo Evaluation of Antitumor Compounds Against the MX-1 Human Breast Carcinoma Xenograft

Thirteen antitumor compounds were formulated for an i.v. dosing regime (single i.v. bolus, qd x 1 schedule) prepared in the following vehicle: 5% ethanol, 5% Cremophor and 90% isotonic saline. Compounds were formulated in the manner as described for Solution 1 of Example 10. Taxol (paclitaxel, Bristol Meyers Squibb), used as a control, was obtained as the marketed pharmaceutical drug. The dosing volume was 0.3 ml per 20 g mouse.

The MX-1 carcinoma is representative of estrogen-independent human breast carcinomas and is sensitive to a number of chemotherapy drugs including Taxol. In fact, Taxol, on its optimal five day schedule, can produce tumor shrinkage in MX-1 bearing mice, but is much less effective on a qd x 1 schedule, as shown in this study.

Female NCr-nude mice bearing MX-1 neoplasms (implanted subcutaneously in the flank with 1 mm³ human carcinoma fragments) were pair-matched into groups of six mice each on Day 1, with the mean tumor size ranging from 219-226 mg. Treatment groups consisted of: treatment with the vehicle (Group 1); treatment with Taxol (Group 2); and treatment with antitumor compounds 0503, 0536, 0663, 0670, 0687, 0706, 0908, 0947, 0951, 0983, 0999, 1003 and 1011 (Groups 3-15, respectively). Treatment groups are summarized in Table 4.

The antitumor compounds were given intravenously in a single maximum tolerated dose (MTD) appropriate for each compound. The Taxol controls were given a split dose (qd x 1 schedule) on Day 1. The MTDs for the antitumor compounds were calculated from earlier single dose data for mice receiving i.v. administration of these compounds. All vehicle control animals in the split dosing regimes were dosed twice intravenously with half the total volume being given separated by one hour. The study was terminated on Day 60.

The results are summarized in Table 4 and include mean days of survival (MDS), number of toxic deaths, number of survivors, number of complete or partial responses, and number with stable disease. The mean days of survival is the number of days after which a neoplasm reached a size of 2.0 g and the animal was euthanized. A complete response is obtained if the tumor is not palpable at the end of the study. A partial response is obtained if the tumor shrinks to a size smaller than its size on Day 1 of the study. "Stable disease" occurs when the treatment limits the growth of the neoplasm to a small size that does not reach 2.0 g in size by the termination of the study.

**Table 4**

| Group | Antitumor Compound | MDS (n) | Toxic Deaths | Survivors | Complete Response | Partial Response | Stable Disease | Mean Score |
|---|---|---|---|---|---|---|---|---|
| 1 | Vehicle control | 19.7 ± 3.5 (6) | 0 | 0 | 0 | 0 | 0 | 1 ± 0 |
| 2 | Taxol (36 mg/kg) | 27.8(1) | 0 | 1 | 0 | 0 | 1 | 1.2 ± 0.8 |
| 3 | No. 0503 (72.6 mg/kg) | -- | 0 | 6 | 4 | 2 | 0 | 9.7 ± 0.2 |
| 4 | No. 0536 (49 mg/kg) | -- | 2 | 4 | 4 | 0 | 0 | 6.7 ± 2.1 |
| 5 | No. 0663 (159 mg/kg) | 28.6 (1) | 0 | 5 | 4 | 0 | 1 | 7.7 ± 1.5 |
| 6 | No. 0670 (123 mg/kg) | 28.5 ± 6.1 (4) | 0 | 2 | 1 | 1 | 0 | 4.3 ± 1.6 |
| 7 | No. 0687 (159 mg/kg) | -- | 1 | 5 | 5 | 0 | 0 | 8.3 ± 1.7 |
| 8 | No. 0706 (108 mg/kg) | -- | 1 | 5 | 5 | 0 | 0 | 8.3 ± 1.7 |
| 9 | No. 0908 (159 mg/kg) | -- | 0 | 6 | 5 | 1 | 0 | 9.7 ± 0.3 |
| 10 | No. 0947 (76.9 mg/kg) | 48.3 (1) | 2 | 3 | 3 | 0 | 0 | 5.5 ± 2.1 |
| 11 | No. 0951 (72.6 mg/kg) | -- | 0 | 6 | 5 | 1 | 0 | 9.5 ± 0.5 |
| 12 | No. 0983 (67.4 mg/kg) | -- | 0 | 6 | 6 | 0 | 0 | 10 ± 0 |
| 13 | No. 0999 (49 mg/kg) | 45.3 (1) | 0 | 5 | 4 | 1 | 0 | 8.2 ± 1.2 |
| 14 | No. 1003 (49 mg/kg) | 29.1 ± 10.8 (2) | 0 | 4 | 4 | 0 | 0 | 7.3 ± 1.7 |
| 15 | No. 1011 (108 mg/kg) | -- | 0 | 6 | 6 | 0 | 0 | 10 ± 0 |

All MX-1 carcinomas grew rapidly and progressively in all six animals receiving vehicle (Group 1). The MDS for these mice to reach the 2.0 g tumor cut-off point was 19.7 days. Taxol given i.v. at a single dose of 36 mg/kg (Group 2) was not well tolerated in this study; four toxic deaths were recorded. One mouse experienced a condition of stable disease and the other remaining animal achieved an MDS of 27.8 days (calculated). Therefore, Taxol demonstrated moderate anti-tumor activity in the two mice that did not experience sever side effects.

A significant tumor response rate was achieved in this MX-1 study with thirteen antitumor compounds. Five antitumor compounds (0536, 0687, 0706, 0983, and 1011) caused complete and durable tumor regressions in all the treated mice (with four toxic deaths occurring also). Three other antitumor compounds (0503, 0908, 0951) produced durable complete responses and partial responses in 100% of the treated animals. In all, 14 complete responses and 4 partial responses were produced by these three compounds, with no serious toxicity reported. The final set of five antitumor compounds (0663, 0670, 0947, 0999, and 1003) caused a mixed set of responses including 16 complete responses, 2 partial responses, one case of stable disease, and nine cases where tumors in treated mice reached the 2.0 g cut-off, but with MDS values greater than that calculated for the vehicle-control group. Two compound 0947-treated mice experienced toxic deaths.

### Example 14

### In Vivo Evaluation of Antitumor Compounds Against DU 145 Human Prostate Carcinoma Xenografts

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) was obtained as the marketed pharmaceutical drug. The dosing volume was 0.3 ml per 20 g mouse.

Male NCr-nude mice were implanted subcutaneously with 1 mm³ DU145 prostate carcinoma fragments in the flank and sorted into treatment groups of five mice each. The DU145 group mean size range was 223-228 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered i.p. on a qd x 5 schedule at a daily dose of 18 mg/kg. The vehicle was administered i.v. to control mice in Group 1 on the qd x 1 schedule. The study was terminated on Day 91. Treatment groups are detailed in Table 5.

**Table 5**

| **Group** | **Compound** | **Mg/kg** | **Route** | **Schedule** |
|---|---|---|---|---|
| 1 | Vehicle | --- | i.v. | Qd x 1 |
| 2 | Taxol | 24 | i.v. | Qd x 1 |
| 3 | Taxol | 18 | i.p | Qd x 5 |
| 4 | 0499 | 77 | i.v. | Qd x 1 |
| 5 | 0503 | 72.6 | i.v. | Qd x 1 |
| 6 | 0670 | 123.1 | i.v. | Qd x 1 |
| 7 | 0706 | 107.6 | i.v. | Qd x 1 |
| 8 | 0838 | 72.6 | i.v. | Qd x 1 |
| 9 | 0854 | 76.9 | i.v. | Qd x 1 |
| 10 | 1011 | 107.6 | i.v. | Qd x 1 |

The results are summarized in Table 6 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 6**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 34.3 ± 9.4 (3) | 1 | 1 | 1 | 0 | 0 | 2.8 ± 1.8 |
| 2 | 37.4 ± 8.9 (3) | 1 | 1 | 0 | 0 | 1 | 2 ± 1 |
| 3 | --- | 5 | 0 | 0 | 0 | 0 | 0 ± 0 |
| 4 | 67.2 ± 8.3 (3) | 0 | 2 | 1 | 0 | 1 | 4.4 ± 1.4 |
| 5 | --- | 1 | 4 | 2 | 0 | 2 | 6.2 ± 1.9 |
| 6 | 89.7 (1) | 0 | 4 | 0 | 1 | 3 | 4.8 ± 0.9 |
| 7 | 67.4 ± 1.9 (2) | 0 | 3 | 2 | 0 | 1 | 6.2 ± 1.7 |
| 8 | 59.5 (1) | 0 | 4 | 2 | 1 | 1 | 7 ± 1.5 |
| 9 | --- | 5 | 0 | 0 | 0 | 0 | 0 ± 0 |
| 10 | 55.8 ± 16.5 (2) | 3 | 0 | 0 | 0 | 0 | 0.8 ± 0.6 |

DU145 tumors grew progressively in three of the five mice treated with vehicle (Group 1); an MDS value of 34.3 days was calculated for these animals. One mouse in Group 1 died of unknown causes on Day 40 and the carcinoma of another mouse in Group 1 regressed slowly from its 220 mg starting size of pair-match until the neoplasm was no longer palpable on Day 71. This latter case is most likely an example of a poor tumor take.

The prostate carcinomas in three of five animals receiving Taxol at 24 mg/kg (i.v.; qd x 1) grew steadily and reached the 1.5 g endpoint with a MDS = 37.4 days (Group 2). This 9% increase in survival compared to Group 1 controls is not statistically different (p = 0.82; unpaired t-test). One animal in Group 2 died on Day 32, possibly from drug-related side effects. The fifth mouse in Group 2 did not experience net tumor growth over the 91 day study; the carcinoma was the same size (196 mg) on Day 6 as on Day 91. Taxol administered i.p. at a dose of 18 mg/kg on a qd x 5 schedule (Group 3) was highly toxic. All five mice died of drug-related side effects including substantial (often more than 20%) weight loss.

Antitumor compounds 0503 and 0838 produced a total response rate of at least 50%. Antitumor compound 0838 (Group 8) was active, producing 2 complete responses and a 60% total response rate. Antitumor compound 0503 (Group 5) caused a mix of complete responses, partial responses, stable disease responses, and extended MDS values compared to Group 1 controls.

Antitumor compounds 0499, 0670 and 0706 gave a total response rate between 20% and 50%. Compound 0706 (Group 7) produced 2 complete responses and one case of stable disease and an MDS value of 67.4 days for the remaining 2 mice (strong trend towards being significantly different from the MDS = 34.3 days for Group 1 controls at p = 0.07; unpaired t-test). Antitumor compound 0706 was also well-tolerated. The other four compounds produced a mix of complete responses, partial responses, stable disease cases, and MDS values well extended compared to the Group 1 MDS of 34.3 days.

Two compounds 0854 and 1011, caused at least two toxic lethalities at the administered dose out of the five animals treated with each compound (40% mortality rate or higher). Antitumor compound 0854 was 100% lethal to the mice at the administered dose. All of these compounds produced weight loss over 20% in a number of the treated mice prior to their deaths.

The remaining compounds were well-tolerated in the experiment, each causing no or one toxic death, and producing 8 - 12% maximal mean weight losses in the second week of the test. These modest side effects are well within NCI guidelines for acceptable toxicity in mice caused by administration of chemotherapy drugs.

### Example 15

### In Vivo Evaluation of Antitumor Compounds Against A2780 Human Ovarian Carcinoma Xenografts

Antitumor compounds were prepared in a vehicle of 5% ethanol, 5% Cremophor and 90% isotonic saline in the manner as described for Solution 1 in Example 10. Taxol (paclitaxel; Bristol Meyers Squibb) and Taxotere (docetaxel; Rhone-Poulenc Rorer)Taxol were obtained as the marketed pharmaceutical drugs. The dosing volume was 0.3 ml per 20 g mouse.

Female NCr-nude mice were implanted subcutaneously with 1 mm³ A2780 melanoma fragments in the flank and sorted into treatment groups of 5 mice each, except for the Taxotere treatment group which was a group of six mice. The A2780 group mean size range was 237-243 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered i.p. on a qd x 5 schedule at a daily dose of 15 mg/kg. Taxotere was given i.v. on a qd x 1 schedule at a dose of 70 mg/kg. The vehicle was administered i.v. to control mice in Group 1 on the qd x 1 schedule. The study was terminated on Day 60. Treatment groups are detailed in Table 7.

**Table 7**

| **Group** | **Compound** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 15 | i.p. | qd x 5 |
| 4 | 0503 | 72.6 | i.v. | qd x 1 |
| 5 | 0536 | 49 | i.v. | qd x 1 |
| 6 | 0663 | 159.4 | i.v. | qd x 1 |
| 7 | 0687 | 159.4 | i.v. | qd x 1 |
| 8 | 0706 | 107.6 | i.v. | qd x 1 |
| 9 | 0908 | 159.4 | i.v. | qd x 1 |
| 10 | 0947 | 76.9 | i.v. | qd x 1 |
| 11 | 0951 | 72.6 | i.v. | qd x 1 |
| 12 | 0983 | 72.6 | i.v. | qd x 1 |
| 13 | 0999 | 49 | i.v. | qd x 1 |
| 14 | 1003 | 49 | i.v. | qd x 1 |
| 15 | 1011 | 107.6 | i.v. | qd x 1 |
| 16 | Taxotere | 70 | i.v. | qd x 1 |

The results are summarized in Table 8 and include mean days of survival (MDS), number of toxic deaths, number of survivors, number of complete or partial responses, and number with stable disease. The mean days of survival is the number of days after which a neoplasm reached a size of 2.0 g and the animal was euthanized. A complete response is obtained if the tumor shrinks to a size smaller than its size on Day 1 of the study. "Stable disease" occurs when the treatment limits growth of the neoplasm to a small size that does not reach 2.0 g in size by the termination of the study.

**Table 8**

| **Group** | **MDS (n)** | **Toxic Deaths** | **Survivors** | **Complete Response** | **Partial Respons** | **Stable Disease** | **Mean Score** |
|---|---|---|---|---|---|---|---|
| 1 | 11.7 ± 2.5 (5) | 0 | 0 | 0 | 0 | 0 | 1.2 ± 0.2 |
| 2 | 21.9 ± 5.5 (5) | 0 | 1 | 0 | 0 | 0 | 1.8 ± 0.4 |
| 3 | 26.5(1) | 4 | 0 | 0 | 0 | 0 | 0.6 ± 0.6 |
| 4 | 32.4 ± 4.7 (5) | 0 | 0 | 0 | 0 | 0 | 2.8 ± 0.2 |
| 5 | 42.6 ± 3.3 (5) | 0 | 0 | 0 | 0 | 0 | 3 ± 0 |
| 6 | 36.7 ± 7.0 (3) | 0 | 2 | 0 | 1 | 1 | 4.4 ± 1 |
| 7 | 28.6 ± 4.1 (5) | 0 | 0 | 0 | 0 | 0 | 2.4 ± 0.4 |
| 8 | 37.0 ± 4.1 (5) | 0 | 0 | 0 | 0 | 0 | 3 ± 0 |
| 9 | 43.1 ± 8.0 (2) | 1 | 2 | 0 | 1 | 1 | 3.8 ± 1.5 |
| 10 | 32.7 ± 5.4 (2) | 1 | 2 | 0 | 2 | 0 | 4.8 ± 1.8 |
| 11 | 43.2 ± 2.5 (4) | 0 | 1 | 0 | 0 | 1 | 3.6 ± 0.6 |
| 12 | --- | 5 | 0 | 0 | 0 | 0 | 0 ± 0 |
| 13 | 29.1 ± 9.2 (3) | 0 | 2 | 1 | 0 | 1 | 4.4 ± 1.5 |
| 14 | 39.5 ± 10.2 (3) | 0 | 2 | 0 | 2 | 0 | 4.6 ± 1.2 |
| 15 | 34.4(1) | 0 | 4 | 1 | 3 | 0 | 8 ± 1.3 |
| 16 | 27.4 ± 3.5 (6) | 0 | 0 | 0 | 0 | 0 | 2.5 ± 0.3 |

The ovarian tumors in all five mice in Group 1 receiving vehicle grew progressively and reached the 2.0 g cut-off with a MDS value of 11.7 days. Thus, this ovarian carcinoma xenograft is a fast growing neoplasm that kills its host in a relatively short time period.

Taxol given on a qd x 1 schedule (Group 2) was moderately efficacious in this test. The MDS value of 21.9 days calculated for Group 2 mice represents a 87% increase in survival compared to Group 1 control animals, which is not significant at p = 0.13; unpaired t-test. One animal in Group 2 was a recorded case of stable disease on Day 61. Taxol administered i.p. at a dose of 15 mg/kg on a qd x 5 schedule (Group 3) was highly toxic; four mice succumbed to drug-related side effects.

Taxotere (70 mg/kg; i.v.; qd x 1) was more active than Taxol in this experiment. The MDS = 27.4 days for all five mice in Group 16 gives a calculated survival extension of 134% compared to Group 1 controls, which is statistically significant at p = 0.007 (unpaired t-test).

Six antitumor compounds (0663, 0908, 0947, 0999, 1003 and 1011) produced at least one complete response or partial response (Total response rate of at least 20%) in mice bearing A2780 ovarian carcinomas. Compound 1011 (Group 15), caused 3 partial responses and 1 complete response out of five animals, for a total response rate of 80%. Antitumor compound 1011 treatment was also well-tolerated, resulting in no toxic deaths and a maximal mean group weight loss of 11.3% on Day 5. Two other compounds, 0947 and 1003, each produced 2 partial responses among the five mice treated. These compounds also caused several cases of stable disease, and in general achieved significant survival extensions compared to Group 1 controls. For example, compounds 0663 (MDS = 36.7 days) and 0908 (MDS = 43.1 days) increased survival by 214% and 268%, respectively, as compared to Group 1 controls (p values of 0.006 and 0.003 respectively; unpaired t-test).

Compound 0951 produced stable disease and high MDS values, but no tumor shrinkage. Antitumor compound 0951 produced one case of stable disease, and a survival extension of 269% (MDS = 43.2 days) compared to Group 1 controls (p < 0.0001; unpaired t-test).

Four of the compounds (0503, 0536, 0687 and 0706) achieved significant MDS values, but did not cause any complete responses, partial responses, or cases of stable disease. Treatment with these compounds resulted in approximately 100% - 300% survival extensions compared to Group 1 controls. Antitumor compound 0536 produced a 264% increase in survival (MDS = 42.6 days) compared to Group 1 controls (p < 0.0001; unpaired t-test)

Only compound 0983 demonstrated excessive toxicity. All five mice treated with compound 0983 (Group 12) at its dose of 72.6 mg/kg were toxic lethalities at the administered dose. Only two other toxic deaths occurred in the study, one each in Groups 9 (0908) and 10 (0947). The groups treated with antitumor compounds (except Group 12) experienced maximal mean group weight losses in the 3% to 19% range around Day 11, and the animal weights rebounded thereafter. Thus the side effects recorded for all antitumor compounds (except 0983) were within the NCI acceptable range, and indicated that the animals in the A2780 experiment received proper MTD dosages.

### Example 16

### In Vivo Evaluation of Antitumor Compounds Against A375 Human Melanoma Xenografts

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) and Taxotere (docetaxel; Rhône-Poulenc Rorer) were obtained as the marketed pharmaceutical drugs. The dosing volume was 0.3 ml per 20 g mouse.

Female NCr-nude mice were implanted subcutaneously with 1 mm³ A375 melanoma fragments in the flank were sorted into treatment groups of six mice each, except the Taxotere treatment group which was a group of seven mice. The A375 tumor group mean size range was 206-212 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered i.p. on a qd x 5 schedule at a daily dose of 18 mg/kg. Taxotere was given i.v. on a qd x 1 schedule at a dose of 70 mg/kg. The vehicle was administered i.v. to control mice in Group 1 on the qd x 1 schedule. The study was terminated on Day 60. Treatment groups are detailed in Table 9.

**Table 9**

| **Group** | **Agent** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 18 | i.p. | qd x 5 |
| 4 | 0499 | 77 | i.v. | qd x 1 |
| 5 | 0503 | 72.6 | i.v. | qd x 1 |
| 6 | 0670 | 123.1 | i.v. | qd x 1 |
| 7 | 0706 | 107.6 | i.v. | qd x 1 |
| 8 | 0838 | 72.6 | i.v. | qd x 1 |
| 9 | 0854 | 63.3 | i.v. | qd x 1 |
| 10 | 1011 | 107.6 | i.v. | qd x 1 |
| 11 | Taxotere | 70 | i.v. | qd x 1 |

The results are summarized in Table 10 and include mean days of survival (MDS), number of toxic deaths, number of survivors, number of complete or partial responses, and number with stable disease. The mean days of survival is the number of days after which a neoplasm reached a size of 2.0 g and the animal was euthanized. A complete response is obtained if the tumor shrinks to a size smaller than its size on Day 1 of the study. "Stable disease" occurs when the treatment limits growth of the neoplasm to a small size that does not reach 2.0 g in size by the termination of the study.

**Table 10**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 14.4 ± 0.9 (6) | 0 | 0 | 0 | 0 | 0 | 1 ± 0 |
| 2 | 18.1 ± 0.6 (4) | 2 | 0 | 0 | 0 | 0 | 0.8 ± 0.3 |
| 3 | --- | 6 | 0 | 0 | 0 | 0 | 0 ± 0 |
| 4 | 30.5 ± 3.5 (3) | 2 | 1 | 0 | 0 | 1 | 2.3 ± 0.9 |
| 5 | 28.1 ± 5.8 (3) | 2 | 1 | 1 | 0 | 0 | 2.8 ± 1.5 |
| 6 | 15.3 ± 1.5 (6) | 0 | 0 | 0 | 0 | 0 | 1.2 ± 0.2 |
| 7 | 28.3 ± 1.6 (5) | 1 | 0 | 0 | 0 | 0 | 1.8 ± 0.4 |
| 8 | 33.5 ± 3.4 (5) | 0 | 1 | 1 | 0 | 0 | 3.8 ± 1.2 |
| 9 | --- | 6 | 0 | 0 | 0 | 0 | 0 ± 0 |
| 10 | 47.5 ± 5.4 (3) | 3 | 0 | 0 | 0 | 0 | 1.5 ± 0.7 |
| 11 | 21.5 ± 0.5 (7) | 0 | 0 | 0 | 0 | 0 | 2 ± 0 |

The A375 melanoma implants grew rapidly and progressively in all six mice treated with vehicle (Group 1). These neoplasms reached the 2.0 g cut-off with a MDS value of 14.4 days.

Animals administered i.v. Taxol on a qd x 1 schedule (24 mg/kg; Group 2) experienced a modest therapeutic benefit from the chemotherapy drug. The MDS value of 18.1 days represented a 26% survival extension compared to Group 1 controls. This survival increase was statistically significant (p = 0.016; unpaired t-test). Taxol given on a qd x 5 schedule (18 mg/kg; i.p.) was highly toxic to the mice. All six animals dosed at 18 mg/kg died of toxicity.

Taxotere given i.v. at a dose of 70 mg/kg (qd x 1; Group 11) produced a MDS = 21.5 days. This 49% survival extension was significant compared to Group 1 controls at p < 0.0001 (unpaired t-test).

Three antitumor compounds (0499, 0503 and 0838) each produced at least one case of complete response, partial response, or stable disease on Day 60 (when the study was terminated), and excellent survival extensions in the other treated mice as evidenced by the MDS values for these agents. Antitumor compound 0503 (Group 5) caused 1 complete response and a survival extension of 95% over the MDS for Group 1 controls (significant at p = 0.012; unpaired t-test). One complete response was achieved with antitumor compound 0838 (Group 8) administration, and the remaining five mice experienced a 133% survival extension over Group 1 controls (p = 0.0002; unpaired t-test). Antitumor compound 0499 caused one case of stable disease and a significant MDS value.

Antitumor compound 0706 improved survival as compared to Group 1 controls at p < 0.05 (unpaired t-test). One antitumor compound, 0670, was not active in the study.

Two antitumor compounds (0854 and 1011) caused at least three toxic deaths each among the six mice per group receiving these compounds. In general, 20 - 30% mean group weight losses were recorded for animals treated with these compounds. The side effect profile for other groups administered antitumor compounds was generally within NCI guidelines; mean weight losses for groups ranged between 5 - 19% early in the test, and then animal weights rebounded.

The acceptable level of weight losses seen with the efficacious agents in the A375 experiment indicates that these mice were treated with reasonable MTDs of these antitumor compounds.

### Example 17

### In Vivo Evaluation of Antitumor Compounds Against Panc-1 Human Pancreatic Carcinoma

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) was obtained as the marketed pharmaceutical drug. The dosing volume was 0.3 ml per 20 g mouse.

Female NCr-nude mice were implanted subcutaneously with 1 mm³ Panc-1 pancreatic carcinoma fragments in the flank and sorted into treatment groups of six mice each. The Panc-1 tumor group mean size range was 215-223 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 18 mg/kg per split dose (total dose = 36 mg/kg). The vehicle was administered i.v. to control mice in Group 1 on the qd x 1 schedule. The study was terminated on Day 60. Treatment groups are detailed in Table 11.

**Table 11**

| **Group** | **Agent** | **Mg/kg** | **Route** | **Schedule** |
|---|---|---|---|---|
| 1 | Vehicle | --- | i.v. | qd x 1 |
| 2 | Taxol | 36 | i.v. | qd x 1 |
| 3 | 0499 | 77 | i.v. | qd x 1 |
| 4 | 0503 | 72.6 | i.v. | qd x 1 |
| 5 | 0670 | 123.1 | i.v. | qd x 1 |
| 6 | 0706 | 107.6 | i.v. | qd x 1 |
| 7 | 0838 | 72.6 | i.v. | qd x 1 |
| 8 | 0854 | 76.9 | i.v. | qd x 1 |

The results are summarized in Table 12 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 12**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 18.5 ± 1.8 (6) | 0 | 0 | 0 | 0 | 0 | 1.2 ± 0.2 |
| 2 | 41.9 ± 5.1 (3) | 3 | 0 | 0 | 0 | 0 | 1.6 ± 0.7 |
| 3 | 56.1 ± 1.0 (2) | 0 | 4 | 2 | 1 | 1 | 7.4 ± 1.4 |
| 4 | 52.5 ± 2.1 (3) | 0 | 3 | 0 | 0 | 3 | 3.6 ± 0.4 |
| 5 | 51.2 ± 5.9 (2) | 1 | 3 | 1 | 0 | 2 | 4.2 ± 1.7 |
| 6 | 59.5 (1) | 0 | 5 | 0 | 3 | 2 | 6.6 ± 1 |
| 7 | 54.2 ± 0.9 (2) | 1 | 3 | 0 | 2 | 3 | 3.6 ± 1.1 |
| 8 | --- | 6 | 0 | 0 | 2 | 0 | 0 ± 0 |

Panc-1 tumors grew progressively in all six mice treated with vehicle (Group 1); a MDS = 18.5 days was calculated for this control group.

I.v. administration of 36 mg/kg Taxol (qd x 1; Group 2) caused a 126% survival extension in three mice (MDS = 41.9 days) compared to Group 1 controls (significant at p = 0.0009; unpaired *t*-test). Three other animals receiving Taxol died of toxicity.

Three antitumor compounds (0499, 0670 and 0706) produced total response rates in the 20% - 50% range. Compound 0499 (Group 3) achieved 2 complete responses and 1 partial response (along with one case of stable disease) from among six animals. Two other compounds in the Panc-1 experiment 1 (0503 and 0838) did not cause any complete responses or partial responses, but each produced three cases of stable disease.

The MDS values calculated for compounds (except 0854) in this experiment (0499, 0503, 0670, 0706 and 0838) were in the range of 47 - 56 days, substantially longer than the MDS value of 18.5 days calculated for Group 1 control mice (all values are significant at p ≤ 0.05; unpaired *t*-test).

Antitumor compound 0854 (Group 8) was highly toxic to mice at its dose of 76.9 mg/kg. All six animals receiving 0854 died from drug-related side effects. One toxic death was recorded in each of Group 5 (0670), and Group 7 (0838); these two animals experienced severe weight loss before succumbing to toxicity. Otherwise, mean group weight losses were in the 2% - 12% range, indicating that reasonable MTDs had been selected for all antitumor compounds in this experiment with the exception of compound 0854.

### Example 18

### In Vivo Evaluation of Antitumor Compounds 0854 and 1011 Against Panc-1 Human Pancreatic Carcinoma

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) was obtained as the marketed! pharmaceutical drug. The dosing volume was 0.3 ml per 20 g mouse.

Female NCr-nude mice were implanted subcutaneously with 1 mm³ Panc-1 pancreatic carcinoma fragments in the flank and sorted into treatment groups of six mice each. The Panc-1 tumor group mean size range was 192-213 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered at a dose of 15 mg/kg i.p. on a qd x 5 schedule. The vehicle was administered i.v. to control mice in Group 1 on the qd x 1 schedule. The test was terminated on Day 63. Treatment groups are detailed in Table 13.

**Table 13**

| **Group** | **Agent** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 15 | i.p. | qd x 5 |
| 4 | 0854 | 46.6 | i.v. | qd x 1 |
| 5 | 1011 | 107.6 | i.v. | qd x 1 |

The results are summarized in Table 14 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 14**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 23.0 ± 3.1 | 0 | 1 | 0 | 0 | 1 | 2 ± 0.8 |
| 2 | 34.7 ± 3.4 | 1 | 1 | 0 | 0 | 1 | 2.2 ± 0.8 |
| 3 | --- | 4 | 1 | 0 | 1 | 0 | 1.2 ± 1.2 |
| 4 | --- | 6 | 0 | 0 | 0 | 0 | 0 ± 0 |
| 5 | --- | 3 | 3 | 0 | 3 | 0 | 4 ± 1.8 |

The Panc-1 neoplasms of mice receiving vehicle (Group 1) reached the 1.5 g cut-off with a calculated MDS of 23.0 days. One carcinoma in the vehicle control group did not grow out, presumably because of a poor tumor take.

Taxol (Group 2) administered i.v. at a dose of 24 mg/kg (qd x 1) produced one case of stable disease, and caused a MDS of 34.7 days in four other mice (significantly different from Group 1 controls at p = 0.038; unpaired *t*-test). One mouse died from the toxicity caused by this dose of Taxol. Taxol given i.p. at a dose of 15 mg/kg (qd x 5) caused four toxic deaths among the six animals in Group 3.

Antitumor compound 1011 produced an overall response rate of 100%. Antitumor compound 1011 produced three partial responses, but also caused three toxic lethalities at the administered dose. Antitumor compound 0854 was toxic; all animals receiving this agent died from drug-related side effects.

### Example 19

### In Vivo Evaluation of Antitumor Compounds Against VM46 Human Colon Carcinoma

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) and Taxotere (docetaxel; Rhône-Poulenc Rorer) were obtained as the marketed pharmaceutical drugs. The dosing volume was 0.3 ml per 20 g mouse.

Female NCr-nude mice were implanted subcutaneously with 1 mm³ VM46 colon carcinoma fragments in the flank and sorted into treatment groups of six mice each. The VM46 tumor group mean size range was 181-188 mg. Dosing was initiated on Day 1. The antitumor compounds were given i.v. at an estimated maximum tolerated dose (MTD) appropriate for each agent on a qd x 1 schedule (single administration of compound). The i.v. taxol was given on the qd x 1 schedule as two split doses (one hour between) of 12 mg/kg per split dose (total dose = 24 mg/kg). Taxol was also administered at a dose of 15 mg/kg i.p. on a qd x 5 schedule. Taxotere was given i.v. at a dose of 70 mg/kg on a qd x 1 schedule. The vehicle was administered i.v. to control mice in Group 1 on the qd x 1 schedule. The test was terminated on Day 64. Treatment groups are detailed in Table 15.

**Table 15**

| **Group** | **Compound** | **mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 15 | i.p. | qd x 5 |
| 4 | 0499 | 77 | i.v. | qd x 1 |
| 5 | 0503 | 72.6 | i.v. | qd x 1 |
| 6 | 0670 | 123.1 | i.v. | qd x 1 |
| 7 | 0706 | 107.6 | i.v. | qd x 1 |
| 8 | 0838 | 72.6 | i.v. | qd x 1 |
| 9 | 0854 | 46.6 | i.v. | qd x 1 |
| 10 | 0983 | 67.4 | i.v. | qd x 1 |
| 11 | Taxotere | 70 | i.v. | qd x 1 |

The results are summarized in Table 16 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 16**

| Response Summary for the VM46 Study (Colon Carcinoma) | | | | | | | |
|---|---|---|---|---|---|---|---|
| **Group** | **MDS (n)** | **Toxic Deaths** | **Survivors** | **Complete Response** | **Partial Response** | **Stable Disease** | **Mean Score** |
| 1 | 31.0 ± 4.4 (6) | 0 | 0 | 0 | 0 | 0 | 1.2 ± 0.2 |
| 2 | 43.3 ± 3.7 (4) | 0 | 2 | 0 | 1 | 1 | 3.8 ± 1.4 |
| 3 | 36.1 ± 12.2 (2) | 3 | 1 | 0 | 1 | 0 | 1.8 ± 1.3 |
| 4 | 41.9 ± 4.2 (5) | 0 | 1 | 0 | 0 | 1 | 2 ± 0.3 |
| 5 | 56.1 (1) | 0 | 5 | 0 | 4 | 1 | 7.3 ± 1.2 |
| 6 | 40.1 ± 5.8 (5) | 0 | 1 | 0 | 1 | 0 | 2.8 ± 1.2 |
| 7 | 48.6 ± 0.9 (3) | 0 | 3 | 1 | 2 | 0 | 6.2 ± 1.4 |
| 8 | 49.9 ± 13.3(2) | 0 | 4 | 0 | 1 | 3 | 4.8 ± 1 |
| 9 | 52.9 ± 6.6 (2) | 1 | 3 | 0 | 2 | 1 | 4.7 ± 1.6 |
| 10 | 56.0 ± 6.8 (2) | 3 | 1 | 0 | 0 | 1 | 1.7 ± 0.8 |
| 11 | 45.3 ± 3.6 (6) | 0 | 0 | 0 | 0 | 0 | 2 ± 0 |

The colon cancers in all six mice receiving vehicle (Group 1) grew progressively, and reached the 1.5 g endpoint with a calculated MDS value of 31.0 days.

Taxol (Group 2) given at 24 mg/kg (i.v.; qd x 1) caused one partial response, one case of stable disease, and an MDS = 43.3 days for the remaining four mice in the group (not significantly different from controls at p = 0.086; unpaired *t*-test). Taxol administered i.p. at a dose of 15 mg/kg (qd x 5) was excessively toxic, causing three mortalities among the six treated animals. Taxotere produced an MDS value of 45.3 days (significant at p ≤ 0.05 compared to Group 1 controls; unpaired *t*-test).

Two antitumor compounds, 0503 and 0706, produced response rates of 66.7% and 56% respectively, indicating that these two compounds were efficacious among those tested. Antitumor compound 0503 produced four partial responses and two cases of stable disease, and antitumor compound 0706 caused one complete response and two partial responses.

Three other compounds (0670, 0838 and 0854) achieved response rates in the 16.7% - 40% range. A survival extension in the range of 11 to 20 days longer than the MDS of 31.0 days calculated for the control group was obtained with compound 0854. Compound 0983 with a 0% response rate did produce a statistically significant (p #0.05) MDS value as compared to Group 1 controls.

Five of the seven antitumor compounds (0499, 0503, 0670, 0706 and 0838) were well-tolerated in the VM46 study, causing no toxic deaths and maximal mean group weight losses in the 3% -14% range on Day 8. Acceptable toxicity (not more than one death per group, and maximal mean weight loss under 12%) was also reported for compound 0854. One antitumor compound, 0983, produced toxicity causing the deaths of three animals in Group 10.

### Example 20

### In Vivo Evaluation of Antitumor Compound 0503 Against SKMES Human Lung Carcinoma Xenograft

Antitumor compound 0503 was formulated for i.v. administration over a wide dose range in a study to investigate the efficacy of this compound against the SKMES human lung cancer xenograft. The antitumor compound was prepared in a vehicle of 10% ethanol and 90% Liposyn III in the manner as described for Emulsion 3 of Example 9.

Female Nu/Nu mice (6-8 weeks of age; implanted with 1 mm³ SKMES lung carcinoma fragments in the flank) were sorted into five treatment groups of six mice each. SKMES size ranges and group mean SKMES size ranges were 126-405 mg and 235-240 mg, respectively. Treatment groups consisted of: vehicle only (Group 1), and antitumor compound 0503 (dosages of 104 mg/kg, 72.8 mg/kg, 41.6 mg/kg, and 10.4 mg/kg; Groups 2-5, respectively). Treatments were administered as a single IV bolus on Day 1, and the study was terminated on Day 60.

The results are summarized in Table 17 and include mean days of survival (MDS), number of toxic deaths, number of survivors, number of complete or partial responses, and number with stable disease. The mean days of survival is the number of days after which a SKMES neoplasm reached a size of 2.0 g and the animal was euthanized. A complete response is obtained if the tumor is not palpable at the end of the study. A partial response is obtained if the tumor shrinks to a size smaller than its size on Day 1 of the study. Stable disease occurs when the treatment limits the growth of the neoplasm to a small size that does not reach 2.0 g in size by the termination of the study.

**Table 17**

| Group | MDS (n) | Toxic Deaths | Survivors | Complete Response | Partial Response | Stable Disease | % Total Response |
|---|---|---|---|---|---|---|---|
| 1 | 17.4 ± 1.3 (6) | 0 | 0 | 0 | 0 | 0 | 0 |
| 2 | 46.6 ± 3.2 (3) | 0 | 3 | 3 | 0 | 0 | 50.0 |
| 3 | 37.4 ± 5.2 (4) | 0 | 2 | 1 | 1 | 0 | 33.3 |
| 4 | 33.8 ± 3.4 (5) | 0 | 1 | 0 | 0 | 1 | 0 |
| 5 | 26.0 ± 2.5 (6) | 0 | 0 | 0 | 0 | 0 | 0 |

Three mice in Group 2 were recorded as complete tumor regressions. One complete response and one partial response were recorded for Group 3. The MDS values for the other mice in Groups 2 and 3, (46.6 days and 37.4 days respectively) were highly significant over the vehicle only treated group (Group 1) based on the difference in MDS values (p=0.0001, and p=0.0019; unpaired t-test). The survival extension for the other mice was 167% for Group 2 and 114% for Group 3.

### Example 21

### In Vivo Evaluation of the Oral Administration of Antitumor Compound 0687 Against SKMES Human Lung Carcinoma Xenograft

Antitumor compound 0687 was administered as a single bolus by the oral route in a study to investigate the efficacy of this compound against the SKMES human lung cancer xenograft over a wide dose range. The antitumor compound was prepared in a vehicle of 5% ethanol, 5% Cremophor and 90% isotonic saline in the manner as described in Example 6.

Female Nu/Nu mice (6-8 weeks of age; implanted with 1 mm³ SKMES lung carcinoma fragments in the flank) were sorted into treatment groups of six mice each. SKMES size ranges and group mean SKMES size ranges were 126-448 mg and 238-241 mg, respectively. Treatment groups consisted of: no treatment (Group 1), vehicle only (Group 2), Taxol (40 mg/kg; Group 3), antitumor compound 0687 (228 mg/kg; Group 4), and antitumor compound 0503 (73 mg/kg; Group 5). Treatments were administered as a single oral bolus on Day 1, and the study was terminated on Day 60.

The results are summarized in Table 18 and include mean days of survival (MDS), number of toxic deaths, number of survivors, number of complete or partial responses, and number with stable disease. The mean days of survival is the number of days after which a SKMES neoplasm reached a size of 2.0 g and the animal was euthanized. A complete response is obtained if the tumor is not palpable at the end of the study. A partial response is obtained if the tumor shrinks to a size smaller than its size on Day 1 of the study. Stable disease occurs when the treatment limits the growth of the neoplasm to a small size that does not reach 2.0 g in size by the termination of the study.

**Table 18**

| Group | MDS (n) | Toxic Deaths | Survivors | Complete Response | Partial Response | Stable Disease | % Total Response |
|---|---|---|---|---|---|---|---|
| 1 | 14.5 ± 1.7 (5) | 0 | 1 | 0 | 0 | 1 | 0 |
| 2 | 13.7 ± 1.4(6) | 0 | 0 | 0 | 0 | 0 | 0 |
| 3 | 13.2 ± 1.4 (6) | 0 | 0 | 0 | 0 | 0 | 0 |
| 4 | --- | 1^{a} | 5 | 4 | 1 | 0 | 100 |
| 5 | 29.8 ± 3.6 (3) | 0 | 3 | 3 | 0 | 0 | 50.0 |

| | | | | | | | |
|---|---|---|---|---|---|---|---|
| ^{a} The animal died due to injury. | | | | | | | |

Antitumor compound 0687 was highly active with a single oral administration. The total response rate was 100%. The total response rate is defined as the percentage of evaluable animals (excluding deaths due to procedure or toxicity) in a group that experience complete or partial responses at the termination of the study. Antitumor compound 0503 also resulted in three complete responses. Both compounds were significantly more effective than Taxol, which showed no statistically significant response.

### Example 22

### In Vivo Evaluation of the Oral Administration of Antitumor Compounds Against the MX-1 Human Breast Carcinoma Xenograft

Antitumor compounds were administered orally in a study to investigate whether such a formulation is efficacious against the MX-1 human breast carcinoma xenograft. Antitumor compound 0499 was formulated as Solution 1 in Example 6. Antitumor compound 0550 was formulated as Solution 2 in Example 6. Antitumor compound 0611 was formulated as Solution 3 in Example 6. Antitumor compound 0748 was formulated as Solution 4 in Example 6. Other antitumor compounds were formulated similarly. The compounds were prepared in a vehicle of 90% saline, 5% Cremophor, and 5% ethanol.

Female *nu*/*nu* mice (11-12 weeks of age; implanted subcutaneously with 1 mm³ MX-1 human breast carcinoma fragments in the flank) were sorted into one control group (n=10) and four treatment (n=6) groups (group mean tumor size of 47-49 mg). Treatments were administered a single oral bolus on Day 1 to the treatment groups as summarized in Table 19. The study was terminated on Day 63.

The results are summarized in Table 19 and include mean days of survival (MDS), number of toxic deaths, number of survivors, number of complete or partial responses, and number with stable disease. The mean days of survival is the number of days after which a tumor reached a size of 1.5 g and the animal was euthanized. A complete response is obtained if the tumor is not palpable at the end of the study. A partial response is obtained if the tumor shrinks to a size smaller than its size on Day 1 of the study. A Stable disease@ occurs when the treatment limits the growth of the tumor to a small size that does not reach 1.5 g in size by the termination of the study.

**Table 19**

| Group | Antitumor Compound | Dose (mg/kg) | Mean Day of Survival (n) | Maximum Weight Loss (Day) | Toxic Deaths | Complete Response | Partial Response | Stable Disease |
|---|---|---|---|---|---|---|---|---|
| 1 | vehicle | n/a | 21.5 ± 0.7 (8) | n/a | 0 | 0 | 0 | 2 |
| 2 | 0499 | 80 | 56.6 ± 2.1 (3) | -11.4% (7) | 0 | 2 | 0 | 1 |
| 3 | 0550 | 73 | n/a ± (0) | -16.1% (5) | 0 | 6 | 0 | 0 |
| 4 | 0611 | 113 | 58.5 ± 0(1) | -15.0% (5) | 1^{a} | 4 | 0 | 0 |
| 5 | 0748 | 200 | 32.4 ± 1.4 (5) | -4.6% (5) | 0 | 0 | 0 | 1 |

| | | | | | | | | |
|---|---|---|---|---|---|---|---|---|
| ^{a} Not toxicity related. | | | | | | | | |

The majority of the antitumor compounds evaluated are active in the MX-1 human breast carcinoma xenograft model, at well tolerated doses. In this study antitumor compound 0550 was efficacious, producing six complete tumor regressions in six animals treated. Compound 0611 was almost as active, producing four complete tumor regressions of five mice treated (one death was non-treatment related). Compound 0499 resulted in two complete tumor regressions of six mice treated, the other mice in the group experienced a highly significant survival increase versus controls before reaching the study endpoint. The response produced by compound 0748 was characterized by significant survival extensions compared to vehicle control mice.

In addition to survival and tumor regression responses, the effect of treatment on blood cell populations was assessed. The major effect of these antitumor compounds on blood cell populations was a marked reduction in the neutrophil cell number. As well, monocyte depletion correlated fairly well with neutrophil reduction for each antitumor compound. The antitumor compounds did not affect the general white blood cell population, lymphocytes, and platelets. There was a correlation between antitumor activity and reduced neutrophil count. In general, toxicity (as determined by neutrophil depletion) and weight loss correlated with efficacy. The most efficacious antitumor compound, 0550, produced complete remission in all six treated mice, the most body weight loss (16.1%, day 5) and the highest neutrophil reduction (95.9%, day 4). Importantly, the toxicities observed with the very efficacious antitumor compounds were manageable and reversible; neutrophil count and body weight rebounded from their low points in just a few days. Table 20 summarizes the weight reduction, neutrophil and monocyte measurements on Day 4, with tumor weight reduction calculated at Day 18:

**Table 20**

| Group | Antitumor Compound | Dose (mg/kg) | Weight (mg) | % Reduction | Neutrophils (K/µL) | % Reduction | Monocytes (K/µL) | % Reduction |
|---|---|---|---|---|---|---|---|---|
| 1 | vehicle | n/a | 868.5 | - | 0.9115 | | 0.096 | - |
| 2 | 0499 | 80 | 0 | 100.0% | 0.077 | 91.6% | 0.073 | 24.0% |
| 3 | 0550 | 73 | 0 | 100.0% | 0.037 | 95.9% | 0.019 | 80.2% |
| 4 | 0611 | 113 | 0 | 100.0% | 0.057 | 93.7% | 0.042 | 56.3% |
| 5 | 0748 | 200 | 268.7 | 69.1% | 0.269 | 70.5% | 0.117 | - |

### Summary of Results for Tumor Scoring System

In the animal studies described in the examples above, antitumor compounds 0503, 0706, 0838 and 1011 consistently demonstrated excellent efficacy scores for the tumors targeted in these studies. The 75 percentile score for all antitumor compounds against a given tumor model was calculated. These four antitumor compounds scored at or above the 75 percentile score. Antitumor compounds 0947 and 1003 also demonstrated good scores in the models in which it was tested.

## Claims

1. A taxane having the formula 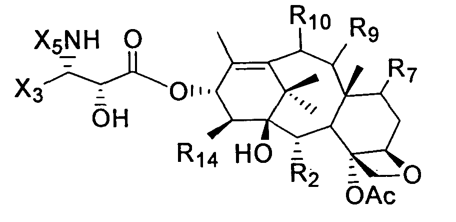
R₂ is acyloxy;
R₇ is hydroxy;
R₉ is keto, hydroxy, or acyloxy;
R₁₀ is R₁₀ₐCOO-;
R₁₀ₐ is hydrocarbyl, substituted hydrocarbyl, or heterocyclo wherein said hydrocarbyl or substituted hydrocarbyl contains carbon atoms in the alpha and beta positions relative to the carbon atom of which R₁₀ₐ is a substituent;
R₁₄ is hydrido or hydroxy;
X₃ is heterocyclo or substituted or unsubstituted alkyl, alkenyl, or alkynyl, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo; and
Ac is acetyl.

2. The taxane of claim 1 wherein R₁₀ₐ is substituted or unsubstituted C₂ - C₈ alkyl, C₂ - C₈ alkenyl or C₂ - C₈ alkynyl.

3. The taxane of claim 2 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

4. The taxane of claim 2 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

5. The taxane of claim 2 wherein X₅ is -COX₁₀ and X₁₀ is phenyl, or X₅ is -COOX₁₀ and X₁₀ is t-butyl.

6. The taxane of claim 2 wherein R₁₄ is hydrido.

7. The taxane of claim 6 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

8. The taxane of claim 6 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

9. The taxane of claim 6 wherein X₅ is -COX₁₀ and X₁₀ is phenyl, or X₅ is -COOX₁₀ and X₁₀ is t-butyl.

10. The taxane of claim 2 wherein R₂ is benzoyloxy.

11. The taxane of claim 10 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

12. The taxane of claim 10 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂- C₈ alkynyl or X₅ is -COOX₁₀ and X₁₀ is substituted or unsubstituted C₁ - C₈ alkyl, C₂- C₈ alkenyl, or C₂ - C₈ alkynyl.

13. The taxane of claim 10 wherein X₅ is -COX₁₀ and X₁₀ is phenyl, or X₅ is -COOX₁₀ and X₁₀ is t-butyl.

14. The taxane of claim 2 wherein R₁₄ is hydrido and R₉ is keto.

15. The taxane of claim 14 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

16. The taxane of claim 14 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

17. The taxane of claim 14 wherein X₅ is -COX₁₀ and X₁₀ is phenyl, or X₅ is -COOX₁₀ and X₁₀ is t-butyl.

18. The taxane of claim 2 wherein R₂ is benzoyloxy and R₉ is keto.

19. The taxane of claim 18 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

20. The taxane of claim 18 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

21. The taxane of claim 18 wherein X₅ is -COX₁₀ and X₁₀ is phenyl, or X₅ is -COOX₁₀ and X₁₀ is t-butyl.

22. The taxane of claim 2 wherein R₁₄ is hydrido and R₂ is benzoyloxy.

23. The taxane of claim 22 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

24. The taxane of claim 22 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

25. The taxane of claim 22 wherein X₅ is -COX₁₀ and X₁₀ is phenyl, or X₅ is -COOX₁₀ and X₁₀ is t-butyl.

26. The taxane of claim 2 wherein R₁₄ is hydrido, R₉ is keto, and R₂ is benzoyloxy.

27. The taxane of claim 26 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

28. The taxane of claim 26 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

29. The taxane of claim 26 wherein X₅ is -COX₁₀ and X₁₀ is phenyl, or X₅ is -COOX₁₀ and X₁₀ is t-butyl.

30. The taxane of claim 1 wherein R₁₀ₐ is C₂ - C₈ alkyl.

31. The taxane of claim 30 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

32. The taxane of claim 30 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

33. The taxane of claim 30 wherein X₅ is -COX₁₀ and X₁₀ is phenyl, or X₅ is -COOX₁₀ and X₁₀ is t-butyl.

34. The taxane of claim 30 wherein R₁₄ is hydrido.

35. The taxane of claim 34 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

36. The taxane of claim 34 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

37. The taxane of claim 34 wherein X₅ is -COX₁₀ and X₁₀ is phenyl, or X₅ is -COOX₁₀ and X₁₀ is t-butyl.

38. The taxane of claim 30 wherein R₂ is benzoyloxy.

39. The taxane of claim 38 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

40. The taxane of claim 38 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂- C₈ alkenyl, or C₂ - C₈ alkynyl.

41. The taxane of claim 38 wherein X₅ is -COX₁₀ and X₁₀ is phenyl, or X₅ is -COOX₁₀ and X₁₀ is t-butyl.

42. The taxane of claim 30 wherein R₁₄ is hydrido and R₉ is keto.

43. The taxane of claim 42 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

44. The taxane of claim 42 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

45. The taxane of claim 42 wherein X₅ is -COX₁₀ and X₁₀ is phenyl, or X₅ is -COOX₁₀ and X₁₀ is t-butyl.

46. The taxane of claim 30 wherein R₂ is benzoyloxy and R₉ is keto.

47. The taxane of claim 46 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

48. The taxane of claim 46 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂- C₈ alkenyl, or C₂ - C₈ alkynyl.

49. The taxane of claim 46 wherein X₅ is -COX₁₀ and X₁₀ is phenyl, or X₅ is -COOX₁₀ and X₁₀ is t-butyl.

50. The taxane of claim 30 wherein R₁₄ is hydrido and R₂ is benzoyloxy.

51. The taxane of claim 50 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

52. The taxane of claim 50 wherein X₅ is -COX₁₀ and; X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂- C₈ alkenyl, or C₂ - C₈ alkynyl.

53. The taxane of claim 50 wherein X₅ is -COX₁₀ and X₁₀ is phenyl, or X₅ is -COOX₁₀ and X₁₀ is t-butyl.

54. The taxane of claim 30 wherein R₁₄ is hydrido, R₉ is keto, and R₂ is benzoyloxy.

55. The taxane of claim 54 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

56. The taxane of claim 54 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

57. The taxane of claim 54 wherein X₅ is -COX₁₀ and X₁₀ is phenyl, or X₅ is -COOX₁₀ and X₁₀ is t-butyl.

58. The taxane of claim 1 wherein R₁₀ₐ is ethyl.

59. The taxane of claim 58 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

60. The taxane of claim 58 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂- C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

61. The taxane of claim 58 wherein X₅ is -COX₁₀ and X₁₀ is phenyl, or X₅ is -COOX₁₀ and X₁₀ is t-butyl.

62. The taxane of claim 58 wherein R₁₄ is hydrido.

63. The taxane of claim 62 wherein X₃ is 2-furyl, 3-furyl, 2-thienyl, 3-thienyl, 2-pyridyl, 3-pyridyl, 4-pyridyl, C₂ - C₈ alkyl, C₂- C₈ alkenyl, or C₂ - C₈ alkynyl.

64. The taxane of claim 62 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

65. The taxane of claim 62 wherein X₅ is -COX₁₀ and X₁₀ is phenyl, or X₅ is -COOX₁₀ and X₁₀ is t-butyl.

66. The taxane of claim 58 wherein R₂ is benzoyloxy.

67. The taxane of claim 66 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

68. The taxane of claim 66 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

69. The taxane of claim 66 wherein X₅ is -COX₁₀ and X₁₀ is phenyl, or X₅ is -COOX₁₀ and X₁₀ is t-butyl.

70. The taxane of claim 58 wherein R₁₄ is hydrido and R₉ is keto.

71. The taxane of claim 70 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

72. The taxane of claim 70 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

73. The taxane of claim 70 wherein X₅ is -COX₁₀ and X₁₀ is phenyl, or X₅ is -COOX₁₀ and X₁₀ is t-butyl.

74. The taxane of claim 58 wherein R₂ is benzoyloxy and R₉ is keto.

75. The taxane of claim 74 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

76. The taxane of claim 74 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

77. The taxane of claim 74 wherein X₅ is -COX₁₀ and X₁₀ is phenyl, or X₅ is -COOX₁₀ and X₁₀ is t-butyl.

78. The taxane of claim 58 wherein R₁₄ is hydrido and R₂ is benzoyloxy.

79. The taxane of claim 78 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

80. The taxane of claim 78 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

81. The taxane of claim 78 wherein X₅ is -COX₁₀ and X₁₀ is phenyl, or X₅ is -COOX₁₀ and X₁₀ is t-butyl.

82. The taxane of claim 58 wherein R₁₄ is hydrido, R₉ is keto, and R₂ is benzoyloxy.

83. The taxane of claim 82 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

84. The taxane of claim 82 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl or X₅ is -COOX₁₀ and X₁₀ is substituted or unsubstituted C₁ - C₈ alkyl, C₂ - C₈ alkenyl, or C₂ - C₈ alkynyl.

85. The taxane of claim 82 wherein X₅ is -COX₁₀ and X₁₀ is phenyl, or X₅ is -COOX₁₀ and X₁₀ is t-butyl.

86. The taxane of claim 82 wherein X₅ is -COOX₁₀ and X₁₀ is t-butyl.

87. The taxane of claim 86 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

88. The taxane of claim 86 wherein X₃ is furyl or thienyl.

89. The taxane of claim 86 wherein X₃ is 2-furyl.

90. The taxane of claim 86 wherein X₃ is 2- thienyl.

91. The taxane of claim 86 wherein X₃ is cycloalkyl.

92. A taxane having the formula 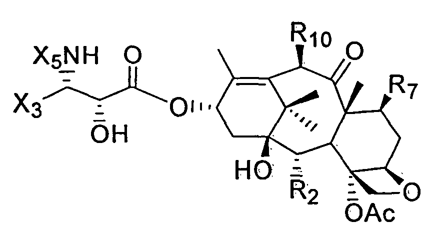
R₂ is benzoyloxy;
R₇ is hydroxy;
R₁₀ is R₁₀ₐCOO-;
X₃ is substituted or unsubstituted alkyl, alkenyl, alkynyl, or heterocyclo, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo; and
R₁₀ₐ is hydrocarbyl, substituted hydrocarbyl, or heterocyclo wherein said hydrocarbyl or substituted hydrocarbyl contains carbon atoms in the alpha and beta positions relative to the carbon of which R₁₀ₐ is a substituent; and
Ac is acetyl.

93. The taxane of claim 92 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

94. The taxane of claim 93 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

95. The taxane of claim 93 wherein X₅ is -COX₁₀ and X₁₀ is phenyl, or X₅ is -COOX₁₀ and X₁₀ is t-butyl.

96. The taxane of claim 92 wherein X₃ is furyl or thienyl.

97. The taxane of claim 96 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

98. The taxane of claim 96 wherein X₅ is -COX₁₀ and X₁₀ is phenyl, or X₅ is -COOX₁₀ and X₁₀ is t-butyl.

99. The taxane of claim 93 wherein X₃ is cycloalkyl.

100. The taxane of claim 99 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

101. The taxane of claim 99 wherein X₅ is -COX₁₀ and X₁₀ is phenyl, or X₅ is -COOX₁₀ and X₁₀ is t-butyl.

102. The taxane of claim 93 wherein X₃ is isobutenyl.

103. The taxane of claim 102 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

104. The taxane of claim 102 wherein X₅ is -COX₁₀ and X₁₀ is phenyl, or X₅ is -COOX₁₀ and X₁₀ is t-butyl.

105. The taxane of claim 92 wherein R₁₀ₐ is ethyl or propyl.

106. The taxane of claim 105 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

107. The taxane of claim 106 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

108. The taxane of claim 106 wherein X₅ is -COX₁₀ and X₁₀ is phenyl, or X₅ is -COOX₁₀ and X₁₀ is t-butyl.

109. The taxane of claim 105 wherein X₃ is furyl or thienyl.

110. The taxane of claim 109 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

111. The taxane of claim 109 wherein X₅ is -COX₁₀ and X₁₀ is phenyl, or X₅ is -COOX₁₀ and X₁₀ is t-butyl.

112. The taxane of claim 105 wherein X₃ is cycloalkyl.

113. The taxane of claim 112 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

114. The taxane of claim 112 wherein X₅ is -COX₁₀ and X₁₀ is phenyl, or X₅ is -COOX₁₀ and X₁₀ is t-butyl.

115. The taxane of claim 105 wherein X₃ is isobutenyl.

116. The taxane of claim 115 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

117. The taxane of claim 115 wherein X₅ is -COX₁₀ and X₁₀ is phenyl, or X₅ is -COOX₁₀ and X₁₀ is t-butyl.

118. The taxane of claim 92 wherein X₃ is furyl or thienyl, R₁₀ₐ is ethyl, and X₅ is -COX₁₀ and X₁₀ is phenyl, or X₅ is -COOX₁₀ and X₁₀ is t-butyl.

119. The taxane of claim 92 wherein X₃ is substituted or unsubstituted furyl, R₁₀ₐ is ethyl, and X₅ is -COX₁₀ and X₁₀ is phenyl, or X₅ is -COOX₁₀ and X₁₀ is t-butyl.

120. The taxane of claim 92 wherein X₃ is substituted or unsubstituted thienyl, R₁₀ₐ is ethyl, and X₅ is -COX₁₀ and X₁₀ is phenyl, or X₅ is -COOX₁₀ and X₁₀ is t-butyl.

121. The taxane of claim 92 wherein X₃ is isobutenyl, R₁₀ₐ is ethyl, and X₅ is -COX₁₀ and X₁₀ is phenyl, or X₅ is -COOX₁₀ and X₁₀ is t-butyl.

122. The taxane of claim 92 wherein X₃ is alkyl, R₁₀ₐ is ethyl, and X₅ is -COX₁₀ and X₁₀ is phenyl, or X₅ is -COOX₁₀ and X₁₀ is t-butyl.

123. The taxane of claim 92 wherein X₃ is 2-furyl or 2-thienyl, R₁₀ₐ is ethyl, X₅ is -COOX₁₀ and X₁₀ is t-butyl.

124. The taxane of claim 92 wherein X₃ is 2-furyl, R₁₀ₐ is ethyl, X₅ is -COOX₁₀ and X₁₀ is t-butyl.

125. The taxane of claim 92 wherein X₃ is 2-thienyl, R₁₀ₐ is ethyl, X₅ is -COOX₁₀ and X₁₀ is t-butyl.

126. The taxane of claim 92 wherein X₃ is isobutenyl, X₅ is -COOX₁₀ and X₁₀ is t-butyl.

127. The taxane of claim 92 wherein X₃ is cycloalkyl, R₁₀ₐ is ethyl, X₅ is -COOX₁₀ and X₁₀ is t-butyl.

128. A pharmaceutical composition comprising the taxane of claim 1 and at least one pharmaceutically acceptable carrier.

129. The pharmaceutical composition of claim 128 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

130. The pharmaceutical composition of claim 129 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

131. The pharmaceutical composition of claim 129 wherein X₅ is -COX₁₀ and X₁₀ is phenyl, or X₅ is -COOX₁₀ and X₁₀ is t-butyl.

132. The pharmaceutical composition of claim 128 wherein R₁₀ₐ is ethyl or propyl.

133. The pharmaceutical composition of claim 132 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

134. The pharmaceutical composition of claim 133 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

135. The pharmaceutical composition of claim 133 wherein X₅ is -COX₁₀ and X₁₀ is phenyl, or X₅ is -COOX₁₀ and X₁₀ is t-butyl.

136. The pharmaceutical composition of claim 129 wherein X₃ is furyl or thienyl, R₁₀ₐ is ethyl, and X₅ is -COX₁₀ and X₁₀ is phenyl, or X₅ is -COOX₁₀ and X₁₀ is t-butyl.

137. The pharmaceutical composition of claim 129 wherein X₃ is substituted or unsubstituted furyl, R₁₀ₐ is ethyl, and X₅ is -COX₁₀ and X₁₀ is phenyl, or X₅ is -COOX₁₀ and X₁₀ is t-butyl.

138. The pharmaceutical composition of claim 129 wherein X₃ is substituted or unsubstituted thienyl, R₁₀ₐ is ethyl, and X₅ is -COX₁₀ and X₁₀ is phenyl, or X₅ is -COOX₁₀ and X₁₀ is t-butyl.

139. The pharmaceutical composition of claim 129 wherein X₃ is isobutenyl, R₁₀ₐ is ethyl, and X5 is -COX₁₀ and X₁₀ is phenyl, or X₅ is -COOX₁₀ and X₁₀ is t-butyl.

140. The pharmaceutical composition of claim 129 wherein X₃ is alkyl, R₁₀ₐ is ethyl, and X₅ is -COX₁₀ and X₁₀ is phenyl, or X₅ is -COOX₁₀ and X₁₀ is t-butyl.

141. The pharmaceutical composition of claim 129 wherein X₃ is 2-furyl or 2-thienyl, R₁₀ₐ is ethyl, X₅ is -COOX₁₀ and X₁₀ is t-butyl.

142. The pharmaceutical composition of claim 129 wherein X₃ is 2-furyl, R₁₀ₐ is ethyl, X₅ is -COOX₁₀ and X₁₀ is t-butyl.

143. The pharmaceutical composition of claim 129 wherein X₃ is 2-thienyl, R₁₀ₐ is ethyl, X₅ is -COOX₁₀ and X₁₀ is t-butyl.

144. The pharmaceutical composition of claim 129 wherein X₃ is isobutenyl, X₅ is -COOX₁₀ and X₁₀ is t-butyl.

145. The pharmaceutical composition of claim 129 wherein X₃ is cycloalkyl, R₁₀ₐ is ethyl, X₅ is -COOX₁₀ and X₁₀ is t-butyl.

146. A pharmaceutical composition comprising the taxane of claim 92 and at least one pharmaceutically acceptable carrier.

147. A pharmaceutical composition comprising the taxane of claim 96 and at least one pharmaceutically acceptable carrier.

148. A composition for oral administration comprising the taxane of claim 1 and at least one pharmaceutically acceptable carrier.

149. The composition of claim 148 wherein X₃ is 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl.

150. The composition of claim 149 wherein R₁₀ₐ is ethyl or propyl.

151. The composition of claim 150 wherein X₅ is -COX₁₀ and X₁₀ is phenyl, or X₅ is -COOX₁₀ and X₁₀ is t-butyl.

152. The composition of claim 150 wherein R₁₄ is hydrogen, R₂ is benzoyloxy, and X₅ is -COX₁₀ wherein X₁₀ is phenyl, or X₅ is -COOX₁₀ wherein X₁₀ is t-butyl.

153. The composition of claim 152 wherein X₃ is isobutenyl; R₁₀ₐ is ethyl; and X₅ is -COOX₁₀ and X₁₀ is t-butyl.

154. The composition of claim 152 wherein X₃ is phenyl; R₁₀ₐ is ethyl; and X₅ is -COOX₁₀ and X₁₀ is t-butyl.

155. The composition of claim 152 wherein X₃ is furyl or thienyl; R₁₀ₐ is ethyl; and X₅ is -COOX₁₀ and X₁₀ is t-butyl.

156. The composition of claim 155 wherein X₃ is furyl.

157. The composition of claim 152 wherein X₃ is phenyl; R₁₀ₐ is ethyl; and X₅ is -COX₁₀ and X₁₀ is phenyl.

158. The use of a taxane as defined in any one of claims 1 - 127 for manufacturing a pharmaceutical composition effective for inhibiting tumor growth in a mammal when orally administering a therapeutically effective amount of said pharmaceutical composition, containing said taxane and at least one pharmaceutically acceptable carrier, to said mammal.

159. The use of claim 158 wherein X₃ is 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂- C₈ alkynyl.

160. The use of claim 159 wherein R₁₀ₐ is ethyl or propyl.

161. The use of claim 160 wherein X₅ is -COX₁₀ and X₁₀ is phenyl, or X₅ is -COOX₁₀ and X₁₀ is t-butyl.

162. The use of claim 161 wherein R₁₄ is hydrogen and R2 is benzoyloxy.

163. The use of claim 162 wherein X₃ is isobutenyl; R₁₀ₐ is ethyl; and X₅ is -COOX₁₀ and X₁₀ is t-butyl.

164. The use of claim 162 wherein X₃ is phenyl; R₁₀ₐ is ethyl; and X₅ is -COOX₁₀ and X₁₀ is t-butyl.

165. The use of claim 162 wherein X₃ is furyl or thienyl; R₁₀ₐ is ethyl; and X₅ is -COOX₁₀ and X₁₀ is t-butyl.

166. The use of claim 165 wherein X₃ is furyl.

167. The use of claim 162 wherein X₃ is phenyl; R₁₀ₐ is ethyl; and X₅ is -COX₁₀ and X₁₀ is phenyl.

168. A pharmaceutical composition as defined in any one of the preceeding claims, with an ID50 value, of at least 4 and preferably 5, 6, 7, 8, 9 or 10 times less than the ID50 value of paclitaxel or docetaxel, when measured as defined herein.

169. A pharmaceutical composition as defined in any one of the preceeding claims, in a single dose formulation comprising at least 20 mg of said taxane per m² of patient body surface area.

170. The pharmaceutical composition of claim 169, comprising less than 600 mg, preferably between 25 and 400 mg, more preferably between 40 and 300 mg and even more preferably between 50 and 200 mg, of said taxane per m² of patient body surface area, for oral administration, wherein the average body surface area for a human is 1.8 m².

171. The pharmaceutical composition of claim 169, comprising less than 500 mg, preferably 40 to 400 mg and more preferably 60 to 350 mg of said taxane per m² of patient body surface area, for parenteral administration, wherein the average body surface area for a human is 1.8 m².

172. A pharmaceutical composition as defined in any one of the preceeding claims, in the form of a liquid composition containing between 0.01 and 10 mg/ml of taxane, preferably between 0.1 and 7 mg/ml, more preferably between 0.5 and 5 mg/ml and most preferred between 1.5 and 4 mg/ml.

173. A pharmaceutical composition as defined in any one of the preceeding claims, in the form of a liquid composition containing between 5 wt% and 50 wt%, preferably between 8 wt% and 40 wt%, more preferred between 10 wt% and 30 wt% of said taxane.

174. A pharmaceutical composition as defined in any one of the preceeding claims, comprising a taxane antitumor agent in an amount effective for achieving significant tumor growth inhibition, preferably partial tumor shrinkage (Partial Response) and most preferably complete tumor shrinkage (Complete Response) as defined herein, in at least one of human lung carcinoma, human colon carcinoma, human breast carcinoma, human prostate carcinoma, human ovarian carcinoma, human melanoma and human pancreatic carcinoma.

175. The pharmaceutical composition of claim 174, said composition being more efficient than paclitaxel and/or docetaxel in comparable amount.

176. The pharmaceutical composition of any one of claims 168 - 175, said taxane selected from the compounds defined in claim 92 or preferably, claim 96.

177. The pharmaceutical composition of any one of claims 168 - 176, said taxane selected from compounds corresponding to formula 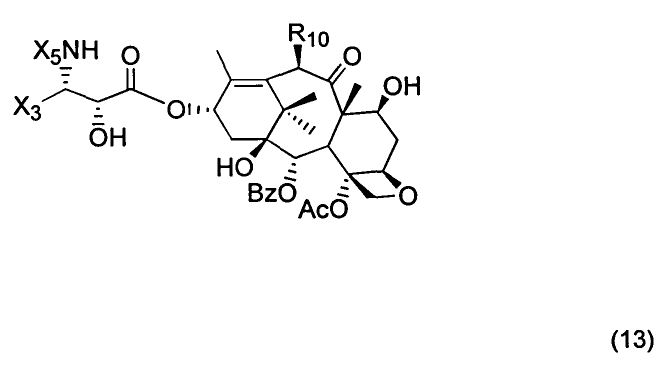
with X₅ = tBuOCO-, X₃ = Isobutenyl and R₁₀ = EtCOO-,
or X₅ = tBuOCO-, X₃ = 2-pyridyl and R₁₀ = EtCOO-,
or X₅ = tBuOCO-, X₃ = 2-furyl and R₁₀ = EtCOO-,
or X₅ = tBuOCO-, X₃ = 2-thienyl and R₁₀ = EtCOO-,
or X₅ = tBuOCO-, X₃ = Phenyl and R₁₀ = EtCOO-,
or X₅ = PhCO-, X₃ = 2-furyl and R₁₀ = EtCOO-,
or X₅ = PhCO-, X₃ = 3-furyl and R₁₀ = EtCOO-,
or X₅ = PhCO-, X₃ = 2-thienyl and R₁₀ = EtCOO-,
or X₅ = PhCO-, X₃ = Cyclopropyl and R₁₀ = EtCOO-,
or X₅ = PhCO-, X₃ = Phenyl and R₁₀ = EtCOO-,
or X₅ = tBuOCO-, X₃ = Isobutenyl and R₁₀ = CproCOO-,
or X₅ = PhCO-, X₃ = 2-furyl and R₁₀ = CproCOO-,
or X₅ = tBuOCO-, X₃ = Isobutenyl and R₁₀ = PrCOO-,
or X₅ = tBuOCO-, X₃ = 2-pyridyl and R₁₀ = PrCOO-,
or X₅ = tBuOCO-, X₃ = 2-furyl and R₁₀ = PrCOO-,
or X₅ = tBuOCO-, X₃ = 3-furyl and R₁₀ = PrCOO-,
or X₅ = tBuOCO-, X₃ = 2-thienyl and R₁₀ = PrCOO-,
or X₅ = tBuOCO-, X₃ = 3-thienyl and R₁₀ = PrCOO-,

178. The pharmaceutical composition of claim 177, wherein the taxane corresponds to Formula 13
with X₅ = tBuOCO-, X₃ = 2-pyridyl and R₁₀ = EtCOO-,
or X₅ = PhCO-, X₃ = Cyclopropyl and R₁₀ = EtCOO-,
or X₅ = tBuOCO-, X₃ = Isobutenyl and R₁₀ = CproCOO-,
or X₅ = tBuOCO-, X₃ = Isobutenyl and R₁₀ = CproCOO-,
or X₅ = tBuOCO-, X₃ = Isobutenyl and R₁₀ = PrCOO-,
or X₅ = tBuOCO-, X₃ = 2-thienyl and R₁₀ = PrCOO-,
or X₅ = tBuOCO-, X₃ = 3-thienyl and R₁₀ = PrCOO-.

179. The pharmaceutical composition of any one of the preceeding claims, the taxane having a solubility in ethanol of at least 100 mg/ml and preferably up to 800 mg/ml and higher.
